# EUROPEAN PATENT APPLICATION

(11) **EP 1 380 844 A2**
(43) Date of publication of application: **14.01.2004**
(21) Application number: 03021138.7
(22) Date of filing: 24.03.1993
(51) Int. Cl.: G01N 35/02

(54) **Heater assembly for temperature and delivery control of liquids**

(30) Priority: 27.03.1992 US 859218; 18.03.1993 US 27268; 18.03.1993 US 27270; 18.03.1993 US 27387; 18.03.1993 US 27388; 18.03.1993 US 27481
(62) Divisional of application: 93908481.0
(71) Applicant: Abbott Laboratories, Abbott Park, Illinois 60064-6008 (US)
(72) Inventor: Clark, Frederic L., Plano, TX 75023 (US); Clift, Gilbert, Mesquite, TX 75150 (US); Hendrick, Kendall B., Southlake, TX 76092 (US); Kanewske, William J.III, Dallas, TX 75208 (US); Lagocki, Peter A., Park Ridge, IL 60068 (US); Martin, Richard R., Irving, TX 75063 (US); Mitchell, James E., Lake Barrington, IL 60010 (US); Moore, Larry W, Plano, TX 75075 (US); Pennington, Charles D., Lake Zurich, IL 60047 (US); Walker, Edna S., Chicago, IL 60618 (US); Smith, Jane B., Vernon Hills, IL 60061 (US); Tayi, Apparao, Grayslake, IL 60030 (US); Vaught, James A., Euless, TX 76039 (US); Yost, David A, Poolesville, MD 20837 (US); Merriam, Richard A., Dallas, TX 75218 (US); Walker, Donny Ray, Coppell, TX 75019 (US); McDowell, Douglas D., Wildwood, IL (US); Rumbaugh, William A., Carrollton, TX 75006 (US); Clemens, John M., Wadsworth, IL 60083 (US)
(74) Representative: Modiano, Guido, Dr.-Ing.

(57) **Abstract**

A heater assembly for precision temperature and delivery control of liquids and an automated, continuous and random access analytical system containing such heater assembly and capable of simultaneously effecting multiple assays of a plurality of liquid samples are disclosed. The heater assembly comprises a body having internal resistance heating means and temperature control means, said body having a cavity suitable for accommodating liquid tubing means, said liquid tubing means in communication with liquid inlet and outlet means, wherein said cavity is suitable for housing a tubing means for maintaining liquids at a predetermined temperature, and wherein said liquid outlet means is suitable for forced or gravity release of the liquids and is mounted directly above analytical means for receiving liquids.

## Description

### Field of the Invention

The present invention relates to an automated analytical system and methods for the analysis of liquid test samples. In another aspect, the invention is related to a continuous and random access system which is capable of simultaneously performing a plurality of assays, particularly heterogeneous and/or homogeneous immunoassays. In yet another aspect, the present invention relates to the various components incorporated into and utilized by such system.

### Background of the Invention

Although various known clinical analyzers for chemical, immunochemical and biological testing of samples are available, clinical technology is rapidly changing due to increasing demands in the clinical laboratory to provide new levels of service. These new levels of service must be more cost effective to decrease the operating expenditures such as labor cost and the like, and must provide shorter turnaround time of test results to reduce the patient's length of stay in the hospital as well as improve efficiency of outpatient treatment. Modernization of analytical apparatus and procedures demands consolidation of work stations to meet the growing challenge placed on clinical laboratories.

Generally, analysis of a test sample involves the reaction of test samples with one or more reagents with respect to one or more analytes wherein it is frequently desired that the analysis be performed on a selective basis with respect to each test sample. However, due to the high demands placed on clinical laboratories regarding not only volume throughput but also the number and frequency of various analyses, there is a need to provide an automated analysis system which is capable of combining accurate analytical results, high throughput, multiple test menu versatility as well as low reagent consumption.

Typically, analysis of a test sample involves forming a reaction mixture comprising the test sample and one or more reagents, and the reaction mixture is then analyzed by an apparatus for one or more characteristics of the test sample. Reliance on automated clinical analyzers improves the efficiency of the laboratory procedures inasmuch as the technician has fewer tasks to performed. Automated clinical analyzers provide results much more rapidly while frequently avoiding operator or technician error, thus placing emphasis on accuracy and repeatability of a variety of tests. Automated clinical analyzers presently available for routine laboratory tests include a transport or conveyor system designed to transport containers of sample liquids between various operating stations. For example, a reaction tube or cuvette containing a test sample may pass through a reagent filling station, mixing station, reaction forming station, detection stations, analysis stations, and the like. However, such transport systems are not flexible in that transport is in one direction and the reaction tubes or cuvettes, once inserted into the apparatus, must pass through without access before analysis occurs.

Automated immunoassay analyzers have been provided such as the Abbott IMx® analyzer and the Abbott TDx® analyzer (Abbott Laboratories, Abbott Park, Illinois, USA) which utilize procedures involving a variety of different assay steps but typically rely on detection and measurement of optical changes in a reaction mixture during the assay process. For example, a number of well-known techniques using single or multi-wavelength fluorescence include fluorescent polarization immunoassays (FPIA) employing homogeneous immunoassay techniques, microparticle enzyme immunoassays (MEIA) employing heterogeneous immunoassay techniques, and the like. The MEIA technology, such as that used on the Abbott IMx® analyzer, is used for high and low molecular weight analytes requiring greater sensitivity, and FPIA technology, such as that used on the Abbott TDx® analyzer, is used primarily for lower molecular weight analytes. A front surface fluorometer is used to quantify a fluorescent product generated in the MEIA assays, while a fluorescence polarization optical system is used to quantify the degree of tracer binding to antibody in the FPIA assays. The test samples are automatically processed in the Abbott IMx® analyzer and Abbott TDx® analyzer by a robotic arm with a pipetting probe and a rotating carousel which positions the samples for processing. These instruments are compact table-top analyzers which offer fully automated, walk-away immunoassay testing capabilities for both routine and specialized immunoassays. These nonisotopic methods eliminate radioactivity disposal problems and increase reagent shelf life while meeting the diverse requirements of a multitude of different assays.

Instead of loading the test sample into a container and obtaining sequential testing, such as one direction only systems as described above, the Abbott IMx® analyzer and the Abbott TDx® analyzer, often referred to as batch analyzers, permit the analysis of multiple samples and provide for access to the test samples for the formation of subsequent reaction mixtures. However, such batch analyzers permit only one type of analysis at a time. In a random access analyzer, not only can multiple test samples be analyzed, but multiple analytes may be analyzed from each test sample. Another common feature of presently available sequential and random access analyzers is the inclusion of various reagents within the apparatus itself or placed near the apparatus for pipetting purposes. Liquid reagents, in bulk form, are selected for the various types of tests which are to be performed on the test sample, and are stored in or near the apparatus. The reagent delivery units, such as pumps and the like, along with valves, control and pipette mechanisms, are included in these automated analyzers so that different reagents can be mixed according to the type of test to be performed. The Abbott IMx® analyzer automatically performs all the steps required for analysis of test samples and includes numerous checks of the subsystems to insure that the assay can be run to completion and that results are valid. Quantification of the fluorescence intensity in the MEIA method and polarization in the FPIA method, as well as the final data reduction, are also fully automated on the analyzer. Results are printed by the analyzer and can be accessed through suitable means for automatic data collection by a laboratory computer.

Automated analytical apparatus for performing homogeneous assays, the detection of precipitate formed by reaction between antigens and antibodies in a test sample-cell to form light scattering centers, and methods and apparatus for detecting immunological agglutination reactions are also known in the art. Such apparatus and methods include, for example, the steps of measuring light absorption of the liquid medium with antibody before and after the antigen-antibody reaction by using light which is absorbable by the antibody, and calculating the difference of the absorptions. In this way, the presence or absence of agglutination can be detected based on the fact that the agglutination reaction reduces the concentration of antibody, which affects the light absorption of the liquid medium. As is typical of methods and apparatus for performing homogeneous assays, these procedures do not require separation of a solid phase from the reaction mixture for further analysis.

Heterogeneous assays are also known through the use of a sample analyzer for quantitating relatively small amounts of clinically significant compounds in a liquid test sample by focusing a light source onto the sample so that, for example, fluorescent particles in the sample cause fluorescent conditions, the intensity of which is the function of the intensity of the light beam and the concentration of fluorescent particles in the sample. A detector senses photons forming the fluorescent emissions of the particles when excited by the light beam. The introduction of a solid phase material into the sample requires subsequent separation of the solid phase from the reaction mixture for further analysis and before the fluorescent emissions can be detected and measured.

Recently, apparatus and methods have been proposed for performing, selectively on the same sample, various homogeneous and heterogeneous assays concurrently in a random access fashion. Such apparatus and methods provide for the analysis of a plurality of liquid samples wherein each sample is analyzed with respect to at least one analyte utilizing both homogeneous and heterogeneous assay techniques.

Various assay protocols and formats often have specific temperature requirements for incubation and various analytical reactions during the course of an assay. Therefore, liquids such as, for example, the test sample, buffers, wash liquids, liquid reagents and the like, are generally maintained within such temperature requirements, and in many cases, require precision temperature control upon addition to an assay reaction. These temperature dependent assays require, in addition to general ambient air temperature control, specific liquid temperature control which cannot be provided by general ambient air temperature flux.

When multiple assays are run simultaneously in a random format, the control of carryover or contamination of test samples or reagents involved in different assays is an issue to consider with respect to assay performance and reliability. Although not all assays are at risk for such carryover, every assay requires handling of test samples and reagents, which can be a source of carryover for any assay susceptible to carryover contamination. Accordingly, all assays either perform a wash step after each pipetting step where a test sample is pipetted, or these assays susceptible to carryover contamination must perform a wash step every time such pipetting steps are performed. Such approach, however, requires the system to operate on a cautious basis, requiring excessive amounts of wash fluids. In addition, if a pipette probe performs a number of time consuming amd unnecessary washing steps, the efficiency of performing assays is hindered to result in low throughput.

Previous attempts to identify interactive steps within and between multiple assays have given rise to cumbersome wash tables having rows and columns marked by every pipetting sequence performed by the instrument. For example, every combination of sequences is empirically tested and the volume of wash is determined that eliminates carryover between the two steps, wherein the wash volume then goes into the table. However, this type of approach is laborious to implement and difficult to control when new assays are introduced. Another approach is utilized by the Abbott IMx® Select Analyzer (Abbott Laboratories, Abbott Park, IL) to reduce wash volume. In such analyzer, a sensitive step is identified (step B) that requires extra wash when it follows step A, but not when it follows another step B. A flag is used to identify when a pipetting step occurs after step A and a larger wash is used. However, application of this approach is limited because only a limited number of assays are run simultaneously on such analyzer, and does not address the problems of possible carryover and contamination which may be encountered in a random access, continuous access analyzer.

Generally, automated analytical systems which perform different types of assays on a plurality of test samples require appropriate test sample handling and loading means. However, the operation of such automated analytical systems can be limited by test sample container handling and loading due to the varying dimensions of test sample containers which may be received by a laboratory. Such varying dimensions often require that a test sample be transferred from one container to a container which is adapted to a particular analytical instrument. Thus, a need exists in these systems to provide means for adapting an analytical instrument to receive such test sample container in order to provide flexibility, precision and throughput.

Automated pipetting systems that contact a liquid test sample or liquid reagent with an electrode or level sensing device have been described. For example, a conducting pipette tip or an electrode adjacent to the pipette tip generates an electrical signal when the conducting pipette tip or the electrode touches the surface of an electrically conducting fluid, such as a buffer solution, a liquid test sample, and liquid reagents. Detecting the surface of a fluid is very important for the precise pipetting of that fluid. Locating the fluid or liquid surface permits the controlled immersion of the pipette tip into the liquid. By controlling the depth of immersion of the pipette tip in the liquid, a consistent amount of liquid will adhere to the outside of the tip to result in greater consistency of the total volume dispensed. Non-invasion liquid surface sensing systems have also been described, including methods involving blowing or forcing air utilizing stepper-motor controls to detect a liquid surface level, as well as utilization of a bridged circuit which generates a signal corresponding to a vertical displacement of a pipette for extracting a liquid sample when such vertical displacement movement is caused.

However, problems exist with such liquid level sensing systems previously described, particularly with respect to ambient air pressure sensing and electronic circuitry sensing. In the case of the ambient air sensing, blowing of air can cause bubbles and generate aerosols in and around the liquid samples. Moreover, the prior art liquid level sensing apparatus previously described utilizing, for example, electrostatic capacitance between the pipette and liquid level is instable and varies due to variations in humidity, device parameter drift with time and temperature, and variations in the electrical grounding, which must be as close as possible to the vessel in which fluid is to be sensed results in false readings because of background signal noise and the like.

Automated chemiluminescent instruments utilizing photographic means and a densitometer for recording signals have been described. Automated chemiluminescent instruments which process assays in a lock-step mode have also been described. However, the architecture of such systems are inflexible and accordingly, a need for chemiluminescent detection methods within an automated, continuous and random access analytical system exists since certain immunoassay processing requires greater sensitivity than can be provided using, for example MEIA and FPIA technologies. These fluorescence-based technologies, while robust, offer limited sensitivity with some assays as compared to chemiluminescent detection methods.

Accordingly, since such previously described automated analyzers do not contemplate an automated analytical system for simultaneously performing both homogeneous and heterogeneous assays in a continuous and random access fashion utilizing a commonality of various process work stations and transfer means, there is a need to provide an automated analytical system having these features and sufficient flexibility to meet the growing needs of the modern clinical laboratory.

### Summary of the Invention

The automated analytical system of the present invention is capable of simultaneously performing two or more assays on a plurality of test samples in a continuous and random access fashion. In particular, the automated immunoassay analytical system apparatus of the invention can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled.

According to the invention, automated, continuous and random access analytical system capable of simultaneously effecting multiple assays of a plurality of liquid samples is provided, and enables performing a method wherein various assays are scheduled for a plurality of liquid samples. Through kitting means the present system is capable of creating a unit dose disposable by separately transferring liquid sample and reagents to a reaction vessel without initiation of an assay reaction sequence. From the kitting means multiple, kitted unit dose disposables are transferred to a process area, wherein an aliquot is mixed for each independent sample with one or more liquid reagents at different times in a reaction vessel to form independent reaction mixtures. Independent scheduling of such kitting and mixing is achieved during incubation of the multiple reaction mixtures, simultaneously and independently.

The system of the present invention is capable of performing more than one scheduled assay in any order in which plurality of scheduled assays are presented. The incubated reaction mixtures are analyzed independently and individually by at least two assay procedures which are previously scheduled.

The automated, continuous and random access analytical system apparatus of this invention is comprised of a front end carousel assembly inclusive of a sample cup carousel, a reagent pack carousel and a reaction vessel carousel mounted concentrically and serviced by a transfer pipetting means suitable for kitting and/or mixing reagents with a sample. The kitted and pipetted reaction vessels are transferred through a transfer station which provides means for transferring the kitted and pipetted reaction vessels to a processing work station 4 which includes a controlled environment for maintaining temperature and provides timing for mixing of reagents and incubation. At least two assay procedural apparatus are provided which are scheduled for the various samples and kitted reagents in a unit dose disposable means for analyzing the incubated reaction mixtures. The unit dose disposable reaction vessels are removed from the process carousel by operation of the transfer station, which includes means for removing the disposable reaction vessel from the system.

Additional advantages and novel features of the invention will be set forth in part in the description which follows, and will become apparent to those skilled in the art upon examination of the following or may be learned by practice of the invention. The objects and advantages of the invention may be obtained by means of the exemplary combinations more particularly pointed out in the following specification and appended claims, including all equivalents thereof.

### Brief Description of the Drawings

FIGURE 1 is an isometric view of the automated analytical system illustrating the system cabinetry, exposed front end carousel, computer screen and keyboard.
FIGURE 2 is an isometric view of the automated analytical system apparatus frame and cabinet.
FIGURE 3 is a top plan view of the automated analytical system in section with component covers removed to show the automated analytical system apparatus in detail and relative position.
FIGURE 3A is a top plan view of the automated analytical system in section with component covers removed to show the automated analytical apparatus in detail and relative position inclusive of a chemiluminescent reader for a magnetic particle capture technology and a chemiluminescent reader for membrane particle capture technology.
FIGURE 3B is a cross sectional view of a detection head of the detection device for chemiluminescent detection.
FIGURE 3C is a cross sectional view taken along the perpendicular axis of the detection head shown in FIGURE 3B.
FIGURE 3D is a cross sectional view in section of the detection device light pipe positioned over a chemiluminescent particle capture container with light shield in place.
FIGURE 4 is a front elevational view of the automated analytical system in isolation and partial section of elements of the front end carousel.
FIGURES 4A and 4B represent a perspective side elevational view and partial end view of a reagent pack and reagent pack cover means for use with the automated analytical system.
FIGURE 4C is a perspective view of a test sample container segment assembly.
FIGURE 4D is a bottom view of the test sample container segment assembly of FIGURE 4C.
FIGURE 4E is a cross sectional view in isolation of the sample carousel with a mounted test sample container segment assembly also in cross section.
FIGURE 4F is a cross sectional view of a modified sample cup with skirts.
FIGURE 4G is a perspective view of a short test sample Vacutainer® tube segment assembly.
FIGURE 4H is a top cross sectional view of the short test sample Vacutainer® tube segment assembly taken along the line A-A of FIGURE 4G.
FIGURE 4I is a bottom view of the short test sample Vacutainer® tube segment assembly of FIGURE 4G.
FIGURE 4J is a cross sectional view of a long test sample cup adaptor with tube in place.
FIGURE 4K is a cross sectional view of a short test sample cup adaptor with a tube in place.
FIGURE 5 is a top view in isolation and partial section of drive and guide elements of the front end carousel of the automated analytical system being removed.
FIGURE 6 is a cross-sectional side view of a process carousel of the automated analytical system in isolation with two reaction vessels in place, one of which is in position for an FPIA read.
FIGURE 7 is an isometric view of the probe, probe arm and pipettor of the automated analytical system in isolation.
FIGURE 8 is a schematic side view of the probe arm wiring and sensor means of the automated analytical system.
FIGURE 8A is a simplified diagram showing one embodiment of the liquid level sensing device of the present invention in connection with an automated analytical system.
FIGURE 8B is a simplified diagram showing a current flow with the sense amplifier measuring only current from the antenna, the diluent amount not included.
FIGURE 8C is a graph showing system noise versus loop frequency, stressing the importance of having a high center frequency along with a narrow filter band width.
FIGURES 8D and 8E are graphs showing conditions where threshold is crossed (fluid detection) even though the probe is not in contact with fluid or liquid.
FIGURE 9 is a cross-sectional side elevational view of an automatic bubble flushing syringe apparatus of the automated analytical system.
FIGURE 9A is a sectional side view in isolation of the syringe bore end portion of the automatic bubble flushing syringe with the reciprocating piston near the end of travel toward the bore end portion.
FIGURE 9B is a sectional end view in isolation of the piston and bore of the automatic bubble flushing system syringe taken along line 9B-9D.
FIGURES 10 and 10A represent a top plan view of a reaction vessel and a side view of the reaction vessel for use with the automated analytical system, respectively, with reaction vessel compartments labeled where appropriate for FPIA processing.
FIGURE 11 is a sectional side view of the transfer element of the automated analytical system engaging a reaction vessel for transfer from the main carousel into the transfer station.
FIGURE 12 is a perspective side elevational view of the transfer station of the automated analytical system.

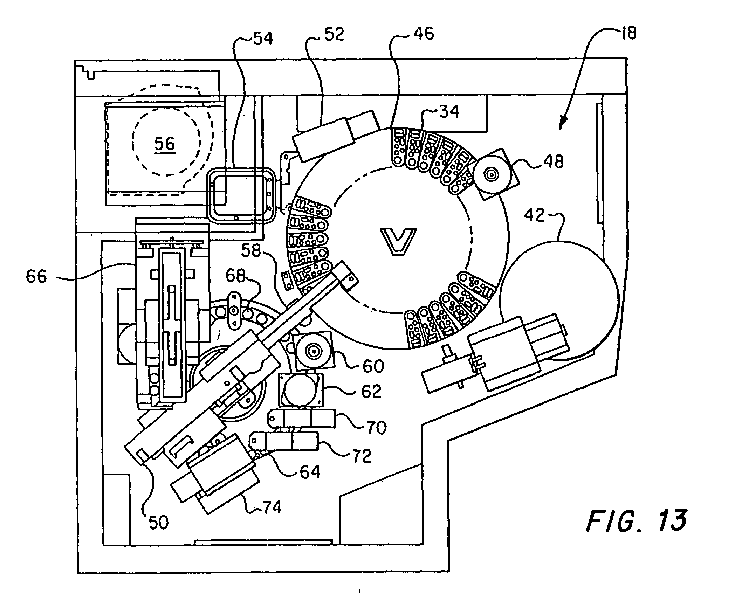

FIGURE 13 is a top plan view in section illustrating in isolation the controlled environment portion of the automated analytical system.
FIGURE 14 is a top plan view in section of the lower cabinet of FIGURES I and 2 illustrating water and/or buffer supply station as well as liquid and solid waster containers of the automated analytical system.
FIGURE 15 is a schematic view illustrating the system control environment airflow and temperature control of the automated analytical system.
FIGURE 15B is a perspective view of a heater assembly for liquid temperature control.
FIGURE 15C is a cross-sectional view through the heater assembly of FIG. 15B showing the heater element within the block.
FIGURE 15D is a partial cross-sectional view of the heater assembly of FIG. 15B showing liquid tubing, for example, a tubing coil within the heater assembly.
FIGURE 16 is a side elevational view in partial section of a MEIA cartridge for use with the automated analytical system.
FIGURE 17 is a side elevational view in section of a MEIA cartridge feeder of the automated analytical system.
FIGURE 18 is a side sectional view in isolation of the MEIA cartridge feeder-cartridge orientation pin mechanism of the automated analytical system.
FIGURE 19 is a side sectional view in isolation of the MEIA cartridge ejector of the automated analytical system.
FIGURE 20 is a box diagram of the optics signal processor of the automated analytical system.
FIGURE 21 is a schematic of the FPIA optical system of the automated analytical system.
FIGURE 22 is a schematic of the FPIA reader sequence of the automated analytical system.

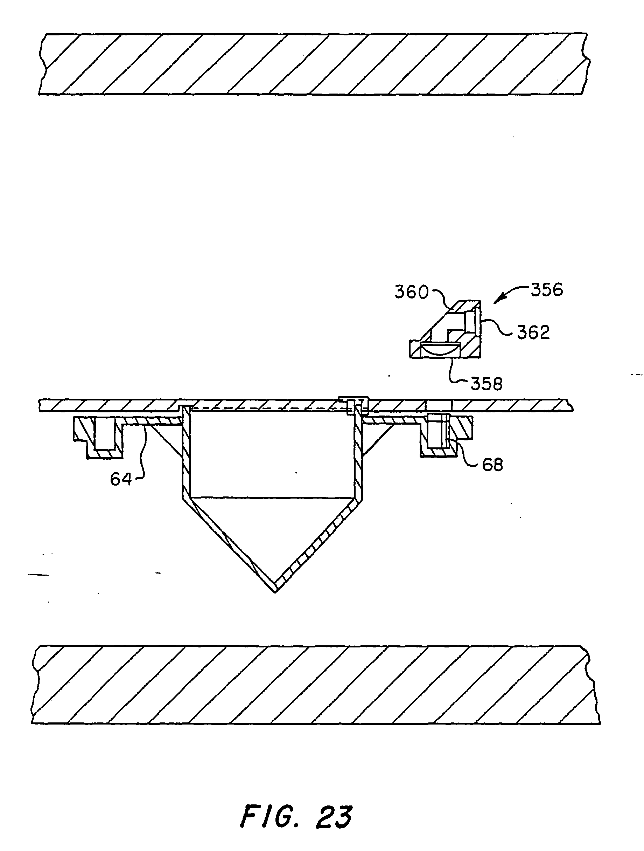

FIGURE 23 is a side sectional view in isolation of a MEIA cartridge carousel of the automated analytical system, MEIA cartridge and MEIA reader.
FIGURE 24 is a schematic of the MEIA system optical assembly of the automated analytical system.
FIGURE 25 is a schematic of the MEIA read sequence of the automated analytical system.
FIGURE 26 is a schematic reaction sequence of a FPIA for T4 performed on the automated analytical system.
FIGURE 27 is a schematic reaction sequence of a one-step sandwich MEIA performed on the automated analytical system.
FIGURE 28 is a schematic reaction sequence of a two-step sandwich MEIA performed on the automated analytical system.

### Description of the Invention

### Definitions

The following definitions are applicable to the present invention:

The term "test sample", as used herein, refers to a material suspected of containing the analyte. The test sample can be used directly as obtained from the source or following a pretreatment to modify the character of the sample. The test sample can be derived from any biological source, such as a physiological fluid, including, blood, saliva, ocular lens fluid, cerebral spinal fluid, sweat, urine, milk, ascites fluid, raucous, synovial fluid, peritoneal fluid, amniotic fluid or the like. The test sample can be pretreated prior to use, such as preparing plasma from blood, diluting viscous fluids, or the like; methods of treatment can involve filtration, distillation, concentration, inactivation of interfering components, and the addition of reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte.

The term "analyte" or "analyte of interest", as used herein, refers to the compound or composition to be detected or measured and which has at least one epitope or binding site. The analyte can be any substance for which there exists a naturally occurring binding member or for which a binding member can be prepared. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), virus particles and metabolites of or antibodies to any of the above substances. The term "analyte" also includes any antigenic substances, haptens, antibodies, macromolecules and combinations thereof.

The term "analyte-analog", as used herein, refers to a substance which cross-reacts with an analyte-specific binding member, although it may do so to a greater or lesser extent than does the analyte itself. The analyte-analog can include a modified analyte as well as a fragmented or synthetic portion of the analyte molecule, so long as the analyte-analog has at least one epitopic site in common with the analyte of interest. An example of an analyte-analog is a synthetic peptide sequence which duplicates at least one epitope of the whole-molecule analyte so that the analyte-analog can bind to an analyte-specific binding member.

The term binding member", as used herein, refers to a member of a binding pair, i.e., two different molecules wherein one of the molecules specifically binds to the second molecule through chemical or physical means. In addition to antigen and antibody binding pair members, other binding pairs include, as examples without limitation, biotin and avidin, carbohydrates and lectins, complementary nucleotide sequences, complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), and the like. Furthermore, binding pairs can include members that are analogs of the original binding member, for example, an analyte-analog or a binding member made by recombinant techniques or molecular engineering. If the binding member is an immunoreactant it can be, for example, a monoclonal or polyclonal antibody, a recombinant protein or recombinant antibody, a chimeric antibody, a mixture(s) or fragment(s) of the foregoing, as well as a preparation of such antibodies, peptides and nucleotides for which suitability for use as binding members is well known to those skilled in the art.

The term "detectable moiety", as used herein, refers to any compound or conventional detectable chemical group having a detectable physical or chemical property and which can be used to label a binding member to form a conjugate therewith. Such detectable chemical group can be, but is not intended to be limited to, enzymatically active groups such as enzymes, enzyme substrates, prosthetic groups or coenzymes; spin labels; fluorescers and fluorogens; chromophores and chromogens; luminescers such as chemiluminescers and bioluminescers; specifically bindable ligands such as biotin and avidin; electroactive species; radioisotopes; toxins; drugs; haptens; DNA; RNA; polysaccharides; polypeptides; liposomes; colored particles and colored microparticles; and the like.

The term "continuous access", as used herein, refers to the ability to add additional test samples or reagents to the automated analytical system of the present invention without the interruption of assays which are being performed by the automated analytical system of the present invention at the time of such addition.

The term "random access", as used herein, refers to the ability of the automated analytical system of the present invention to simultaneously perform more than one scheduled assay in any order in which such plurality of scheduled assays are presented into the automated analytical system of the present invention.

The term "simultaneous", as used herein, refers to the ability of the automated analytical system of the present invention to independently perform two or more scheduled assays at the same time.

The term "kitting", as used herein, refers to the ability of the automated analytical system of the present invention to create a unit dose disposable by separately transferring test samples and reagents to a reaction vessel of the present invention without initiation of an assay reaction sequence.

The term "quat" refers to a polycationic material solution for assays which use these materials which are not an antibody or antigen to capture the analyte from the sample on the matrix of, for example, MEIA cartridge. In the present inventive system, quat is dispensed to the matrix during test processing, prior to the transfer of the reaction mixture from the reaction vessel.

The term "flexible protocols" refers to the variety of different assay protocols capable of being processed in accordance with the inventive system. Examples include MEIA formats configured in 1- and 2-step sandwich and competitive assay formats; order of activity processing, including the ability to initiate sample processing for both MEIA formats and FPIA formats on the front-end carousel prior to transfer onto the process carousel; variable incubation periods; optical read formats and wash sequences. This contrasts to some prior art, known random access systems which force all assay protocols to adhere to a strict "lock-step" format, in which assay configuration (i.e. 1- versus 2-step formats), activity order, incubation timing, and other similar protocols are fixed by the instrument.

### Scheduler

According to the present invention, a system scheduler generates and optimizes the workload for the system's mechanical resources from all the tests ordered to run on the system. The main goal of the scheduler is to keep the system's resources from sitting idle while there are tests remaining to be processed by the system. Keeping each of the resources busy also minimizes the time required by the instrument to perform the tests.

A high-level view of the scheduling process can be broken into two steps: (1) proper scheduling of each of the activities in a test is ensured before the test is kitted, and (2) an attempt to perform each test activity prior to its original scheduled execution time, to minimize resource idle time and increase test throughput in the system.

To enable scheduling a test in advance of its performance in the system, each test's assay protocol contains several timing parameters used in the scheduling process. Each activity of the test contains time values which the scheduler uses to determine which resources the activity requires and the time period that these resources are needed. Also, each activity in the test can be tied to other activities by incubation periods. These incubation periods, which are dictated by the chemistry of the assay, help the scheduler determine the amount of time that must elapse between the execution of two activities. Each incubation period in the assay protocol provides for the minimum and maximum time that may elapse between the execution of each activity. These limits are referred to in the scheduling process as the incubation window for the activities.

In the inventive system, the operator chooses the order that tests are prepared to run on the instrument by selecting the placement of samples on the instrument. The sample placed closest to the pipette station is the first sample prepared to run on the instrument. To guard against evaporation, a test will not be prepared until the scheduler ensures that all resources used by the test's activities will be available at the required times set forth in the test's assay protocol. Preparation of a particular test will be postponed whenever an activity of another test already in the instrument has a resource scheduled at the time it is needed by an activity on that test. The sample preparation area of the instrument will remain idle until the test can be scheduled without conflicting with tests already in the instrument. When proper scheduling of the test can be achieved, the test will be prepared and transferred into the process area.

The second step in the scheduling process is to optimize the workload for each system resource to minimize both the resource's idle time and the time required to perform the resource's workload. once tests are transferred into the process area, the scheduler optimizes the existing schedule for each resource. At predetermined intervals, the scheduler examines the next interval of work for each resource. If there is any idle time in this interval, the scheduler attempts to minimize the idle time by rearranging the resource's workload to eliminate idle time, providing the activities remain within their allowed incubation windows. When optimization of this interval is complete, this section of the workload is performed by the resource at the designated times.

The scheduler continues to prepare samples as long as there are samples on the instrument that have tests ordered to be run. optimization of the resources' workloads will continue until all tests transferred into the system have finished processing.

### Star Procedure

The inventive system allows special priority handling of specific samples identified by the user as being stat samples. A stat sample, as defined by the inventive system, is a sample that must be processed by the instrument in the shortest amount of time possible. Special handling of stat samples occurs both in the front sample entry area and in the processing area of the instrument.

In the inventive system, the operator chooses the order that tests are prepared to run on the instrument by selecting the placement of samples on the instrument. The sample placed closest to the pipette station is the first sample prepared to run on the instrument. This pattern of sample preparation is interrupted whenever the user places a stat test on the instrument. Whenever a stat test is ordered, the system will finish preparing the test on the current sample, and then move directly to the stat sample to prepare all its tests. To guard against evaporation, sample preparation will not begin for a test before proper scheduling of the test's activities in the processing area is ensured.

The system scheduling algorithm is also modified for stat processing. The scheduling algorithm used for normal tests attempts to maximize the number of tests processed in the instrument each hour. This occurs by allowing sufficient time between test activities to enable other tests' activities to be performed in these gaps. The scheduling approach used for stat tests attempts to process this one test in the shortest amount of time possible. Each activity of a stat test is scheduled at the earliest possible time of execution as defined in the test's assay definition. When all activities of a test are guaranteed proper scheduling in the instrument, sample preparation of the test will begin. After all tests on the stat sample are prepared, the system will return to the sample it was working on before it serviced the stat.

Stat tests receive special consideration in the processing area when there is idle time in a resource's workload. At predetermined intervals, the scheduler examines the next interval of work allocated to each resource in the processing area of the system. If there is any idle time during this interval, the scheduler attempts to minimize it by rearranging the resource's workload. Test activities scheduled for this resource that can be performed earlier than they are currently scheduled, as defined by their assay protocols, are moved forward to fill the idle time. Stat test activities are the first candidates to be pulled forward in the workload, thus further decreasing the amount of time needed to process the stat test in the instrument.

The system stat test handling algorithms have been shown to allow stat tests to be processed in the minimum amounts of time possible, without having a negative effect on the instrument's overall throughput of tests per hour.

The automated analytical system of the present invention is capable of performing various assays employing various detection systems known in the art and include, but are not intended to be limited to, spectrophotometric absorbance assay such as end-point reaction analysis and rate of reaction analysis, turbidimetric assays, nephelometric assays, radiative energy attenuation assays (such as those described in U.S. Patent No. 4,496,293 and U.S. Patent No. 4,743,561 and incorporated herein by reference), ion capture assays, colorimetric assays, fluorometric assays, electrochemical detection systems, potentiometric detection systems, amperometric detection system and immunoassays. Immunoassays include, but are not intended to be limited to, heterogeneous immunoassays such as competitive immunoassays, sandwich immunoassays, immunometric immunoassays, and the like, where the amount of a detectable moiety employed therein can be measured and correlated to the amount of analyte present in a test sample.

Generally, in a spectrophotometric assay, such as those performed on the Abbott Spectrum clinical analyzer and the Abbott Spectrum Series II clinical analyzer (Abbott Laboratories, Abbott Park, IL, USA) the interaction in an assay solution between the analyte to be determined and a reagent system specific for the analyte produces a detectable change in the transmittive properties of the assay solution. The change in the transmittive properties refers to the amount of light absorbed or scattered by an assay solution within a particular wavelength band when a beam of light of known intensity is passed through the assay solution. The change in the transmittive properties of an assay solution is measured by passing monochromic light having a known intensity though the assay solution and determining the ratio of the intensity of the transmitted or scattered light to the intensity of the incident light. Nearly all analytes either absorb energy of a specific wavelength or interact in an assay solution with a particular reagent system to produce a detectable change in the transmittive properties of the assay solution, characteristics which have resulted in the development of numerous specific spectrophotometric assays.

Spectrophotometric assays which rely upon the measurement of the change in the transmittive properties of an assay solution as a measure of an analyte in the assay solution include, for example, assays wherein there is a change in the color of the assay when there is a change in the turbidity of the assay solution, that is, turbidimetric or nephelometric assays.

In a colorimetric assay, the change in the transmittive properties of an assay solution is generally referred to as the absorbance of the assay solution and is dependent upon the change in the color of the assay solution due to the interaction of the analyte to be determined and reagent system specific for the analyte. The absorbance of the assay solution is related to the concentration of the analyte in the assay solution. A colorimetric assay utilizes a chromogenic reagent system capable of interacting in an assay solution with the particular analyte of interest, to produce a detectable change in the transmittive properties, specifically the color, of the assay solution. Numerous chromogenic reagent systems useful in the determination of specific analytes have been developed and are commercially available.

The principle of turbidimetric assays is to determine the amount of light scattered or blocked by particulate matter as light passes though an assay solution. In a turbidimetric assay, the analyte of interest interacts with a reagent system specific for the analyte to form a suspension of particles in the assay solution. As a beam of light having a known intensity is passed through an assay solution, the suspension of particles formed by the interaction of the analyte reagent system blocks or scatters the incident light, thereby reducing the intensity of the light transmitted through the assay solution. The change of the transmittive properties in a turbidimetric assay refers to the decrease in the intensity of the light transmitted through an assay solution, is related to the amount of incident light that is scattered or blocked by the suspension of particles, and depends upon the number of particles present and the cross-sectional area of such particles.

A nephelometric assay is similar to a turbidimetric assay in that the analyte of interest interacts with a reagent system specific for the ligand to form a suspension of particles in the assay solution. In a nephelometric assay, the change in the transmittive properties of the assay solution is also related to the amount of incident light scattered or blocked by the suspension of particles, but unlike a turbidimetric assay wherein the intensity of the light transmitted through the assay solution is measured, the scattered or blocked light is measured at an angle to the light incident to the assay solution. Therefore, in a nephelometric assay the change in the transmittive properties refers to the difference in intensities of light incident to the assay solution and light scattered at an angle to the incident light. Turbidimetric and nephelometric assays are utilized in the analysis of blood, urine, spinal fluid, and the like, for the determination of analytes such as proteins wherein there is no comparable colorimetric. assay due to the lack of an effective chromogenic reagent system. Yoe and Klimman, Photoelectric Chemical Analysis, Vol. II: Nephelometry, Wiley & Sons, Inc., New York, 1929, describe various nephelometric assays. various reagents and reagent systems which can be employed for performing spectrophotometric assays on the automated analytical systems of the present invention include, but are not intended to be limited to, those for the simultaneous determination of glucose and urea, such as described in U.S. Patent No. 5,037,738 and incorporated herein by reference. The simultaneous determination of calcium and phosphorous; the simultaneous determination of cholesterol and triglycerides; determining isoenzymes; determining blood ammonia levels, and the like, can be performed on the apparatus and by the methods of the present invention.

Typically in a fluorometric assay, an analyte in an assay solution is chemically or immunologically transformed into a fluorescent complex or conjugate thereby producing a detectable change in the fluorescent properties of the assay solution. The change in the fluorescent properties of the assay solution is measured by exciting the fluorescent complex or conjugate properties produced with monochromatic light of a wavelength within the excitation wavelength band of the fluorescer, and measuring the intensity of the emitted light at a wavelength within the emission wavelength band of the fluorescer. The fluorescent intensity of the emitted light is related to the concentration of the analyte. However, the intensity of the fluorescence emitted by the assay solution may be inhibited when the ligand to be determined complexes with nonfluorescent interferences such as protein or phosphates present in the sample, or when the sample containing the ligand to be determined has sufficient color so as to act as a filter and thereby reduce the intensity of the emitted fluorescence. It is well recognized that in order to maximize the sensitivity and specificity of a fluorometric assay, these inhibiting factors, if present, must be overcome either by removal of the nonfluorescent interferences or color producing material prior to the analysis, or by compensating for the presence of such factors using an internal standard added to a second aliquot of sample and carrying out the entire assay procedure using the aliquot containing the internal standard.

Generally, homogeneous and heterogeneous immunoassays depend upon the ability of a first binding member of a binding member pair to specifically bind to a second binding member of a binding member pair wherein a conjugate, comprising one of such binding members labeled with a detectable moiety, is employed to determine the extent of such binding. For example, where such binding pair members are an analyte and an antibody to such analyte, the extent of binding is determined by the amount of the detectable moiety present in the conjugate, which either has or has not participated in a binding reaction with the analyte, wherein the amount of the detectable moiety detected and measured can be correlated to the amount of analyte present in the test sample.

Homogeneous immunoassays typically are performed in a competitive immunoassay format involving a competition between an analyte from a test sample and a tracer for a limited number of receptor binding sites on an antibody to the analyte. The tracer comprises the analyte or analog thereof labeled with a detectable moiety wherein the concentration of analyte in the test sample determines the amount of the tracer that will specifically bind to the antibody. The amount of the tracer-antibody conjugate produced by such binding may be quantitatively measured and is inversely proportional to the amount of analyte present in the test sample. For example, fluorescent polarization techniques for making such determination, such as in fluorescent polarization immunoassays as described herein, are based on the principle that a fluorescently labeled compound when excited by linearly polarized light will emit fluorescence having a degree of polarization inversely related to its rate of rotation. When a molecule such as a tracer-antibody conjugate having a fluorescent label is excited with a linearly polarized fluorescent molecule it is constrained from rotating between the time light is absorbed and emitted. When a "free" tracer molecule (i.e., unbound to an antibody) is excited by linearly polarized light, its rotation is much faster than the corresponding tracer-antibody conjugate and the molecules are more randomly orientated, therefore, the emitted light is polarized. Accordingly, when plane polarized light is passed through a solution containing the aforementioned reagents, a fluorescent polarization response is detected and correlated to the amount of analyte present in the test sample.

Various fluorescent compounds which can be employed for performing fluorescent polarization assays on the automated analytical system of the present invention include, but are not intended to be limited to, aminofluoresceins, such as described in U.S. Patent No. 4,510,251 and U.S. Patent No. 4,614,823, incorporated herein by reference; triazinylaminofluoresceins, such as described in U.S. Patent No. 4,420,568 and U.S. Patent No. 4,593,089, incorporated herein by reference; carboxyfluoresceins, such as described in U.S. Patent No. 4,668,640, incorporated herein by reference; and the like.

Heterogenous immunoassays typically involve a labeled reagent or tracer comprising an analyte, an analog of the analyte, or an antibody thereto, labeled with a detectable moiety, to form a free species and a bound species. In order to correlate the amount of tracer in one of such species to the amount of analyte present in the test sample, the free species must first be separated from the bound species, which can be accomplished according to methods known in the art employing solid phase materials for the direct immobilization of one of the binding participants in the binding reaction, such as the antibody, analyte or analog of the analyte, wherein one of the binding participants is immobilized on a solid phase material, such as a test tube, beads, particles, microparticles or the matrix of a fibrous material, and the like, according to methods known in the art.

Heterogenous immunoassays can be performed in a competitive immunoassay format as described above wherein, for example, the antibody can be immobilized to a solid phase material whereby upon separation, the amount of the tracer which is bound to such solid phase material can be detected and correlated to the amount of analyte present in the test sample. Another form of a heterogeneous immunoassay employing a solid phase material is referred to as a sandwich immunoassay, which involves contacting a test sample containing, for example, an antigen with a protein such as an antibody or another substance capable of binding the antigen, and which is immobilized on a solid phase material. The solid phase material typically is treated with a second antigen or antibody which has been labeled with a detectable moiety. The second antigen or antibody then becomes bound to the corresponding antigen or antibody on the solid phase material and, following one or more washing steps to remove any unbound material, an indicator material such as a chromogenic substance which reacts with the detectable moiety (e.g., where the detectable moiety is an enzyme, a substrate for such enzyme is added) to produce a color change. The color change is then detected and correlated to the amount of antigen or antibody present in the test sample.

For example, a heterogeneous immunoassay which can be performed by the automated analytical system of the present invention, in either a competitive or sandwich immunoassay format, is a microparticle capture enzyme immunoassay, such as that described in Clinical Chemistry, Volume 34, No. 9, pages 1726-1732 (1988), employing microparticles as the solid phase material.

In addition, the use of sucrose in microparticle diluent has been found to achieve neutral density of the microparticles. The methodology entails the determination of the optimum sucrose concentration which will eliminate the settling of microparticles. The sucrose concentration required to achieve neutral density is assay specific and microparticle lot specific. The principal involves dissolving sucrose in solution to increase the density of the diluent. When the density of the diluent and microparticles are equivalent, the microparticles will be in a suspended state. Density neutralization can also be achieved by using other materials such as metrizamide and/or metrizoic acid.

Separation of the bound and free species is accomplished by capture of the microparticles on a glass fiber matrix of an MEIA cartridge, a process that relies on the high affinity of glass fibers for the microparticles, wherein the microparticles adhere to the surface of the fibers irreversibly, and nonspecifically bound material can be effectively removed by washing the matrix. The matrix also provides a precisely located mechanical support for the microparticles during the optical quantification phase of the assay protocol as described herein.

When performing a sandwich immunoassay, microparticles coated with antibody to the analyte in the test sample are incubated with the test sample containing the analyte of interest to form a capture complex with the analyte from the test sample. A conjugate comprising antibody to the analyte labeled with a detectable moiety, preferably an enzyme, is then incubated with the capture complex to form the second of a sandwich complex. When performing a competitive immunoassay, microparticles coated with antibody to the analyte in the test sample are incubated with the test sample containing the analyte of interest and a conjugate comprising the analyte or analog thereof labeled with a detectable moiety, preferably an enzyme. Removal of unbound conjugate is accomplished with the glass fiber matrix of the MEIA cartridge and, where the detectable moiety is an enzyme, a substrate for the enzyme capable of providing a detectable signal is added and the signal provided thereby is measured and correlated to the amount of analyte present in the test sample. Preferably, the enzyme-substrate system employed by the competitive and sandwich MEIA formats is alkaline phosphatase and 4-methylumbelliferyl phosphate (MUP), although other enzyme-substrate systems known in the art can be employed as well.

The MEIA cartridge which is employed by the automated analytical system of the present invention comprises a reaction well for retaining and immobilizing microparticle-analyte complexes. The reaction well has an entrance port and means for holding a quantity of sample and assay reaction mixtures positioned over a fibrous matrix which retains and immobilizes microparticle-analyte complexes as described above. The fibrous matrix is composed of fibers having an average spatial separation greater than the average diameter of the microparticles. Preferably, the average fiber spatial separation is greater than 10 microns.

The reaction well further comprises an absorbent material positioned below the fibrous matrix to enhance the flow of sample and assay reaction mixtures through the fibrous matrix. Preferably, the absorbent material is a fibrous material whose fibers predominantly lie in a plane perpendicular to the lower surface of the fibrous matrix. The absorbent material is in fluid communication with the fibrous matrix. Generally, the absorbent material is in physical contact with the lower surface of the fibrous matrix. The interior of the reaction well, therefore, is generally sized or contains positioning means to maintain the fluid communication between the absorbent material and the fibrous matrix. Preferably, a spike located at the bottom of the reaction well can be used to force the absorbent material into contact with the lower surface of the fibrous matrix. Additionally, it is preferable to vent to the atmosphere the gases displaced in the absorbent material by the liquids absorbed therein during the performance of an immunoassay.

According to the immunoassay methodologies described above, standard solutions of the analyte of known concentrations covering the clinical concentration range are typically prepared and assayed as is the test sample to be assayed. This blank assay provides a series of signal measurements corresponding to the known concentrations from which a standard curve is drawn. The optical signal corresponding to the unknown sample is correlated in a concentration value through interpretation from the blank or standard curve.

Automated analytical methodology for effecting analysis of a plurality of test samples according to the present invention is achieved by introducing reagent packs, test sample container and reaction vessels onto concentric carousels of a main carousel. The test sample container can be a test tube, cuvette, vacutainer tube, and the like, for holding a test sample. The reagent packs and test sample containers are identified and aligned respectively with a reaction vessel for transfer and kitting of the reaction vessel by transfer of test sample and specific reagents from the reagent pack for preparation of a predetermined test. The reaction vessel containing the test sample and one or more reagents is transferred to a process carousel wherein controlled environment conditions exist for incubation once the sample has been appropriately mixed with various -reagents to form a reaction mixture. When all assay processing steps have been completed, the reaction mixture is identified and transferred to at least, for example, one of a fluorescent polarization immunoassay reader or a microparticle enzyme immunoassay cartridge positioned on a separate cartridge wheel or carousel for further preparation before reading. The processed test samples are read and the readings are calculated with the resulting data being recorded and/or printed.

The methodology of the automated immunoassay analytical system is achieved through the use of a self-contained, fully automated, continuous and random access instrument comprising a main carousel assembly consisting of the reagent pack carousel, a reaction vessel carousel and a test sample container carousel concentrically and independently rotatable. The main carousel assembly is provided with a transfer pipette operated by a boom arm for transferring and kitting test sample and reagents into the reaction vessel automatically following a predetermined test schedule. The main carousel assembly is provided with bar code readers for reagent packs and test sample containers and has the capability of aligning the reagent pack carousel and test sample container carousel and a reaction vessel for pipette transfer operations. Once the assay to be performed is scheduled, the reaction vessel carousel, the reagent pack carousel and the test sample container carousel are rotated until the reaction vessel, a reagent pack and a test sample container, respectively, are determined to be in the transfer pipette access position. The transfer pipette then transfers the test sample from the test sample container and, depending upon the assay to be performed, the reagents from the reagent pack are transferred to the reaction vessel. The reaction vessel carousel is then rotated to a transfer station position which contacts the reaction vessel with a transfer mechanism and pulls the reaction vessel into the transfer station. The reaction vessel is then loaded onto the process carousel by the transfer mechanism.

When performing a fluorescent polarization immunoassay (FPIA) with the automated analytical system of the present invention, various pipetting activities are performed by a second transfer pipette apparatus which is in service for the process carousel, and the process carousel is rotated so that the reaction vessel, when properly pipetted with, for example, FPIA reagents, is at the read station of the FPIA processing stations and the FPIA determination on reading, is made on the reaction vessel. The process carousel is then rotated so that the read reaction vessel is at the transfer station. The reaction vessel is again contacted and transferred by the transfer station. The transfer station is rotated and pushes the reaction vessel into a release container opening.

For a microparticle enzyme immunoassay (MEIA) performed with the automated analytical system of the present invention, after the various pipetting activities for the MEIA, which can be completed at the main carousel assembly, the reaction vessel is transferred to the process carousel as described in the FPIA process. Pipetting can also be accomplished in the process carousel or jointly between the two carousels. To complete the MEIA, the reaction mixture is transferred from the reaction vessel to a matrix of an MEIA cartridge on a cartridge carousel with the second transfer pipette. The matrix is washed with a buffer and a substrate, such as MUP (defined earlier), or other suitable substrate known in the art. The cartridge carousel is then rotated so that the MEIA cartridge is positioned at an MEIA processing assembly and the MEIA determination is made. The MEIA reaction vessel is ejected into the waste container as described for the FPIA reaction vessel. The MEIA cartridge is independently ejected from the cartridge wheel by an ejector at an appropriate ejector station into a waste container.

Preferably, two distinct analytical technologies as described above, FPIA and MEIA, are incorporated into the automated analytical system of the present invention; however, more than two distinct analytical technologies can be incorporated into the inventive system. These methods are complimentary and share a commonality of apparatus and procedural steps, with the FPIA generally being the method of choice for analytes of low molecular weight and MEIA for molecules such as protein hormones, antibodies or analytes of low molecular weight requiring higher sensitivity. The two technologies share system components including the operator control panel, pipetting boom assemblies, fluidic systems, air and liquid reagent heaters, printers, bar code reader and stepper motors. Such commonality of use of system components allows for a compact instrument despite the dual FPIA and MEIA capability.

The FPIA optic systems (such as described in U.S. Patent No. 4,269,511 and incorporated herein by reference) can utilize a polarizing filter which is an electrically switched liquid crystal, maintaining a compact size and avoiding complex and potentially unreliable moving parts. When performing FPIA assays utilizing the automated analytical system of the present invention, the FPIA reagent packs will typically include a tracer comprising the analyte or analog thereof, coupled to a detectable moiety, an antibody specific to that analyte, and a specimen pretreatment reagent. In a preferred FPIA format, the analyte being determined competes with the tracer for a limited number of binding sites on the antibodies specific to the portion or portions of the analyte and tracer. The detectable moiety component of the tracer is preferably a fluorescent moiety selected from the group consisting of fluoresceins, aminofluoresceins, carboxyfluoresceins, fluoresceinamines, and the like, more preferably carboxymethyl-aminomethyl-fluorescein, carboxyethylaminomethyl-carboxyfluorescein, 6-carboxyfluorescein, 5-carboxyfluorescein, succinylanimomethyl-fluorescein, thiourea-aminofluorescein, methoxytrianolylaminofluorescein, aminofluorescein, and the like.

In another embodiment, the FPIA format utilizes a unique, round, plastic, reaction cuvette suitable for fluorescence polarization and absorbance assay technologies which require no orientation other than top-to-bottom. This plastic reaction cuvette has physical characteristics of low birefringence throughout the optical read region as well as stringent dimensional tolerances which allow reproducible absorbance readings. Bifringence is defined as the degree of retardation of the extraordinary ray as it passes through a material. The greater the degree of retardation, the greater will be the level of birefringence. Retardation of the extra-ordinary ray is dependent on the magnitude and direction of the induced stress. Therefore, passing a ray of linearly polarized light through a material with induced stress will result in depolarization of the ray. In order for a cuvette to be utilized for fluorescence polarization measurements, it is important that the cuvette be prepared under conditions which yield minimum stress. The geometry of the cuvette has been designed to utilize the inherent fluidics of automated medical diagnostic instrumentation to minimize the hydrophobic effect of plastic.

MEIA results can be determined by quantifying the rate of fluorescence developed when fluorogenic substrate is converted by the action of an enzyme labeled conjugate. For example, when performing either a competitive MEIA or sandwich MEIA, the specifically bound alkaline phosphatase on the microparticles is detected by addition of the fluorogenic substrate MUP to the matrix. The alkaline phosphatase catalyzes hydrolysis of the MUP to inorganic phosphate and fluorescent 4-methylumbelliferone (4-MU). The liberated 4-mu is detected by the MEIA optics assembly front surface fluorometer which is designed to detect fluorescence of low concentrations of 4-MU without interference by fluorescence of 4-MUP at a wavelength of 367. A system of lenses and optical filters focus filtered light (wavelength = 365) from a mercury arc lamp on to the surface of the matrix and focus emitted fluorescence from 4-MU (wavelength = 448) on to a photo multiplier tube. Like the FPIA optics assembly, the MEIA optics system is compact and has no moving parts. About five percent of the excitation light is detected by a photodiode, allowing normalization of the fluorescence data and generation of a control signal used by the lamp power supply to maintain the intensity of the Excitation light within five percent over the useful life of the lamp. The MEIA post-processor uses linear regression analysis to convert the data from multiple successive determinations of 4-MU fluorescence to a rate which is proportional to the concentration of alkaline phosphatase conjugate specifically bound to the microparticles.

MEIA formats can be run with a multi-position MEIA auxiliary carousel and process carousel as well as a MEIA reagent pack containing microparticle reagent, an alkaline phosphatase conjugate and, in some cases, a dilute buffer specific for the assay being performed. Because the microparticles tend not to settle out of suspension during the course of the assay, they can readily be pipetted. The effective surface area of polystyrene latex microparticles is several fold greater than that of a large diameter polystyrene bead (e.g., one quarter inch beads) commonly used in commercial immunoassays. Because of this large surface area and the very small diffusion distance between analyte and the capture molecules on the surface of the microparticles, the capture phase employed in many of the MEIA methods being performed reaches equilibrium within several minutes, allowing for a full carousel of test samples to be completed in a very short time frame.

Unlike an FPIA, the heterogeneous immunoassays, such as a MEIA, require a separation step as described above. In particular, after incubation of the microparticles with a test sample, the microparticles are separated from the reaction mixture by transfer to the matrix contained in the MEIA cartridge as described above. The matrix provides a precisely located mechanical support for
the microparticles during the subsequent optical read phase-of the assay. This precisely located mechanical support, i.e. the cartridge, is fit into the auxiliary carousel at a predetermined spacing from the reader apparatus by camming means.

### Detailed Description of the Drawings

Preferred embodiments of the automated immunoassay analytical system according to the present invention are presented only with those components of primary interest with respect to the inventive system apparatus and processes of the present invention. The drawings do not illustrate all of the mechanical and electrical elements for driving and controlling the various components of the system, wherein an of such omitted elements may have various known forms which can be readily realized by one of ordinary skill in the art having knowledge of the information provided herein with regard to the mode of operation of the system and the various components and related processes utilized for treating samples and determining analytical results.

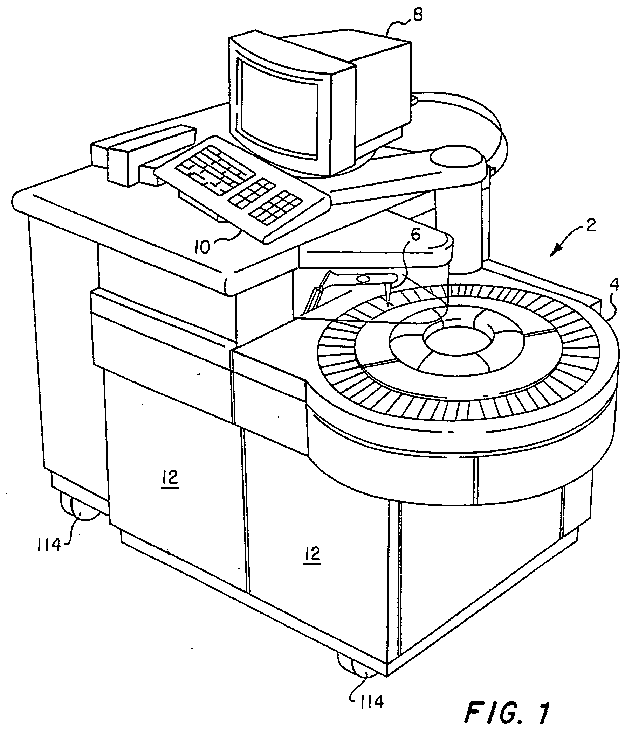

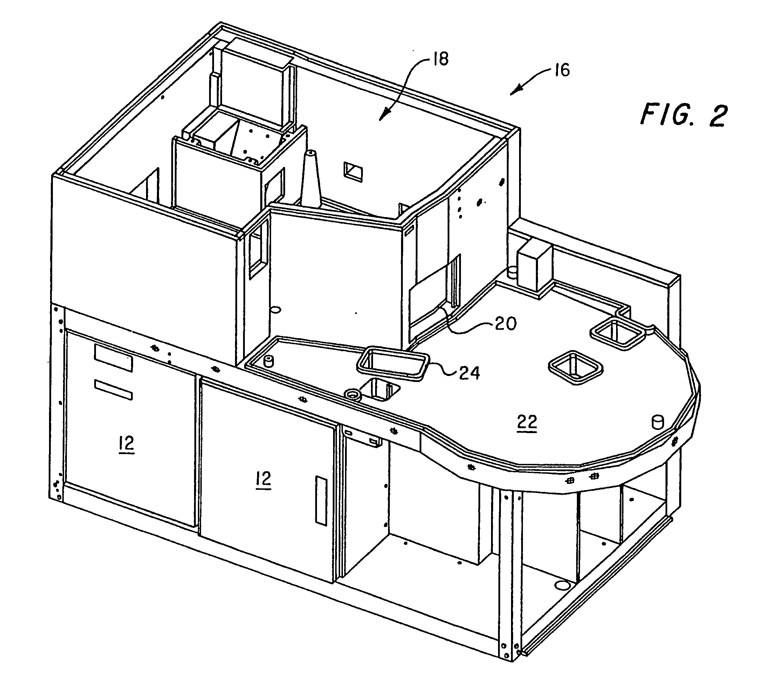

Referring to the drawings, FIGURES 1 and 2 present isometric views of the automatic immunoassay analytical system apparatus of the present invention. The system apparatus as it appears in FIGURE 1 presents the system apparatus as used by the technician, with FIGURE 2 illustrating an isometric view of the frame and cabinetry with component parts removed. The system apparatus of the present invention is identified generally by the reference numeral 2 in FIGURE 1. The system apparatus 2 has an exposed front end carousel 4 which is serviced by a first transfer pipette mechanism 6 for kitting scheduled tests along with samples into a reaction vessel. The system provides a computer screen 8 and computer keyboard 10 along with access panels 12 for accessing storage and waste compartments. The system apparatus 2 is provided with rollers 14 for movement of the system apparatus within a laboratory complex as required. The freedom of movement of the system apparatus through rollers 14 is allowed since the system is fully self-contained but for power requirements.

In FIGURE 2, the system apparatus 2 cabinet frame 16 is illustrated with substantially all functioning components of the system apparatus removed. A controlled environment zone 18 is a closed unit during operation with light shielding and rigid control of airflow as well as temperature as opposed to the open front end carousel 4. The front end carousel 4 communicates with the controlled environment zone 18 through a transfer port 20. The front end carousel 4 is mounted to an aluminum base plate which rests on a support platform 22 and the first transfer pipette mechanism is mounted on means 24.

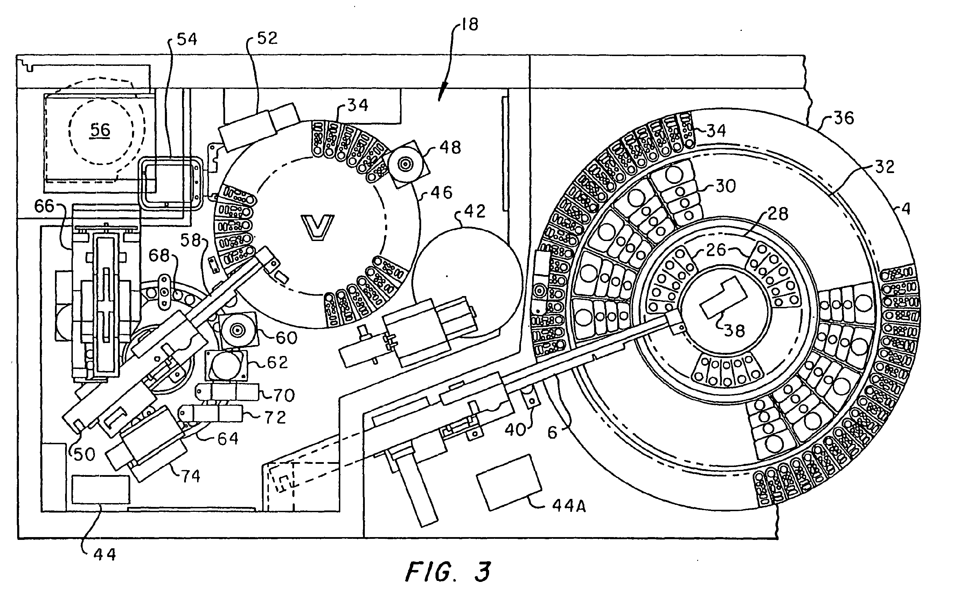

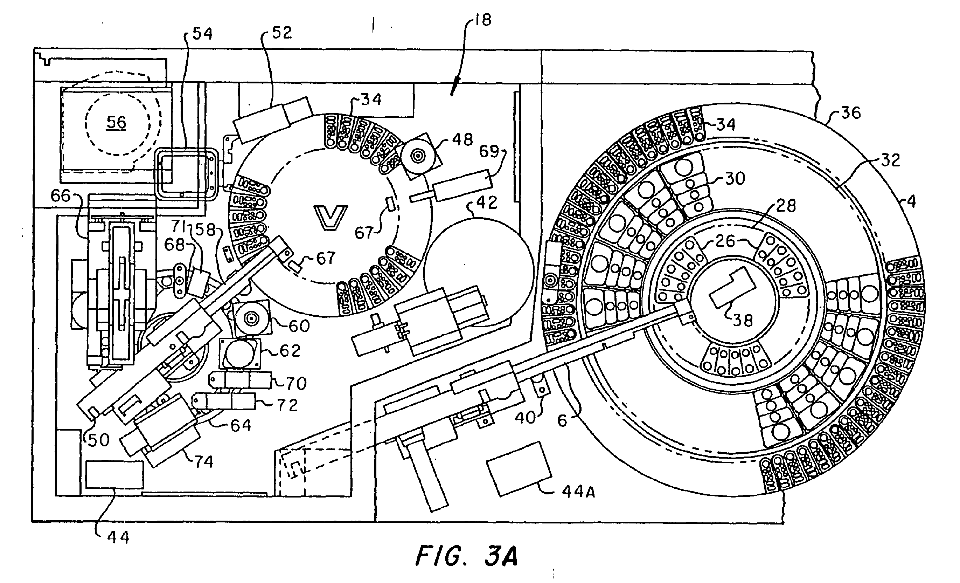

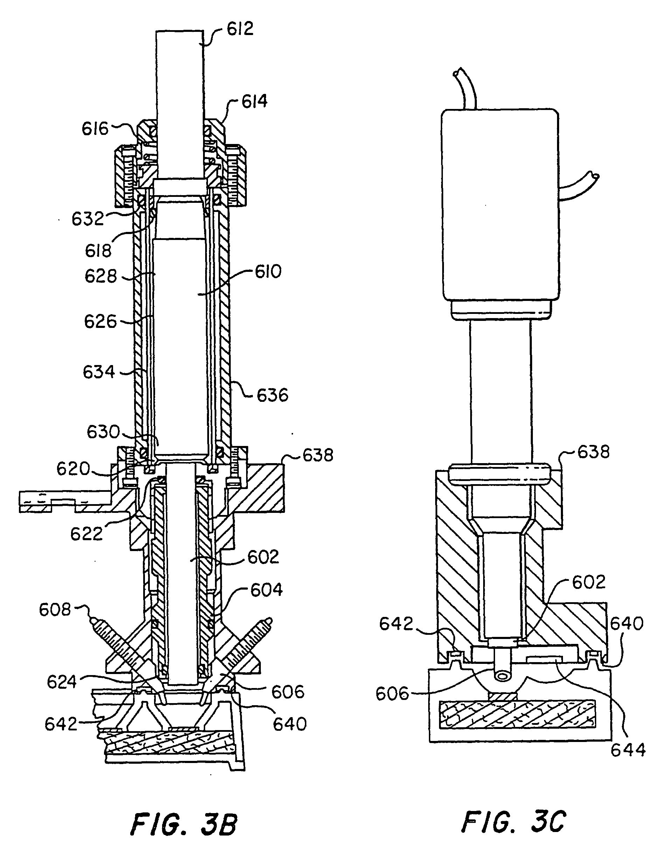

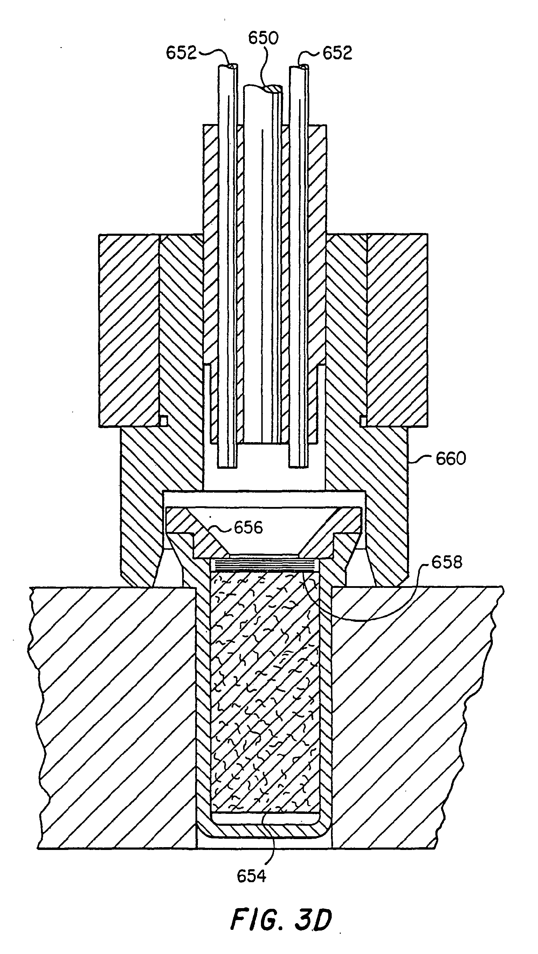

The top plan view in section of FIGURE 3 presents the functioning component system apparatus in some detail with relative positioning of the system apparatus to further illustrate the process flow of the system apparatus. For example, sample cups 26 are mounted on a sample cup carousel 28 which is concentrically fitted within the front end carousel 4 along with reagent pack carousel 32 and reaction vessel carousel 36. The reagent pack carousel 32 is concentrically fitted between the sample cup carousel 28 and the reaction vessel carousel 36. The reagent pack carousel carries reagent packs 30 and the reaction vessel carousel 36 carries reaction vessels 34. The front end carousel 4 has an operable bar code reader 38 for automatically identifying reagent pack carousel 32 and sample carousel 28. A wash cup 40 is provided for the first transfer pipette mechanism 6 for washing as required between transfer of various sample and reagents. The first transfer pipette mechanism 6 is utilized in kitting the various reagent pack liquid materials and sample into a reaction vessel 34. The reagents and the sample are properly kitted through means of the first transfer pipette mechanism 6 inclusive of pump means. The various carousels are rotated and aligned for kitting at the pipetting station. The kitted reaction vessel 34 is positioned by reaction vessel carousel 36 into the proper position for transfer to the transfer station 42. The reaction vessel 34 is transferred to the transfer station 42 through transfer means wherein the transfer station 42 is then rotated to move the reaction vessel onto process carousel 46. As shown, the process carousel is driven by a stepper motor 48 and is serviced by a second transfer pipette mechanism 50. Both the FPIA and MEIA procedures utilize the system apparatus commonly up through and including the process carousel 46. The process carousel 46 includes FPIA processing 52 and FPIA processing lamp 54 for direct reading of FPIA analysis of kitted, pipetted and properly reacted reagents sample from the reaction vessel 34. The controlled environmental zone 18, which includes the transfer station 42 and process carousel 46, provides FPIA processing with air circulation under temperature control by cabinet air circulation fan 56. A wash cup 58 for the second transfer pipette mechanism 50 is provided. The second transfer pipette 50 is utilized for adding reagents (pipetting) under conditions of incubation and timing to the sample in the FPIA test schedule reaction vessel 34 for FPIA processing. MEIA processing can also utilize the second transfer pipette 50 for adding reagents to the sample before the reaction mix is added to MEIA cartridges 68 which are mounted on the cartridge wheel carousel 64. The transfer of the MEIA reagent mixed sample to the MEIA cartridge 68 is by the function of the second transfer pipette 50. A motor 60 drives the cartridge wheel 64. The cartridge wheel 64 is provided with MEIA cartridges 68 through the operation of a cartridge hopper 66 which automatically feeds and positions the MEIA cartridges 68 onto the cartridge wheel 64. The process area includes the second transfer pipette mechanism 50 and heater/pump 44. The cartridge wheel carousel 64 is further serviced by a MEIA buffer heater and dispenser 70, MUP heater and dispenser probe 72, and MEIA reader 74. The MEIA cartridges 68 are removed from the cartridge wheel 64 by a cartridge ejector 62 after the MEIA read has been completed.

According to another embodiment, methods and apparatus for measuring a chemiluminescent signal produced by an immune complex formed with analyte from a test sample such as chemiluminescent homogeneous immunoassays and chemiluminescent heterogeneous immunoassays are provided. According to one embodiment, a chemiluminescent detection signal is produced by an immobilized immune complex comprising antibody coated magnetic particles which are separated by a magnetic field. According to such method, an optical cuvette containing the immune complex bound to magnetic particles suspended in solution is utilized wherein a magnetic field is imposed along the wall of the cuvette to perform the separation. The particles which contain the immune complex are washed, a trigger reagent is added, and the resulting chemiluminescence from the labeled particles is detected and measured in the cuvette using a chemiluminescent detection system. According to another method, analyte is captured in a liquid phase employing, for example, microparticles, polyionic capture agents and the like, having a binding affinity for the analyte wherein the capture analyte is subsequentially immobilized by a porous element and a chemiluminescent signal is then chemically excited and detected. Accordingly, such method within a continuous and random access analytical system advantageously employs fast fusion rates in solution to provide highly sensitive assays for a wide range of analytes. Such methods are particularly useful with an automated, continuous and random access analytical system apparatus as described herein, and which can further include fluorescent and chemiluminescent assay processing on the same platform. Such automated analytical system of the present invention is capable of simultaneously performing two or more assays on a plurality of test samples in a continuous and random access fashion. In particular, the automated immunoassay analytical system apparatus of the invention can be viewed as a microprocessor based system of integrated subassemblies with different groups of assays being run through separate and changeable software modules. The microprocessor based system uses robotic arm pipetters with two degrees of freedom and bidirectional rotating carousels to process samples. Critical assay steps such as incubations, washes and specimen dilution are performed automatically by the instrument as scheduled.

In particular, the automated, continuous and random access analytical system apparatus is capable of performing chemiluminescent assays such as described in commonly owned U.S. Patent No. 5,089,424, which is incorporated herein by reference. In particular, the apparatus includes a container having an aperture and a solid, porous element, preferably in the form of a fibrous matrix, capable of immobilizing a chemiluminescent generating reaction product complex, while at the same time, permitting the passage of other reaction components which are not immobilized by the porous matrix. The reaction product is immobilized by the porous element through particulate reactants or as the result of an interactive property between the porous element and the reaction product such as hydrophilic-hydrophobic binding interactions, ionic binding interactions, and the like. A detection device is situated adjacent to the container which moves to create a light-tight seal with the container to allow low light level chemiluminescent measurements. The detection device includes means for evenly distributing a chemiluminescent activating solution to the porous element. The aperture may be funnel-shaped and the means for applying the activating solution may include ports disposed toward an interior surface of the funnel.

The solid, porous element is preferably in the form of a fibrous matrix and is used to immobilize the immobilizable reaction complex as a result of the interaction therebetween, from which an assay signal can be generated. The porous element can be selected from woven fibrous materials such as glass, cellulose, nylon or other natural or synthetic material known to those skilled in the art. Material choice, dimensions and pore size of these porous elements can be easily selected by those skilled in the art, to provide an effective pore size and adequate void areas to permit proper flow of unreacted reagents and sample through the porous element. It is to be appreciated that the present invention is not intended to be limited to chemiluminescent assays in a heterogeneous immunoassay format as described herein, and that chemiluminescent assays can be performed according to other immunoassay formats known in the art, such as homogeneous immunoassay formats and the like.

FIGURE 3A is a top plan view of the automated analytical system in section with component covers removed to show the automated analytical apparatus in detail and relative position inclusive of a chemiluminescent reader for membrane particle capture technology. The process carousel has two magnetic separation stations 67 and a chemiluminescent reader 69 for magnetic particle capture incorporated thereon for providing chemiluminiscent magnetic particle capture assays. The cartridge wheel carousel has mounted thereon a chemiluminiscent reader 71 for providing microparticle membrane capture assays.

A cross-sectional view of the signal detection module is shown in FIG. 3B and comprises a light guide 602, light-pipe protecting sleeve 604, injectors 606, 608 that are connected to two black Teflon™ fluid lines, photomultiplier tube (PMT) 610, a photomultiplier tube socket 612 and a collar for holding the PMT socket 614. The PMT is springloaded by stainless steel spring wire 616 to ensure proper spacing from the light pipe and is protected from moisture by two O-ring seals 618 and 620. O-rings 622 and 624 fix the light pipe 602 in space and protect the face of the PMT from moisture. O-ring 622 is preferably made of a material with high dielectric constant to prevent Ohmic leakage at the photocathode. A mumetal shield 626 surrounds the PMT, with a nylon spacer 628 inside 626 which protects the PMT during assembly. The mumetal shield 626 is kept electrically isolated by the use of two O-rings 630, 632 and a Teflon™ sleeve 634 to separate it from the outer housing. An electrically conducting outer housing 636 protects the detection module. The bottom of shroud 638 has a groove 640 that locates the surface feature on the disposable device and a light sealing gasket 642 of FIG. 3D. The light sealing gasket is made of black inert compressible polymer. A preferred material is a nylon nap on a rayon backing COE7-1673 (Schlegal Corporation, Rochester, NY). Two low wattage anti-fog heaters 644, 646, are used to create a temperature gradient in the vicinity of the light pipe to prevent any condensation on the light pipe during measurement. A top view of the analyzer system containing the alternate chemiluminescent detection is shown. Magnetic separation stations 67 are positioned adjacent to the optical cuvette on the processing carousel. Trigger reagent is added by the pipettor 50 and the resulting chemiluminescent signal is read by the detector 69.

FIG. 3C is a cross-sectional view taken along the side of the detector device of the present invention and shows a detector assembly, shroud 648, groove 650 and light sealing gasket 642, the anti-fog heating elements 644, 646 and the end of an injector tip 607.

FIG. 3D is an partial cross sectional view illustrating the light pipe 650. The light pipe 650 is shown positioned above the fiber cartridge 654 with fluid injection conduits 652 on either side of the light pipe 650. The fiber cartridge 654 has a funnel 656 and a depth filter 658. A light shield 660 provides light shielding in order to avoid environmental light from interfering with the reading of chemiluminescent released photons which are transmitted through the light pipe 650, generally a quartz light pipe to a photomultiplier tube not shown. The photomultiplier tube assembly inclusive of the light pipe measures the chemiluminescent signal produced on the reaction matrix surfaces of the cartridge 654. During sample processing, an alkaline urea peroxide solution added to the immune complex triggers production of photons of light. The photons are channeled through the quartz light pipe and counted by a photomultiplier tube. The amount of light present (photons) is either directly or inversely proportional to the amount of in light present in the sample.

It is to be understood that the utilization of the first transfer pipette mechanism 6 and the second transfer pipette mechanism 50 as described herein provide a safety mechanism to ensure that test samples and reagents are pipetted to thereby prevent false negative results in the event there are incorrect amounts of the respective sample and reagents for a particular assay.

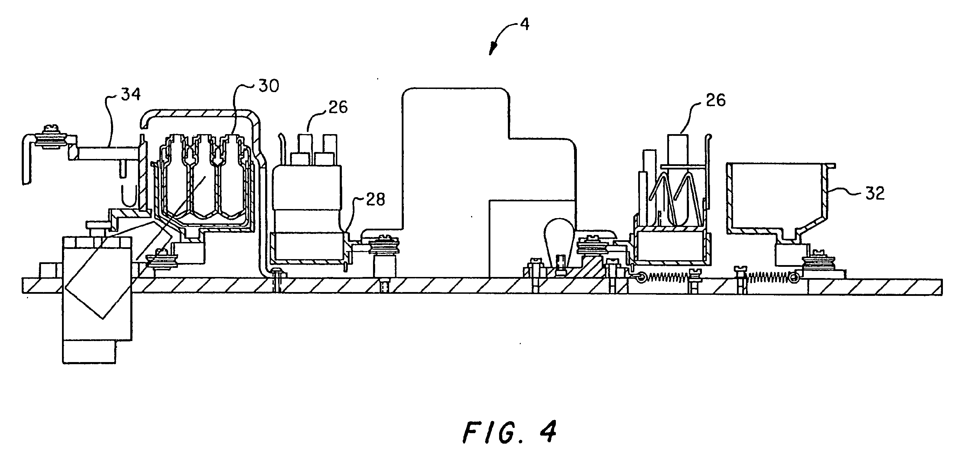

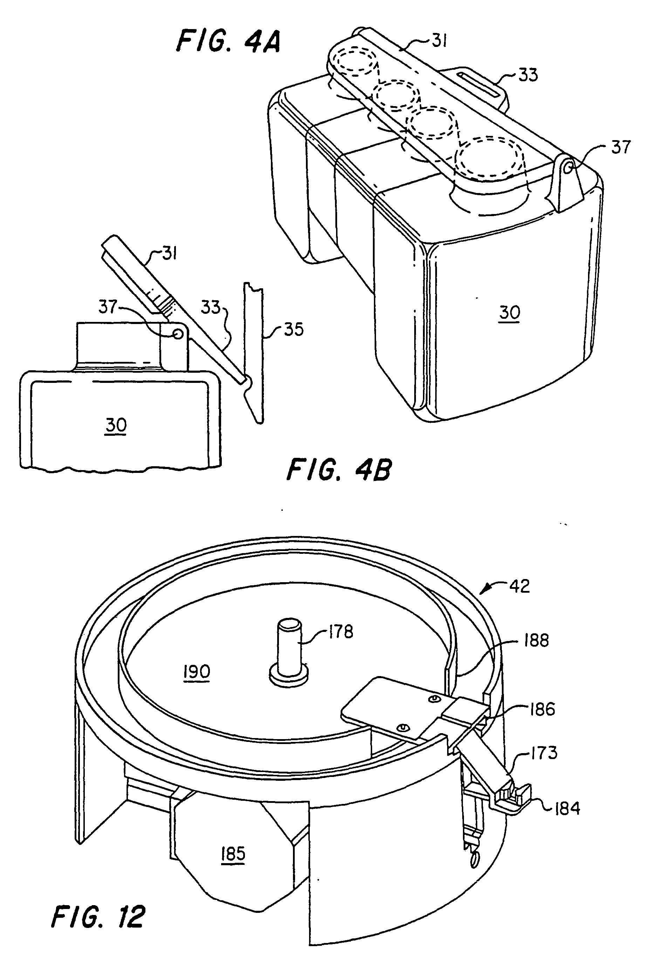

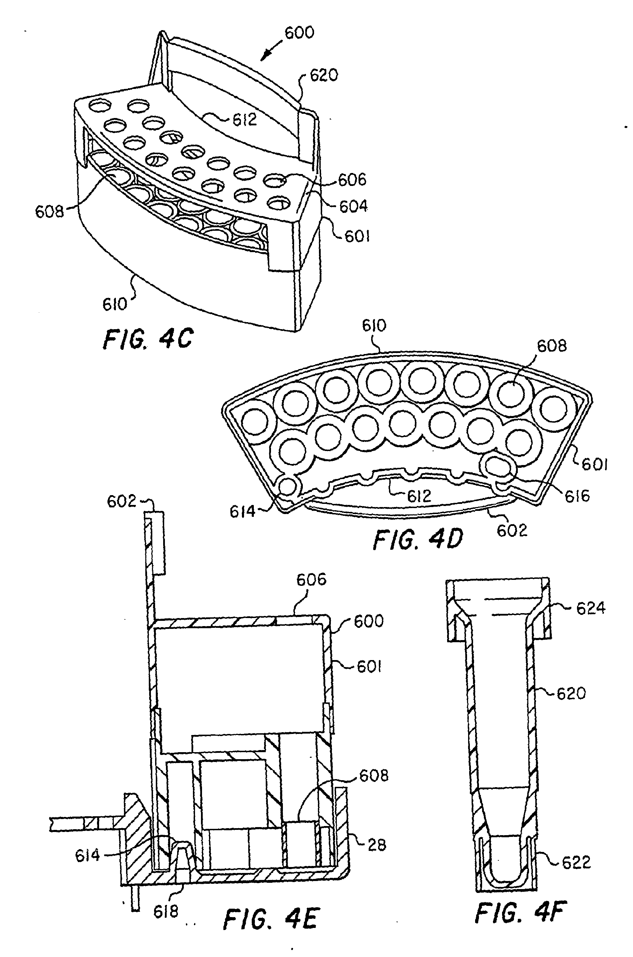

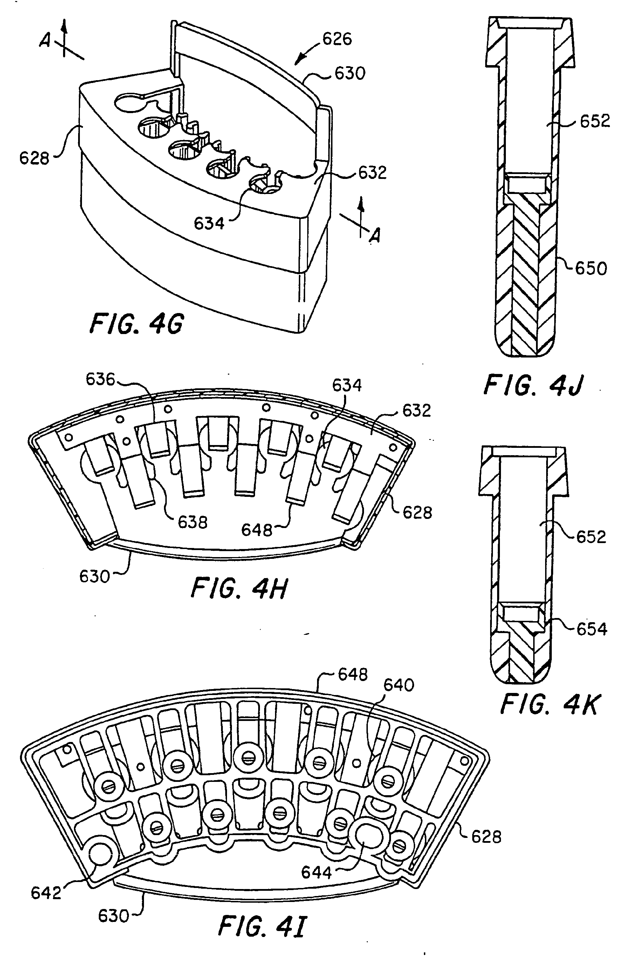

Approaching the operable elements of the system apparatus in greater detail, FIGURE 4 provides a front elevational view in isolation and partial section of elements of the front end carousel 4. FIGURES 4A and 4B illustrate a reagent pack with a cover means 31 which is opened and closed pivoting along axis 37. A return notched drive arm 35 is utilized to open and close the cover means 31 by contact with the cover contact surface 33.

According to another embodiment, a test sample container segment assembly adapted to receive a plurality of test sample containers of varying dimensions for use with an automated analytical instrument is provided. The segment assembly cooperates with a test sample carousel of the automated analytical instrument to provide unlimited variability in test sample containers which can be processed by such automated analytical instrument. Accordingly, the test sample container segment assembly obviates the need to transfer test samples from an otherwise incompatible test sample container to a test sample container which is required for use with an analytical instrument.

In particular, the test sample carousel comprises a plurality of positions for receiving the test sample segments of the present invention. For example, the carousel is preferably adapted to receive six test sample segments, with each segment containing receiving positions for either ten or twelve test sample containers. The segment can accommodate, for example, one or more sample cups and primary tubes, wherein the number of primary tube sizes and dimensions will vary. The primary tubes and sample cups may be mixed within the same sample segment. The different sample segments will support the primary tubes and sample cups so that the aspiration of samples will be accomplished at substantially the same height to provide the automated analytical system with a common level for a probe, which is utilized in combination with pipetting means, to provide sample transfer and kitting of reagents into a reaction vessel on a reaction vessel carousel. Accordingly, since probe pipetting means are in demand in relationship to time and use, the test sample segment assembly enables, for example, less travel and level sense time when such primary tubes and sample cups are utilized to thereby increase throughput of the system. Preferably, each sample segment includes an identifying number and code which is read by an instrument and associated with sample segments for kitting and processing then the automated, random access analytical system instrument. Accordingly, the test sample carousel, in combination with the test sample segment assemblies, provide a maximum amount of accessibility for loading and unloading of sample containers, such as the primary tubes, sample cups, and the like containers as described herein.

The test sample container segment assembly 600 is shown in FIGURE 4C in a perspective view. It is to be understood that test sample containers contemplated according to the present invention include, but are not intended to be limited to, Vacutainer® tubes, test tubes, cuvettes, vials, sample cups such as described in co-pending and commonly owned U.S. Patent Application Serial No. 07/915,165 (filed July 20, 1992) and incorporated herein by reference, and the like, all of which can be of varying sizes and dimensions. The assembly has a frame 601 and a handling means 602. The assembly 600 has a test sample container mounting shelf 604 into which test sample containers can be inserted through container insertion openings 606. Once inserted into the container insertion opening 606, the containers are received and surrounded by liquid level sense sleeves 608. However, insertion openings 606 of the assembly can adequately support and fix said test sample containers without use of sleeves 608. The test sample container segment assembly 600 has two curved dimensions which are in a parallel relationship and are equal to the radius of curvature of the test sample container carousel. The outer curved portion 610 of the test sample container segment assembly 600 and the inner curved 612, both being vertical and in relationship to the container mounting shelf 604 present an assembly which is mateable with the test sample container carousel. The test sample container carousel 28 has positioning and mounting pins which are receivable within pin receivers 614 and 616 of the test sample container segment assembly 600. These pin receiving elements allow an operator to properly position and mount the test sample container segment assembly 600 into the test sample container carousel 28. The test sample container carousel 28 has mounting pins 618 as shown in FIGURE 4E which are received by the test sample container of assembly 600 receiving segments 614 and 616. These receiving segments 614 and 616 are shown in FIGURE 4D, which is a bottom view of the test sample container segment assembly of FIGURE 4C.

A cross sectional view in isolation of the test sample container carousel with a mounted test sample container segment assembly 600 mounted therein is shown in FIGURE 4E. The view in FIGURE 4E clearly illustrates the adaptation of the test sample container segment assembly 600 to the test sample container carousel 28 providing an operator with handling means 602 and alignment pins 618 for fitting into test sample container segment assembly 600 receiving portions 610 and 612.

A cross sectional view of a modified test sample cup 620 (such as described in co-pending U.S. Patent Application Serial No. 915,165) is shown with upper and lower skirt portions 624 and 622, respectively, in FIGURE 4A. Such modified test sample cup 620 can be utilized within the test sample container segment assembly for presenting to the assembly uniform outer dimension which fits routinely into the test sample container segment assembly 600, even though the interior of the test sample cup 620 is buried for various purposes.

A short test sample Vacutainer® tube sample assembly 626 is shown in perspective view in FIGURE 4G. The short test sample Vacutainer® tube segment assembly 626 has a frame 628 and a handling means 630. The assembly has Vacutainer® tube mounting shelf 632 in which Vacutainer® tube insertion opening 634 is provided for mounting the short test sample Vacutainer® tubes into the short test sample Vacutainer® tube segment assembly 626.

A top cross sectional view of the short test sample Vacutainer® tube segment assembly is shown in FIGURE 4H, the view is taken along line A-A of FIGURE 4G. Vacutainer® tube mounting spring means 636 provide a holding means for the insertable Vacutainer® tube elements which are of a tubular or test tube configuration. In addition to the Vacutainer® tube mounting spring means 636, Vacutainer® tube holding arms 638 are presented which further stabilize and maintain the Vacutainer® tubes in a specified position in relationship to the short test sample Vacutainer® tube segment assembly 626 so that when the assembly is inserted into the test sample carousel 28, the test sample Vacutainer® tubes will not only be positioned as to a uniform height, but will also be positioned at a specific location within the carousel and mounted short test sample Vacutainer® tube segment assembly 626.

A bottom view of the short test sample Vacutainer® tube segment assembly of FIGURE 4G is shown in FIGURE 41. Test sample carousel 28 mounting pin receiving elements 642 and 644 provide assembly mounting positioning guides as well as holding means for holding an exact positioned short test sample Vacutainer® tube segment assembly 626 within the test sample carousel 28. In FIGURES 4J and 4K various length test sample cup adaptor sleeves are presented. In FIGURE 4J, a cross sectional view of a long test sample cup adaptor sleeve 650 is shown. In FIGURE 4K, a cross sectional view of a short test sample cup is shown. FIGURES 4J and 4K allow the sample cups of FIGURE 4F to be used in Vacutainer® tube segments.

The sample acquisition carousel utilized in the present instrument preferably has, for example, six positions or sections for mounting test sample container segment assemblies as illustrated in FIGURES 4C and 4G. The test sample segment assemblies are interchangeable, as the operator desires, with positioning pins either on the segments or on the carousel with appropriate receiving means ensuring proper positioning of the segments to the carousel. Accordingly, such interchangeable segments allow the acquisition of a variety of test sample containers which can reside on the carousel at any given point in time. It is to be understood, of course, that the number of sample acquisition carousel positions or sections may vary, and such number will depend upon the size of each portion or section and dimensions of the carousel.

According to the present invention, different types of segment assemblies can be placed in the test sample carousel such as, for example, (1) test sample cup segments which can be used in conjunction with the instrument sample cup 620, wherein the segment can hold about twelve or more of such test sample cups; (2) large Vacutainer® tube segments, which can be used with Vacutainer® tubes from about 0.400 to about 0.650 inches in diameter and about 3.000 to 4.500 inches in length. Such large Vacutainer® tubes can be positioned in the segments to accommodate about ten or more Vacutainer® tubes; and (3) small Vacutaine® tube segments, which can be utilized with the short test sample Vacutainer® tube segment assembly of FIGURE 4G, can accommodate Vacutainer® tubes of about 0.400 to about 0.650 inches in diameter and about 2.000 to 3.000 inches in length, with about ten positions to accommodate about ten or more Vacutainer® tubes.

Sample container adapters are also available which allow sample cup containers to be placed in a Vacutainer® tube segment, particularly for sample cups 620. Such adapters allow sample containers to be used when a minimum number of sample containers are needed and space for a sample container is not available.

Level sensing of fluid in any of the test sample container in the sample segments can be accomplished, for example, by capacitive level sensing. Conductive material is assembled into the segment assemblies and into adaptor sleeves to create a capacitive path. In addition, bar code identification is provided on the segment assemblies and adaptor sleeves. Such bar code identifications are used to identify the segment type and the substitution of, for example, an adaptor sleeve, as well as, of course, identifying the test sample, itself.

In one embodiment, an operator can load empty test sample cups 620 into a test sample segment and pipette test samples into the sample cups 620. The operator may choose to configure the test samples according to a predetermined load list generated via a data entry process. Vacutainer® tube adaptor sleeves and other tubes can, of course, be used in place of a sample cup 620 in the appropriate segment. The operator will then place the test sample segment on the test sample carousel and indicate to the self-contained scheduling and computer system that further test samples are to be processed. The test sample carousel will scan all segments at the appropriate time in order to track all onboard test samples. The instrument will continue, in sequence, to process test samples unless a "stat" test is ordered. When a "stat" test is ordered, the instrument will scan all segments until it finds the one which contains the "stat" test sample. When the stat kitting cycle has completed, the front end carousel will return to a previous sequence. The operator may choose to brace the instrument into a hold phase during loading and unloading of test sample segment assemblies. The kitting process will be suspended after the next kitting cycle. Once loading and unloading is complete, a second instruction will cause the kitting process to resume. The status of onboard test samples will be subject to audit through a data entry screen of the instrument. With such audit, the operator will know which segment assemblies are completed and may be removed. Single test samples may be placed into a segment assembly which is residing on the test sample carousel.

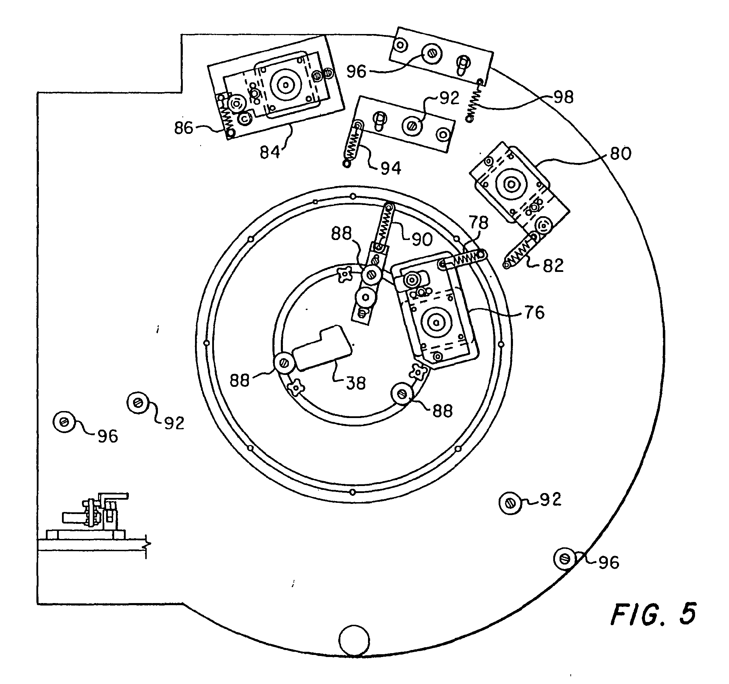

FIGURE 5 provides a top view in isolation and partial section of elements of the drive and guide systems of the main carousel 4 with the various carousels removed. In FIGURE 5 a sample cup carousel stepper motor 76 is shown mounted with mounting spring 78. The reagent pack carousel motor 80 is also shown with a mounting spring 82. The reaction vessel carousel motor 84 and mounting spring 86 are positioned to the exterior of the two inner carousels, i.e. the sample cups carousel 28 and the reagent pack carousel 32. Roller guides 88 are provided for the sample cup carousel 28 and a tensioning spring 90. The reagent pack carousel is provided with roller guides 92 and tensioning means 94. The reaction vessel roller guides 96 are also provided with spring elements 98, the purposes of the guide and these various spring elements being to maintain very finite tracking of the concentric carousels when motivated by the individual stepper motors.

The front end carousel 4 inclusive of the three front end carousels, the sample cup carousel 28, reagent pack carousel 32 and reaction vessel carousel 36 can by example contain the following capacities. The sample cup carousel 28 can hold 60 blood collection tubes, such as Vacutainer blood collection tubes, or 90 sample cups which are injection molded as one piece and can be provided with standalone base mounts. Standalone base mounts are suitable for technician handling and pipetting of samples into the sample cups. The reagent pack carousel 32 provides for 20 different reagent packs 30. The reaction vessel carousel 36 provides 90 reaction vessels 34.

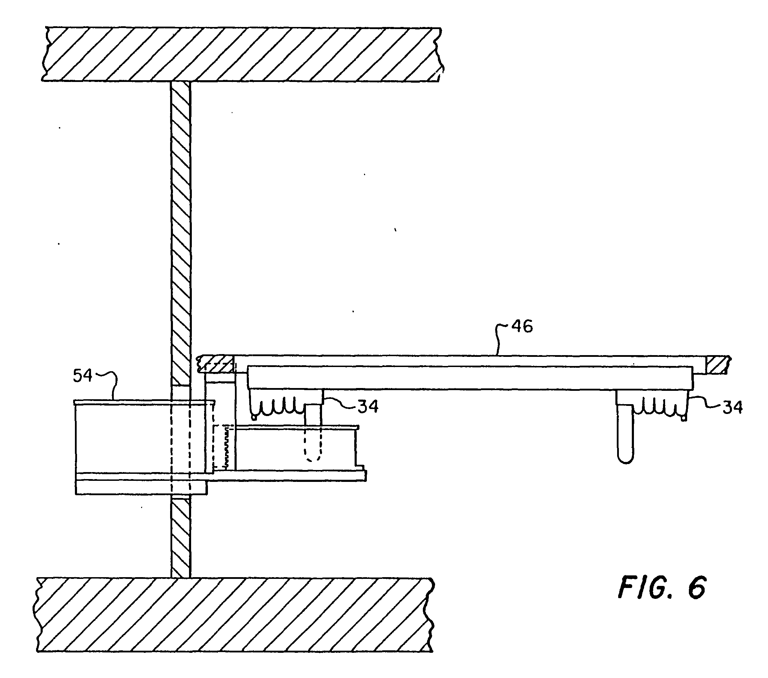

The process carousel 46 as shown in FIGURE 6 is an isolational cross-sectional side view. One reaction vessel 34 is at rest or nonoperative position and a second reaction vessel 34 is in position for FPIA read. The process carousel 46 is capable of bidirectional motion for timely movement of the various reaction vessels 34 to pipettor action, read, or transfer to and from the carousel. Up to about 36 or more reaction vessels 34 can be processed at one time on the process carousel 46 depending on diameter and sizing of the reaction vessels 34.

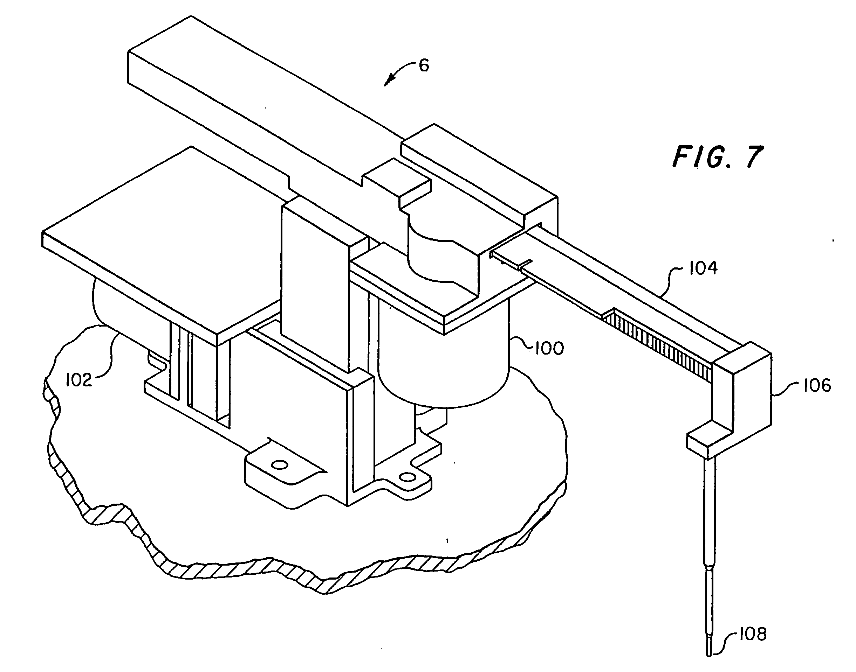

The first transfer pipette mechanism 6 of FIGURE 7 includes a transfer pipette Z axis motor 102 which moves the probe arm 104, probe 106 and probe tip 108 in a vertical direction while transfer pipette R axis motor 100 drives the probe arm 104, probe adjustment means 106 and probe tip 108 in a horizontal motion. The first transfer pipette mechanism 6, sometimes labeled "Sample Probe Arm Mechanism", moves the probe between the sample cup 26, the reagent pack 30, the reaction vessel 34 and the wash cup 40. The wash cup 40 is used to wash the interior and exterior surfaces of the first transfer pipettor mechanism 6 probe. The drive of the first transfer pipette mechanism is a rack-and-pinion drive means along the Z and R axis by two- stepper motor drivers. A brake is provided to hold the Z axis position when power is lost, thus avoiding damage to the system apparatus. For example, the first transfer pipette mechanism can be designed to have a Z axis travel of about 3 inches and an R axis travel of about 11-1/2 inches.

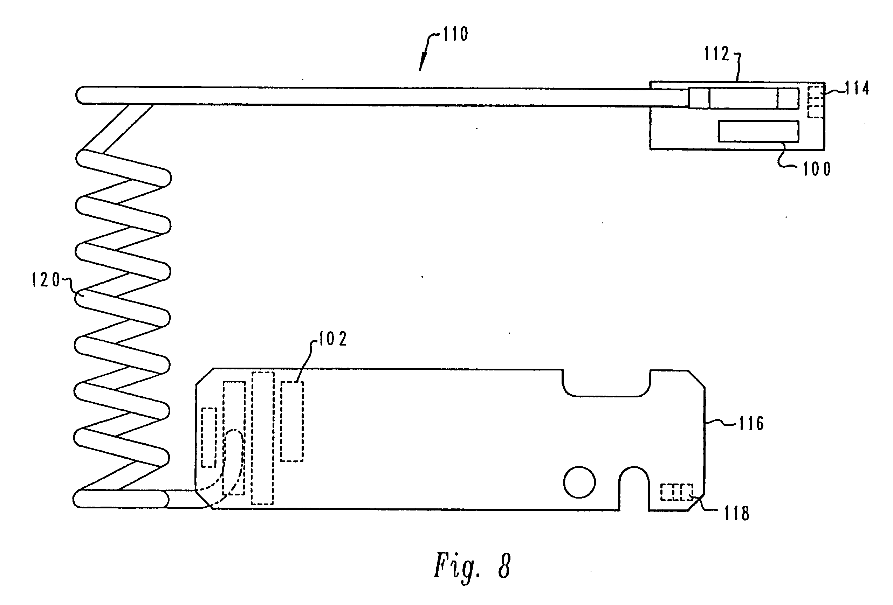

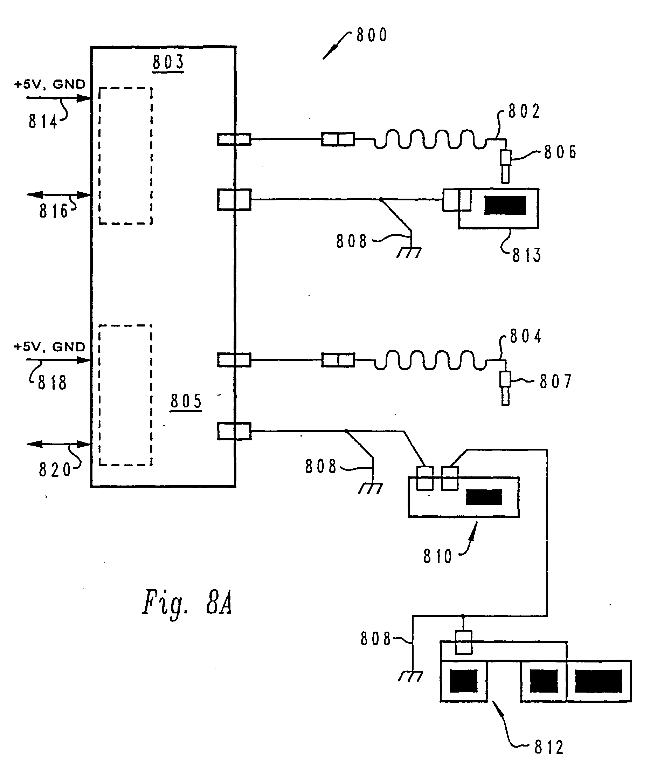

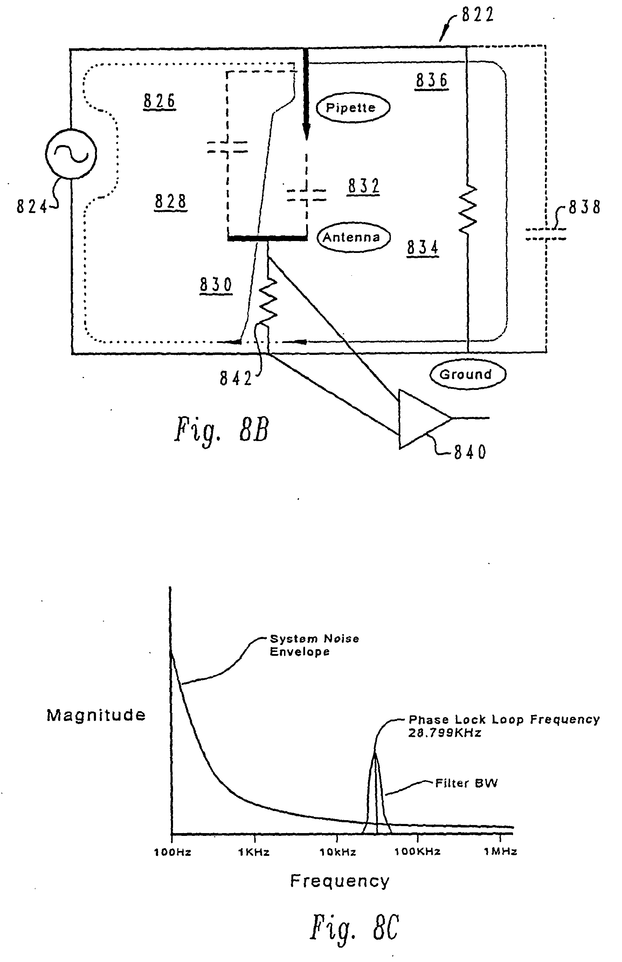

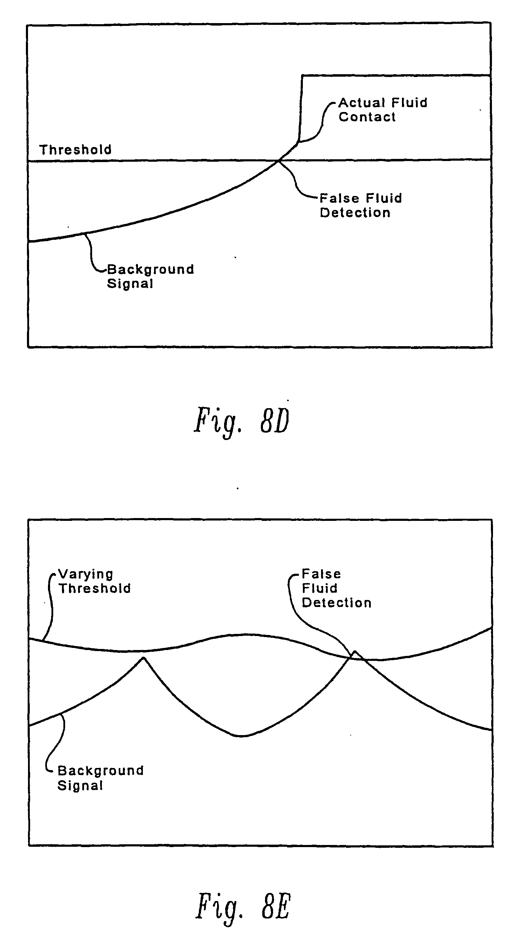

The first transfer pipette mechanism 6 and the second transfer pipette mechanism 50 are closely related in general system apparatus function and design, with variation on travel and size being the only substantial differences. Both units have a probe arm circuit 110 as illustrated by the schematic side view of FIGURE 8. The schematic illustrates the R axis motor 100 and the Z axis motor 102 in relationship to an upper PCB 112 and a R axis home sensor 114. A lower PCB 116 is illustrated in relationship to the Z axis home sensor 118 with a coil cable 120 connecting the various elements.

The present invention also presents two unique approaches to the very necessary and complicated fluidics systems within an automated analytical system. Level sensing by robotic automatic pipetting are presented in two different methodologies, one being by detecting signal amplitude changes associated with a pipettor when it touches a liquid. The significant feature of this liquid level sense is that the signal detection process is based on signal change and rate of signal change, where previous systems have been based on actual signal amplitudes, therefore, being affected by changes induced by temperature, humidity, aging of parts, parts variation, and most significantly, pipettor position. This complementation may be used with conductive diluent in the fluid line to the pipettor. Pipetting is limited to being above a level sense antenna.

A second liquid level sensing methodology for automated analytical systems is presented which does not require an antenna below the pipettor. It requires only a grounded plate, but it cannot be used with conductive fluid in the pipettor line. It can be used with deionized water. It functions by detecting the capacitance change and rate of change when the probe contacts the liquid. The capacitance from probe to ground is measured in the form of an electrical phase shift of a sinusoidal signal present on the liquid probe. The design continually tracks the capacitance of the probe in air and focuses on a quick change in capacitance in response to the probe contacting liquid.

A capacitance liquid level sense 800 is shown in FIGURE 8A as one of the liquid level sense system embodiments according to the present invention. A liquid level sense board is utilized to monitor a probe (pipette, or the like) when enabled by the automated analytical system computer and to stop probe movement when the probe has contacted liquid. As shown in the FIGURE 8A, the board contains process liquid level sense circuits 803 and kitting liquid level sense circuit 805, each completely independent of the other. The liquid level circuit 803 is dedicated to the process center probes and the liquid level circuit 805 is dedicated to the kitting center probe. Process liquid level sense 802 includes a pipette 806 and kitting liquid level sense 804 includes a similar pipette 807. Each of the two circuits are controlled by a calibrated signal and provides two output signals, ready and detect. In operation, "CALIBRATE" is set except when level sensing is desired. The probe is placed over the liquid to sense, preferably immediately over the fluid, and the desired gain bits are set and ready as checked by the controlling computer. When "READY" is asserted, the probe is then moved toward the liquid until liquid is encountered, at which time "DETECT" is set. "DETECT" is routed to the motor controller to stop vertical probe movement, if software has enabled the proper motor control function. The liquid level sense operates by applying a signal to a pipette, probe or the like, made from metal or and otherwise suitable electrically conductive material, and detecting signal changes which occur when the pipette contacts a liquid. An important feature of the liquid level sense is that the signal detection process is based on signal change and rate of signal change conditions and is therefore substantially unaffected by changes caused by temperature, humidity and the like. The liquid level sense system comprises one VME type PC board which contains two separate, independent level sense processing circuits for each level sense circuit, and a probe arm assembly which moves the metal pipettor over the sense position. A coax cable from the system board carries the transmit signal to the pipettor 807 or 806. A receive antenna assembly 806 electrode lies beneath the areas where liquid sensing is desired. The antenna assembly 808 is connected to the board with a triax cable assembly. FIGURE 8A shows the interconnection of the liquid level sense within the environment of an automated, continuous and random access analytical system described herein. It is to be understood that the liquid level sense system of the present invention can be utilized in any automated instrument where liquid level sensing is desired.

The capacitance liquid sense 800 provides a kitting center reaction vessel electrode 810 and a kitting center sample electrode 812, as well as a process area electrode 813. The capacitance liquid level sense 800 further comprises circuitry flow from a VME style PC board back plane 814; motor control via back plane 816; and from VME back plane 818 and motor control via back plane 820. In operation, when the pipette 806 or 807 contacts liquid, the signal from the pipette/liquid combination to the sensor increases above that received before the pipette touches liquid. The transmit/receive operation can be modeled using circuit elements. The transmission media appears as a small capacitance from the pipette to the sensor. The line diluent appears as a large capacitance compared to that from pipette to sensor, when the diluent is conductive or "ionized". Under such conditions, it is difficult and unreliable to detect the signal current 830 as part of the total current 826. Placing the antenna allows only the signal of interest to be detected.

The liquid level sense system operates by detecting signal changes associated with a pipettor when it contacts a liquid. A signal of about 125 KHz is applied to the pipettor, probe or the like, through a low impedance driver. The signal couples across the space between the pipettor and a receive antenna located below the point where liquid sense is desired. As the pipette contacts liquid, the signal transmitted to the antenna increases slightly because the liquid surface, in effect, becomes part of the transmitter, increasing the amount of signal transmitted. The coupling mechanism is primarily by electrical field, which can be mathematically modeled by capacitance. For this reason, the generic type of sensing is referred to as capacitance level sense. Since the electrical field is actually part of an electromagnetic field radiating from the pipette, such sensing devices have also been referred to as "RF" (radio frequency), although the actual frequencies normally employed are about several octaves below standard radio frequencies.

The transmit and receive capacitative liquid level sense of the analytical system described herein employs an electrical sinusoidal signal of about 125 KHz and evaluates the amount of signal as it propagates from probe to a sense antenna. As the probe distance to the antenna changes, and as the probe contacts materials with dielectric constants higher than the surrounding air, the level of the signal reaching the antenna will change. The wavelength of the signal is low compared to the geometries involved, so almost all of the coupling from the probe to antenna is by the electric field. Since capacitance is a theoretical circuit element which models the electrical field, the technique is referred to as "capacitance" level sensing. Circuit analysis tools are refined and easy to use compared to solving electrical field theory problems, so it is common to model complex electrical and magnetic systems as circuit elements or combinations of elements. By applying a low impedance transmit signal to the pipette and receiving the signal with a separate antenna, shunting effects of conductive diluent in the pipettor plumbing can be avoided. FIGURE 8B presents a simplified diagram showing current flow with the sense amplifier measuring only current from the antenna, the diluent current not included. The current pass associated with the transmit and receive capacitance liquid level sense system is shown in FIGURE 8B. As can be seen, current from the transmit source flows into paths. Current leaves the probe, pipette or the like, and flows through diluent to ground and through coupling capacitance to ground, returning to the signal source. Separately, a much smaller current enters the pipette, probe or the like and couples through space to the receive antenna. When the probe, pipette or the like contacts fluid, additional current flows through the additional capacitance added by the increased surface area of the liquid. Since the antenna is positioned to receive mostly signal from the pipette and pipette liquid combination, the signals received can be effectively analyzed to determine if the pipette is contacting liquid. Although the information is present in the current flowing to the pipette, the signal of interest would be very difficult to hold as reliably and repeatedly detect because of the large current flow in the diluent. The simplified current flow 822 of FIGURE 8B has a signal source 824 and a total current 826. Pipette 828 capacitance is shown in the simplified current flow as well as signal current 830 and fluid to antenna capacitance 832. Current in diluent 836 is illustrated in combination with diluents resistance 834 and diluent capacitance 838. The circuitry also illustrates a sense amplifier 840 and sense amplifier input impedance 842.

The system liquid level sense employs a synchronous receiver to provide exceptionally narrow band acceptance of the electrical signals detected by the antennas. The synchronous amplitude detector is one which multiplies the incoming signal by a reference signal to extract amplitude information from the signal. The transmitted signal is generated from the reference signal so that both the transmitted and received signals of interest are of substantially the same frequency. The incoming signal must substantially be in phase with the reference signal. Although the resultant output is complex, the multiplier is followed by a low pass filter of a few KHz to extract the information desired. The filter employed is a Bessel linear phase filter, whereby there is minimal or no overshoot and minimal or no ringing. Synchronous receivers are also referred to as heterodyne receivers and correlation detectors.

FIGURE 8C illustrates the purpose of having a high center frequency and narrow band width. Since the system noise peak is at lower frequencies and reduces as frequency increases, it is advantageous to operate at higher frequencies with narrow band width to reduce noise.

A minimum amount of increase in the received signal enables the system liquid level sense to determine whether fluid has been found. Preferably, such increase will occur rapidly compared to the change that occurs when moving the probe toward the antenna. When the probe, pipettor or the like contacts fluid, the signal increase is sudden. This is accomplished using an autozero loop followed by a simple fixed threshold. The autozero loop timing is such that the output of the Bessel filters is maintained at about zero if the probe is stationary or moving vertically. When a sudden signal change increase occurs due to fluid contact, the autozero circuitry does not prevent the Bessel filter output from increasing. If that increase is sufficient, then a digital bit is output indicating "DETECT", that is, liquid found, at which time the autozero circuit is disabled.

The board mounts in a standard VME card cage, receiving only about +5V and ground from the VME bus. DC/DC converters on the board generate the local operating voltages, isolating the board from the VME bus. There are two liquid level sense circuits, each completely independent. Control signals to and from the board are routed to the system I/O boards. Additionally, the "DETECT" (indicating fluid found) signals are routed to the system motor control boards, so that when fluid is detected, the motor control board can immediately stop the pipettor movement. Each circuit must be referenced to the system ground. The connection for each circuit is done in the immediate vicinity of the sensing area. One circuit operates in the system process area and one circuit operates in the kitting center. Each circuit applies a transmit signal to a pipette through a coax cable. Also, each circuit connects to a sensing "antenna" by a triax cable, with the circuit ground on the outermost conductor. That outermost connector, in addition to connecting to the antenna, connects to the system baseplate adjacent the antenna, providing the ground reference for each circuit. The inner shield of the triax cable is a "driven shield", wherein the signal on the inner conductor is applied to a buffer which, in turn, drives the middle shield. This reduces the effect of the capacitance of the cable and antenna on the sense signal by a factor of about ten or more.

Each circuit is controlled by a "CALIBRATE" signal and three gain bits, all optically isolated. Each liquid level sense circuit outputs two optically isolated signals, "READY" and "DETECT". In operation, "CALIBRATE" is set except when level sensing is required. The "CALIBRATE" control forces the liquid level sense board into an autozero mode, where the analog output of the liquid level sense is forced to zero. This is done so that, after the "CALIBRATE" is removed, the analog output will be simply the change in the signal, not the absolute value. The pipettor is placed preferably immediately over the fluid to sense, the desired gain bits are set, and "READY" is checked by the controlling computer. When "READY" is asserted by the liquid level sense, indicating the analog output has been forced to zero, the system microprocessor removes the "CALIBRATE" command and moves the pipettor downward until fluid is contacted, at which time "DETECT" is set. "DETECT" will remain set as long as the pipette is in fluid. After removal from fluid, "DETECT" resets, but will set again if fluid is contacted again. When the pipette is withdrawn from the fluid and fluid sense is no longer required, "CALIBRATE" is again asserted. In "CALIBRATE" mode "DETECTS" do not occur, being disabled logically, regardless of the activity of the analog signal received.

The liquid level sense board will only pass rapid signal changes, and if the amount of signal change is sufficient to cross a present value, then a "DETECT" signal is output from the board. An autozero circuit nulls the analog output to zero before each level sense operation, as long as "CALIBRATE" is set After "CALIBRATE" is removed, the autozero circuit operates slowly during sensing operation, causing slowly changing signals, such as that caused by pipette movement, to be nulled to zero. Rapid signals, such as those caused by fluid contact, are not immediately affected by autozero. If the fast signal is larger than the fixed threshold, then "DETECT" is triggered and autozero is disabled until "CALIBRATE" is reasserted.

Bessel filters are used in the transmit circuits for repeatability and in the receive circuit for minimum ringing due to noise spikes, meaning minimum noise levels. Sharper filters have potentially high overshoots and actually result in a higher noise level.

According to another embodiment, the invention presents a liquid sensing device which detects the capacitance change when a probe contacts a liquid. Capacitance is sensed between the probe and system ground. This type requires no antenna, but must use only deionized diluent or no diluent. This type is more tolerant of spacing of liquid to ground. The capacitance is measured in the form of electrical phase shift of a sinusoidal signal present on a test sample probe. This liquid level sense continually tracks the capacitance of the probe in air and looks for a rapid change in capacitance in response to contacting liquid. The design uses a phase synchronous detector, followed by a phase locked loop (for background tracking) coupled with detection logic for liquid sensing. The center frequency of the synchronous detection is from between about 27.000 KHz and about 30.000 KHz, preferably about 28.799 KHz. The rejection filter band width for tracking is preferably about ± 1.78 KHz around the center frequency. The rejection filter band width for detection is preferably about ± 85 Hz around the center frequency. FIGURE 8C illustrates the importance of having a high center frequency along with a narrow filter band width pin. Since the system noise (dominated by motor noise) peak is at the lower frequencies and reduces as the frequency increases, it is preferred to operate at higher frequencies and with a narrow band width to eliminate noise problems.

The present liquid level sense device has a tracking function that dynamically subtracts out the background signal and looks for a rapid change in capacitance (contact with liquid) to trigger a detection. Existing fluid sense systems report a fluid detection when the impedance present at the probe exceeds a threshold set by adjusting a sensitivity potential. It is to be understood that since a background signal is not constant, varying due to temperature, humidity, physical proximity to surrounding apparatus and the like surrounding environmental conditions, it has been the cause of a number of problems being experienced with various equipment. According to the present invention, such a fixed threshold does not vary due to temperature, humidity and age.

The result of threshold and background variations can result in non-optimal operation of existing liquid level sense devices. In FIGURES 8D and 8E, additions are illustrated wherein the threshold is crossed (fluid detection) even though the probe is not in contact with the liquid. FIGURE 8D depicts the rise in "background" signal as the sample probe drives into a test sample cup or tube. If the threshold is set too low, a false liquid detection can occur. FIGURE 8E illustrates the background signal as a sample probe drives in and out of an empty test sample cup. On the second drive into the test sample cup, the threshold has varied enough to allow a false detection.

The preferred 28.799 KHz sine wave is one of the inputs (Y) to the phase detector and should be considered the reference for phase detection. The tracker and variable phase control circuitry maintain the output of the phase detector/filter1 at theta volts. Since the transfer function of the phase detector/filter1 is XYcos(0), where theta equals phase angle between the reference signal (Y) and the probe signal (X), the signal from the probe is preferably about 90° out of phase with respect to the reference signal, which is maintained by a variable phase control circuit. The variable phase control circuit is controlled by a tracking circuit that senses the phase detector/filter1 output, compares it to the desired output (about zero volts), and sends a control voltage to the variable phase control circuit. The rate at which the tracker responds depends upon the sample clock speed. The sample clock has two speeds, one for fast tracking of the background during non-fluid sensing movements (sample arm above Z axis home flag movements and horizontal axis movements) and one for slower tracking of the background for fluid sensing movements (sample arm below Z axis home flag movements). The slower tracking is required so that the fluid detection occurs prior to the signal being nulled by the tracking function. When the fluid is detected, the tracking function is halted by a latch until the below home sensor clears the latch. Fluid detection occurs when the phase detector output, after the low pass filters, exceeds the threshold voltage (about 3V) for a time longer than the set delay period of about 1.1 ms (milliseconds). If at any time the signal falls below the threshold prior to the delay period being completed, the delay counter resets to zero and waits for another detection. When a true detection occurs (signal above threshold for longer than the delay period), the fluid detection triggers a user selectable delay. The delay circuit can be set for a delay from between about zero and about fifteen steps into fluid. Upon completion of the user selectable delay, the fluid sense flag is activated, informing the system that fluid was detected.

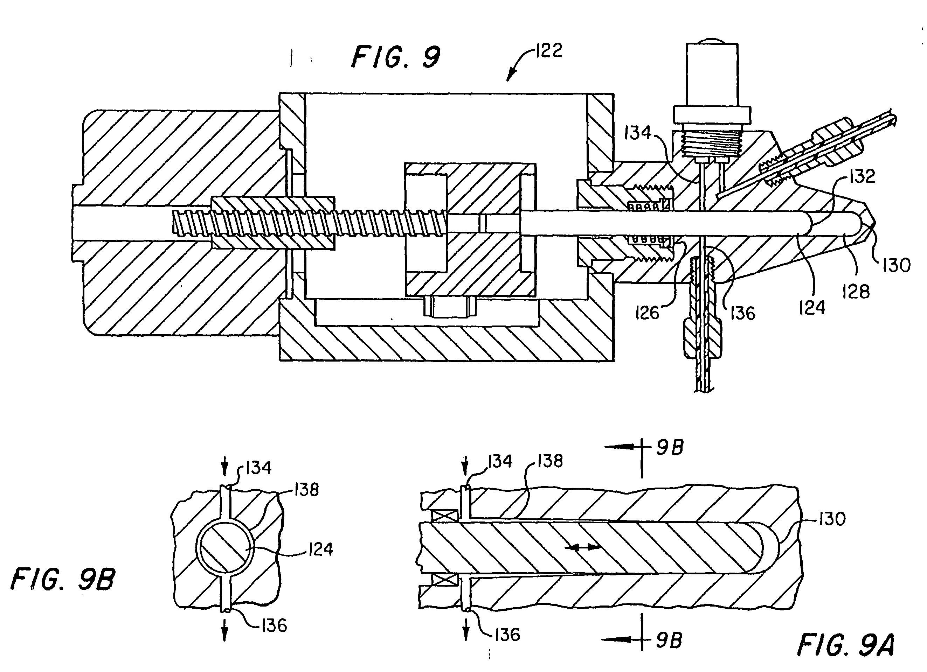

Various elements of syringe 122 which provides automatic bubble flushing and fluids to the various pipetting mechanisms is provided in various views in FIGURES 9, 9A and 9B. The ability of diagnostic instrumentation to accurately perform an assay is critically dependent on the precision and accuracy with which syringes, i.e. pipetting, can aspirate and dispense reagents and samples. The precision and accuracy of a syringe is severely degraded by the presence of small air bubbles inside a syringe. Bubbles, unfortunately, are all too common and are difficult to remove or avoid. Syringe 122 avoids these problems by automatically flushing bubbles completely out of the fluidics system. The syringe 122 is configured such that a piston 124 reciprocates through a seal 126 and into a close-fitting bore 128. The end of the bore 130 is closed. The piston 124 has a piston end 132 which approximates the geometry of the closed bore end 130. Two ports to the bore are 1800 apart and are located near the seal and are comprised of a fluid entry port 134 and a fluid exit port 136. An annulus 138 exists between the piston 124 and bore 128. Pressurized line diluent is introduced to the fluid entry port 134. The fluid flows out into the annulus 138 around both sides of the piston 124 and then into the fluid exit port 136. This crossflow flushes bubbles from the area near the seal. While the crossflow is occurring, the piston 124 is reciprocated inside the bore 128. This reciprocation causes high fluid flow velocities in the annulus 138 between the piston 124 and the bore 128. The high flow velocity dislodges any bubbles that may be adhering to the piston 124 or bore wall. The inward stroke of the piston 124 pushes these dislodged bubbles across the crossflow area where they are swept out of the syringe. The piston end 132 and the bore end 130 have similar spherical shapes. When the piston 124 strokes to its full inward extension, it comes very close to the bore end 130. Any bubble that may be stuck on the bore end 130 is disrupted and dislodged. Likewise, when the piston strokes to its full outward extension, its end is flush with the seal 126. The sequence of reciprocating the piston while crossflowing can be automatically executed any time by the system apparatus.

Once the fluid leaves the fluid exit port 136 of the syringe 122, it must travel through a tube fitting, through a length of tubing, through another tube fitting, into a probe 106 and out the probe tip 108. It is at the probe tip 108 that the aspirating and dispensing of reagents actually occurs. Any bubbles trapped between the syringe and the probe tip will also degrade performance, so there must be no place for the bubbles flushed out of the syringe to lodge. It is therefore necessary to use zero dead volume tubing fittings on the tubing between the syringe and the probe.

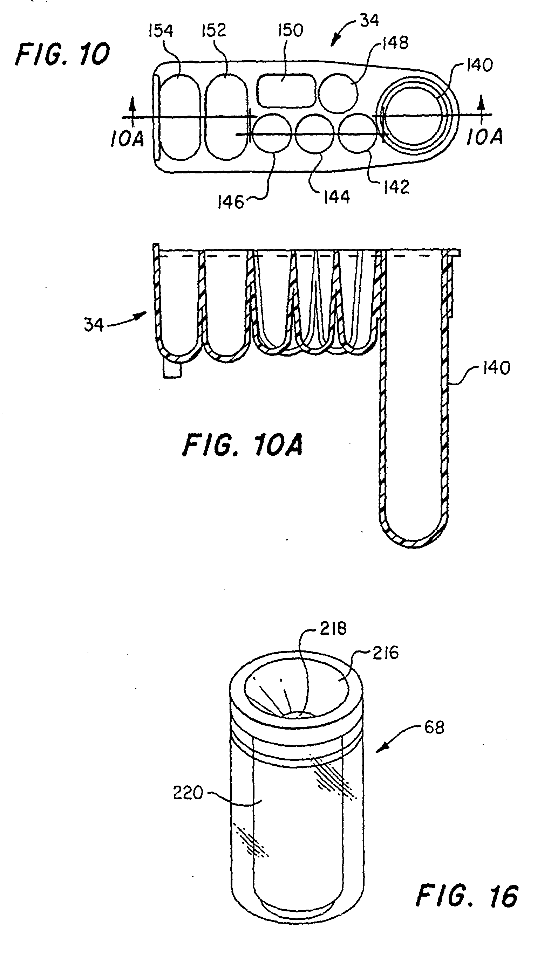

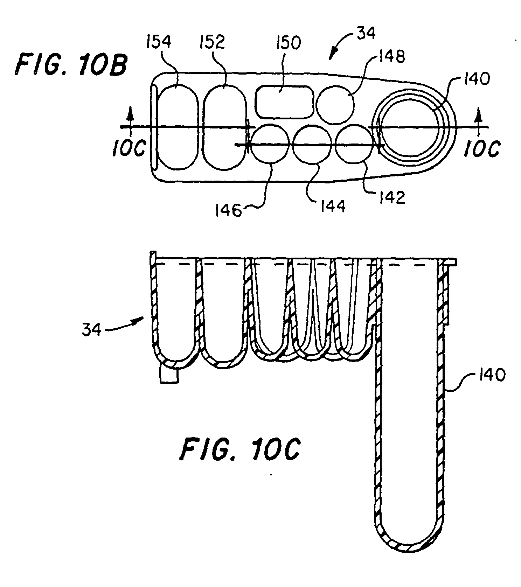

The reaction vessel 34 is discussed in detail relative to either the MEIA scheduling or the FPIA scheduling in FIGURES 10, 10A, 10B and 10C. FIGURES 10 and 10A present the FPIA kitting utilization wherein cuvette 140 is illustrated in both the top plan view, FIGURE 10, and the side view, FIGURE 10A. S reagent antiserum is deposited in well 142 while T reagent tracer is deposited in well 144 with P reagent popper being deposited in well 146. Wells 150 and 152 can serve for providing a variety of reagents, buffers and/or dilution liquids to the apparatus. The sample is deposited in well 148 and predilution liquid in well 154. The utilization of the transfer pipettor in depositing the required reagents into a reaction vessel along with the sample is called kitting. The depositing of the various required reagents and the like into a single reaction vessel along with a sample to be analyzed is called pipetting.

The MEIA reaction vessel as shown in top and side views of FIGURES 10B and 10C, respectively, contains prediluent in well 156; microparticle materials being deposited in well 158; conjugate directly in the reaction well 166; assay diluent in well 162; and the sample in well 164. The buffer well is 168 and predilution well is 170. Once kitting is complete, many of the subsequent FPIA and MEIA pipetting steps can be performed either in the main carousel or in the process carousel utilizing the pipetting mechanisms of both carousels. This is possible because the kitted reaction vessel, once kitted, is transferred immediately into the transfer station and thus into the process carousel which exists in a controlled temperature environment.

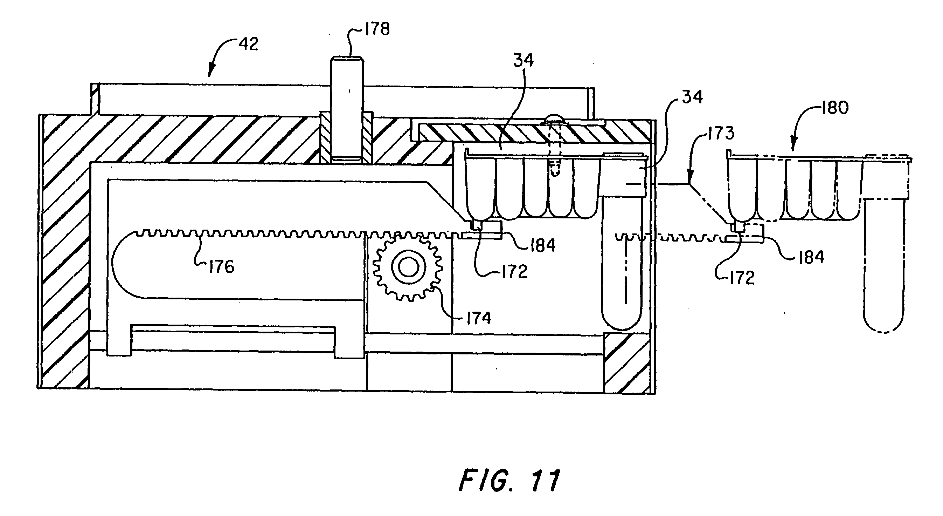

The transfer station 42 plays a key role in apparatus and process function. In FIGURE 11, a sectional side view of the transfer element of the transfer station 42 is shown engaging reaction vessel 34 by means of a reaction vessel transfer projection 172. The transfer arm 173 is projected out between reaction vessel elements of the reaction vessel carousel 36 and, by rotation of the transfer station 42, engages the reaction vessel transfer projection 172. By means of a transfer arm drive gear 174, the transfer arm 173 rack gear 176 moves the transfer arm 173 out and in relationship to the transfer station 42. The transfer station 42 has a rotation axis 178. In FIGURE 11A, a reaction vessel is shown in phantom as would be mounted on the front end carousel 4, reaction vessel carousel 36 engaged by the transfer arm 173 by means of reaction vessel transfer projection 172. The reaction vessel 34 in FIGURE 11 is illustrated onboard the transfer station by reaction transfer station 42 moves the reaction vessel 34 between the front end carousel 4 and the process carousel 46. The transfer station 42 moves the discarded reaction vessel 34 from the process carousel 46 to the waste ejection station (not shown). The transfer station 42 is driven by a stepper motor drive and is supported by precision linear ball bearings and axis of rotation ball bearings.

The process carousel 46 holds, for example, 36 reaction vessels 34 and has a carousel diameter of about 12.5 inches. The process carousel 46 moves the reaction vessel 34 between the transfer station 42, the second transfer pipettor mechanism 50, the point of pipetting, and the FPIA reader processing 52. The process carousel 46 is driven by a stepper motor and supported by three wheels for height control and control of any radial movement caused by irregularly shaped carousel elements.

The second transfer pipette mechanism 50 moves the pipette probe between the wells in the reaction vessel 34 on the process carousel 46 to the MEIA cartridge 68 on the auxiliary carousel 64 and to the wash cup 58. A rack-and-pinion drive through two axis stepper motor drives achieves precision drive on both the R and Z axis. Travel, for example, on the Z axis can be about 3 inches and on the R axis about 4.5 to 5.0 inches.

The auxiliary carousel 64 holds, for example, 32 MEIA cartridges 68 and has a diameter of about 9.5 inches. The auxiliary carouser 64 moves the MEIA cartridges 68 between various stations including the second transfer pipettor mechanism pipette point, the MUP dispense station 72, the MEIA wash station 70 and the MEIA reader 74 and the MEIA cartridge ejection point 62. The auxiliary carousel 64 is stepper motor driven and is carried by three wheels with one wheel located at the Z axis height control at the cartridge insertion point, the second wheel at the pipette point, and the third wheel at the MEIA reader in order to maintain the auxiliary carousel 64 within desired geometric relationships to these various functions.

MEIA cartridges 68 are loaded into a cartridge hopper 66 which feeds the MEIA cartridges 68 into the auxiliary carousel 64. The automatic feeding of the MEIA cartridges 68 is provided with a proper height adjustment of the cartridge 68 into the auxiliary carousel 64 as required by MEIA reading. The cartridge hopper 66 feeds individual cartridges 68 to the auxiliary carousel 64 and changes the axis of orientation of the cartridge 68 from horizontal to vertical by automatic means. Removal of the MEIA cartridges 68 is achieved through the use of an ejector 62 which operates through an ejection rod and forces the MEIA cartridge 68 from the auxiliary carousel 64 which is dropped into a solid waste container.

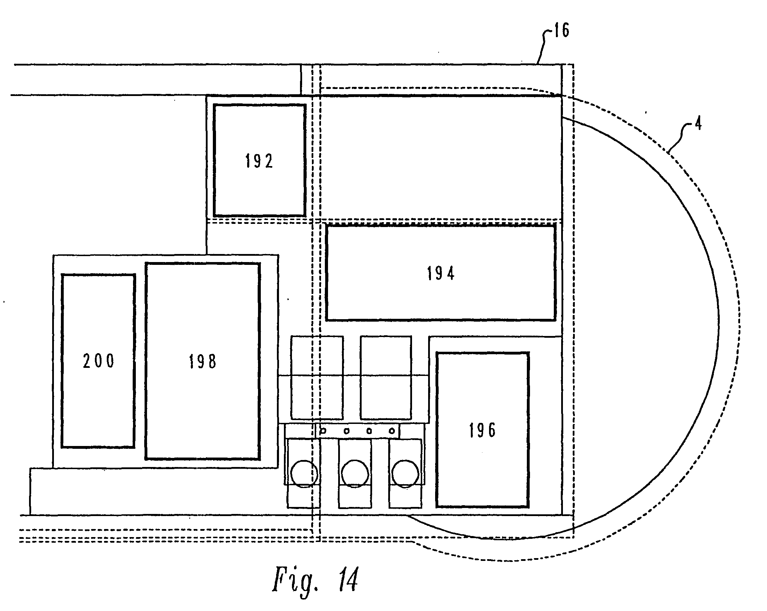

Buffer supply stations are presented in FIGURE 14 which is a top plan view in section of the apparatus showing the cabinet frame 16, front end carousel 4 in partial phantom and a power supply element 192 along with diluent system or buffer pressurization means 194. A supply bottle 196 is also mounted in the lower cabinet of frame 16 as well as solid waste 198 and liquid waste 200 containers for receiving processed liquids and solid waste.

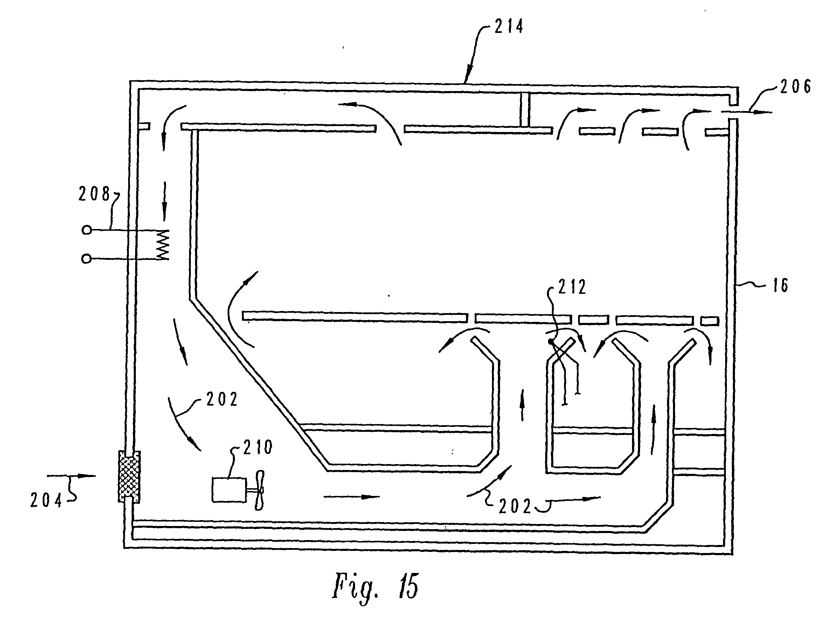

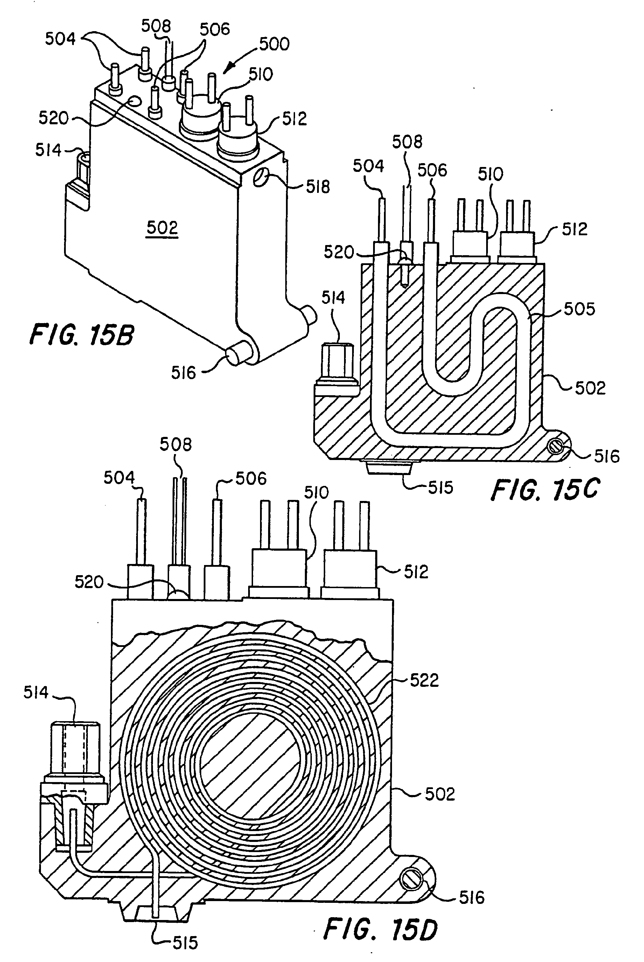

A schematic view illustrating the environmental airflow and temperature control system is shown in FIGURE 15 wherein make up air 204 enters and hot air exits at exhaust 206. Airflow 202 is indicated by arrows and the controlled environmental airflow schematic 214 is provided with at least one heater element 208 and fan element 210. At least one temperature sensor 212 is provided for control of the air temperature and can be correlated with the airflow 202 control.

According to another embodiment of the present invention, a heater assembly for the delivery and precise temperature control of liquids in an automated analytical instrument is provided. The heater assembly provides ambient temperature control of, for example, liquid reagents, liquid buffers, wash liquids, test samples, and the like, in order to maintain high throughput and assay accuracy in an automated analytical instrument. In addition, the heater assembly is capable of providing substantially instantaneous delivery, by force or by gravity, of various liquids into various reaction vessels, cuvettes and the like, within a precisely controlled temperature range. The heater assembly comprises a metal body or block having controllable heating means and internal liquid transfer means to provide heat exchange capabilities for maintaining the temperature of a particular liquid within about ± 1°C of the required temperature of such liquid.

The heater assembly of the present invention is particularly useful with an automated analytical system as described herein which is capable of simultaneously performing two or more assays on a plurality of test samples in a continuous and random access fashion. In particular, the automated immunoassay analytical system apparatus of the invention can be viewed as a microprocessor based system of integrated subassemblies with different groups of assays being run through separate and changeable software modules. The microprocessor based system uses robotic arm pipetters with two degrees of freedom and bidirectional rotating carousels to process samples. Assay steps such as incubations, washes and specimen dilution are performed automatically by the instrument as scheduled, utilizing multiple robotic fluid transfers, as well as reaction vessel transfer and the like, to various workstations. Multiple pipetting actions are performed in order to accomplish high throughput of assays which can be performed on at least two assay procedural systems provided which are scheduled for the various samples.

According to one embodiment of the present invention, a controlled temperature zone or environment in the automated, continuous and random access analytical system as described herein are controlled by various means so that temperature of reagents, wash liquids, buffer solutions, tubing, pipetting and pumping means and the like, within various incubation and reaction zones can be maintained. Such controlled environment zone provides a controlled temperature for optimization of the appropriate analytical reactions being performed by the system. For example, temperature control can be achieved utilizing air flow and air temperature as the thermodynamic working fluid. Although air or gases do not transfer heat as rapidly as a liquid bath, air does provide reasonable ambient air temperature control for major areas of the analytical system instruments. However, since certain assays require precision temperature control, the additional use of a heater assembly of the present invention is particularly useful.

The perspective view of the heater assembly of FIG. 15B illustrates the heater assembly which is constructed of, for example, a metal such as aluminum, silver, copper, or the like, or any other suitable thermoconductive material known in the art, with the various electrical terminals for an electrical resistive heater system, as well as sensing and control elements for controlling precision temperature of the heater assembly for purposes of precision temperature control heat exchange with liquids which flow through the heater assembly and maintained at specific temperatures. The heater assembly 500 comprises a metal body or block 502 having resistance heater electrical post 504 and 506 for providing controlled amounts of energy to the resistance heater element 505 which is shown in FIG. 15C. A thermistor connection 508 provides an electrical resistor whose resistance varies sharply or in a precise manner with temperature and is part of the temperature control feature of the heater assembly 500. The thermistor connection 508 and the thermistor mounted inside the metal block 502 cooperates with thermostatic connections 510 and a back-up thermostatic connection 512 for controlling precisely the temperature of the heater block as well as any liquids contained therein or passing therethrough.

The cross-sectional view of FIG. 15C presents a cross-section through the heater assembly 500 of FIG. 15B and clearly illustrates the resistance heater element 505, as well as liquid inlet 514 and liquid outlet 515. A mounting means 516 is shown as well as a ground pin 520 imbedded in the metal block 502.

A partial cross-sectional view of the heater assembly of FIG. 15B is shown in FIG. 15D, and presents a coiled liquid tubing arrangement within the heater assembly as well as the continuous tubing from the liquid inlet 514 to the liquid outlet 515. The heater assembly 500 can be sized to accommodate increased or decreased liquid volume capacity, as well as heating means for such increased or decreased liquid capacities. The heater assembly 500 is positioned within the automated, continuous and random access analytical systems for precision temperature control of liquids, whether on the process carousel or the MEIA cartridge carousel. The positioning of the heater assembly 500 immediately above the use point avoids significant air gap transfer from the heater assembly 500 to the receiving materials. Temperature controls of the liquids within the heater assembly are controlled from between about ± 1.0°C and about ± 0.5°C of the required liquid temperature. Positioning of the heater assembly 500 in relationship to the receiving means, for example, an MEIA cartridge, allows for about 3/8 inch or less of an air gap transfer from the tip of the liquid outlet 515 to the point of deposition of a liquid onto the cartridge, whereby the liquid is deposited with little or no temperature change thereof.

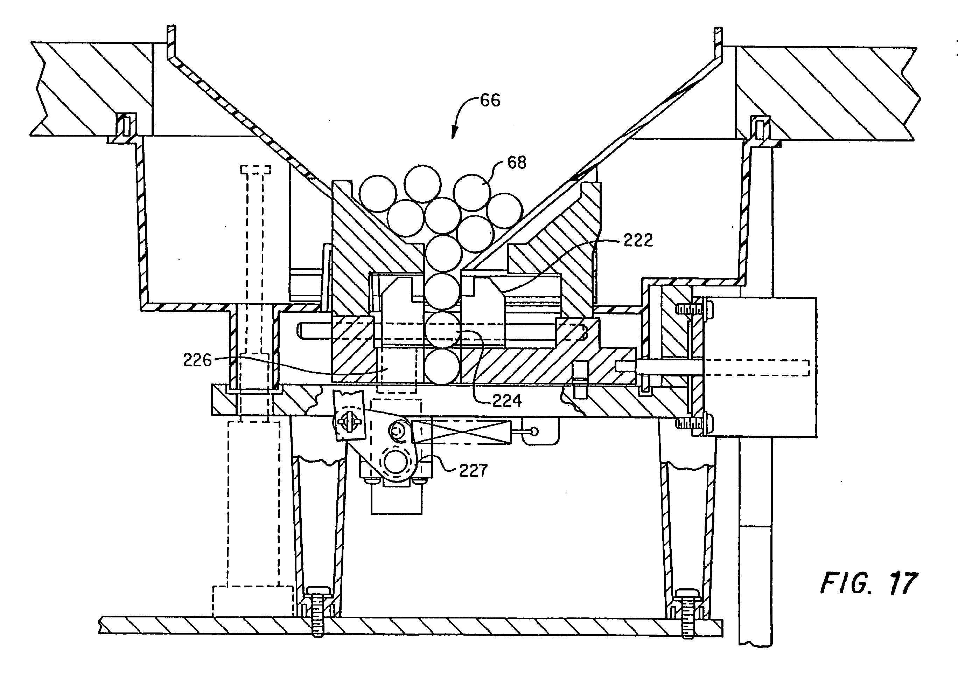

The MEIA cartridge 68 is shown in a side elevational view in FIGURE 16. The MEIA cartridge 68 has a funnel throat 216 and a cartridge opening 218. The MEIA cartridge 68 contains support matrix material 222.

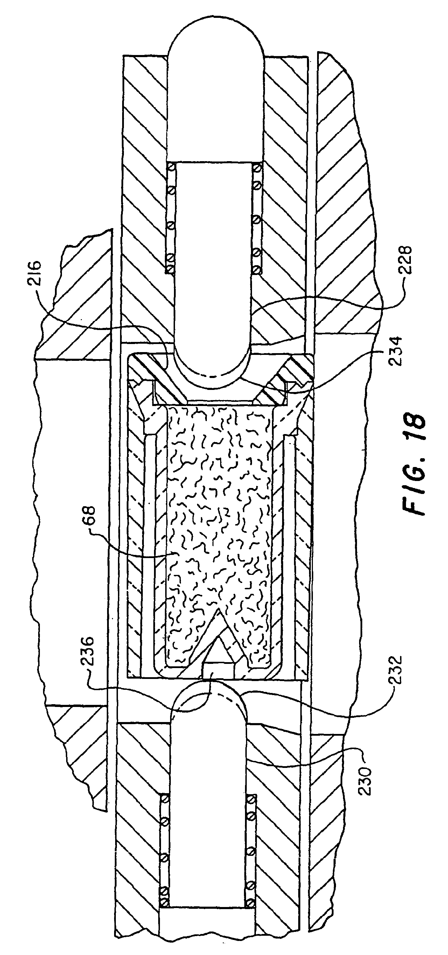

A MEIA cartridge 68 and cartridge hopper 66 are shown in a side elevational view in FIGURE 17. The MEIA cartridges are positioned horizontally in the cartridge hopper 66 and are manipulated from the bottom of the V-shaped cartridge hopper 66 one-by-one through a cartridge shuttle 222. The cartridge feeder has a cartridge cam block 224 and a cartridge orientation shoot 226 which functions through cartridge orientation pin 228 and cartridge orientation pin 230 for providing the MEIA cartridge 68 in vertical alignment for insertion into the auxiliary carousel 64. The orientation pins 228 and 230 are illustrated in FIGURE 18 which is a side sectional view in isolation of the MEIA cartridge feeder cartridge orientation mechanism. The MEIA cartridge 68 is shown in an enlarged view in FIGURE 18 as being engaged and disengaged by cartridge orientation pin 228 and cartridge orientation pin 230. The cartridge orientation pin 230 is shown in engagement position at position 232 against the base 236 of the MEIA cartridge 68 while cartridge orientation pin 228 is shown in engagement position 234 of the cartridge funnel throat portion 216. Upon withdrawal of these pins from the engaging positions, the MEIA cartridge 68 is released from the bottom portion first, i.e. the withdrawal of cartridge orientation pin 230, thus allowing the bottom of a cartridge 68 to drop by gravity before the top of the cartridge is released which is engaged by cartridge orientation pin 228 in the cartridge funnel throat 216. The rounded or semicircular holding surfaces of the orientation pin allow the release of the bottom of the MEIA cartridge and the rolloff of the funnel throat portion 216 from the cartridge orientation pin 228. The vertically aligned MEIA cartridge 68 is then inserted into the auxiliary carousel 64 to a controlled height by the action of an insertion cam means 227 as shown in FIGURE 17.

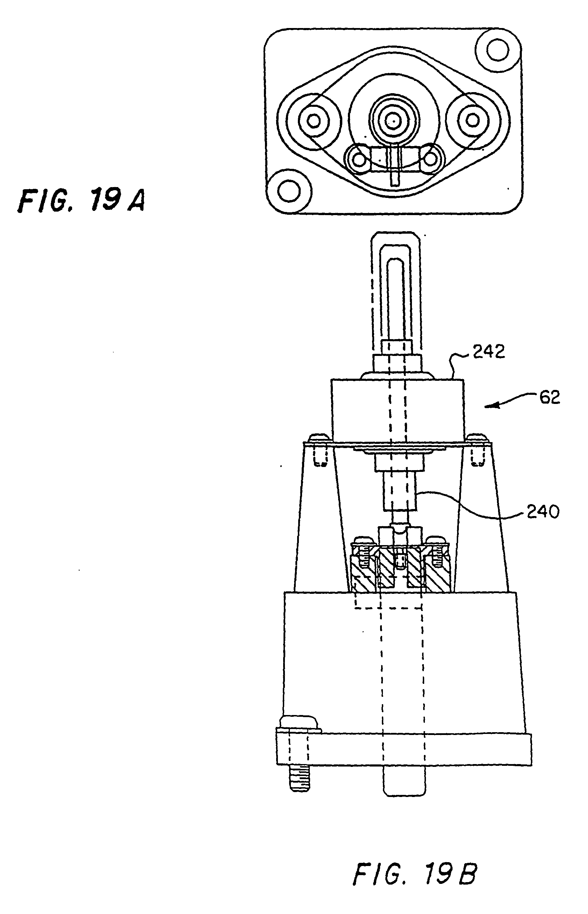

A side view of a MEIA cartridge ejector 62 is illustrated in FIGURE 19. The cartridge ejector 62 functions through an ejector rod 240 and can be driven by manual or automatic drive means 242. The ejected MEIA cartridge is ejected through an ejection passage to the solid waste 198 container.

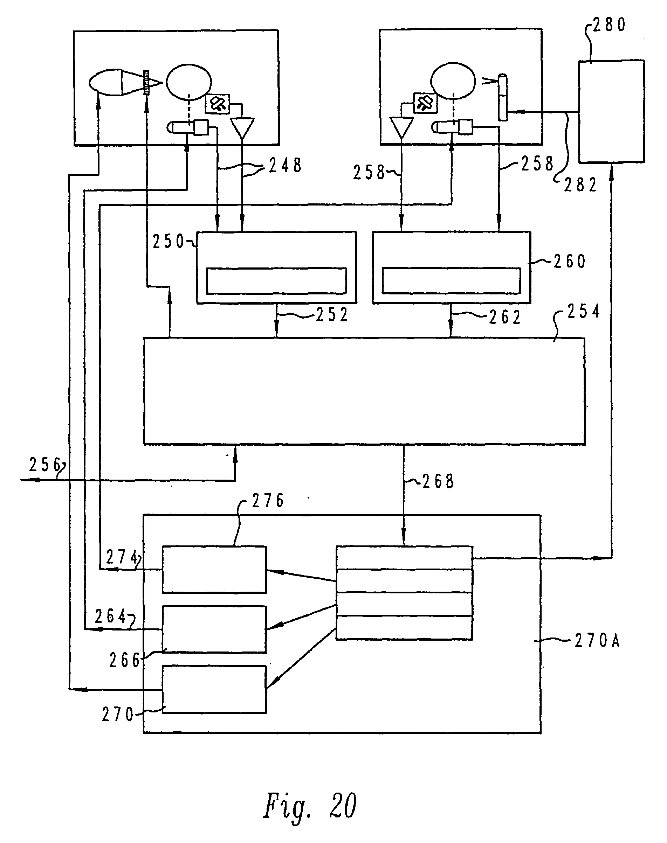

A box diagram of the optics signal processor of the apparatus is provided in FIGURE 20 wherein the signal from the FPIA optics 248 is fed to a DSP A/D 250 which also sends serial bus signal 252 from an optic signal processor 8-bit microcontroller 254. The controller 254 is connected to computer elements through 256. Signal from the MEIA optics 258 are fed into a DSP A/D element 260 which also sends serial bus 262 from the controller 254. Signal is fed to the FPIA optics through 264 from high voltage power supply 266 and serial bus 268 which is in communication between the microcontroller 254 and the optics power supply board 270A. The FPIA tungsten lamp power supply FPIA 270 is in electronic communication with the FPIA optics 272. Signal is sent to the MEIA optics through 274 from high voltage power supply 276 which is in communication through serial bus 268 to the microcontroller 254 and mercury lamp power supply MEIA 280. The MEIA mercury lamp power supply 280 is also in electronic communication with MEIA optics through 282.

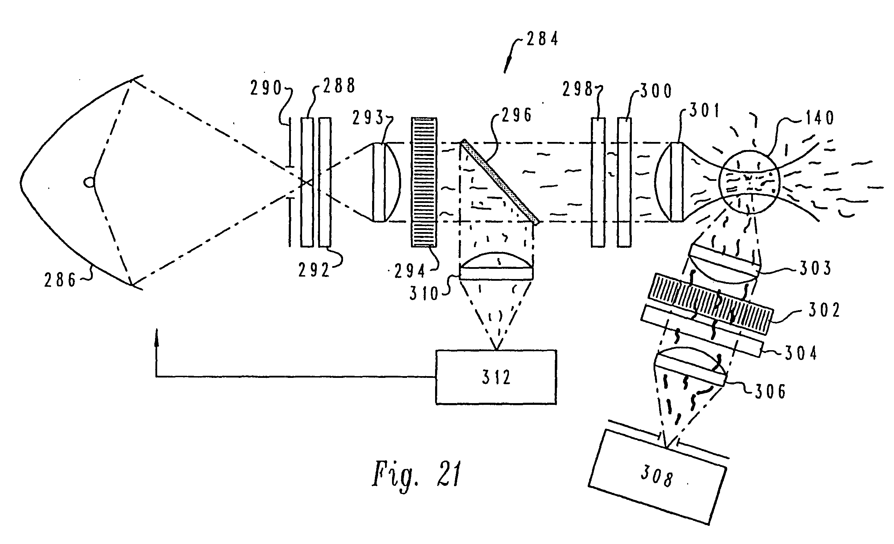

A schematic view of the FPIA optical system 284 is shown in FIGURE 21. The FPIA optical system 284 has a tungsten halogen source lamp 286 which focuses light through a heat reflector 288, an aperture 290 and heat absorber 292 to a lens 293 for introduction into an excitation filter 294. The light energy is then contacted with a beam splitter 296 which presents part of the beam to a polarizer 298 and liquid crystal 300. The light continues into another lens 301 before being focused on the cuvette 140 containing the FPIA reaction mixture. Light is emitted from the cuvette through lens means 303 before entering an emission filter 302. The reflected light from the emission filter 302 passes through a polarizer 304 before going to a focusing lens 306 and being focused for feed into photo multiplier tube 308. The beam splitter 296 splits out part of the light from the original source through lens 310 into a reference detector 312 which, in turn, controls the tungsten halogen source lamp.

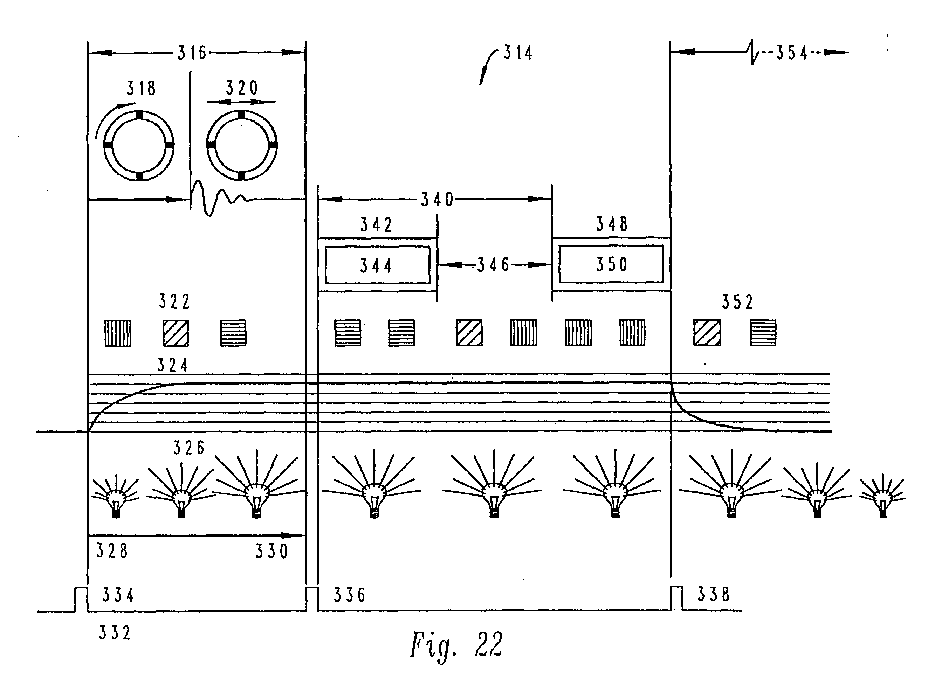

A schematic view of the FPIA read sequence 314 is presented in FIGURE 22. The FPIA read sequence 314 has a preread time 316 divided into carousel move time 318 and carousel settle time 320. Sub-read interval 340 is divided into a horizontal sub-read 342, A/D converter settle time 344, and a liquid crystal activation time 346. A vertical sub-read interval is identified by 348 which is inclusive of A/D converter settle time 350. Liquid crystal relaxation time is indicated by 352. The liquid crystal relaxation time 352 is illustrated in a preread time sequence. High voltage settle time 324 is further illustrated by lamp settle time 326 that shows the lamps in a sinner 328 and full burn 330 activation. Activities of the FPIA read sequence 314 provide for activities where scheduling windows 332 as exemplified by read prep 334, read parameter 336 during which the lamps are at full burn, and collection results 338 during the lamp settlement time and liquid crystal relaxation time 352.

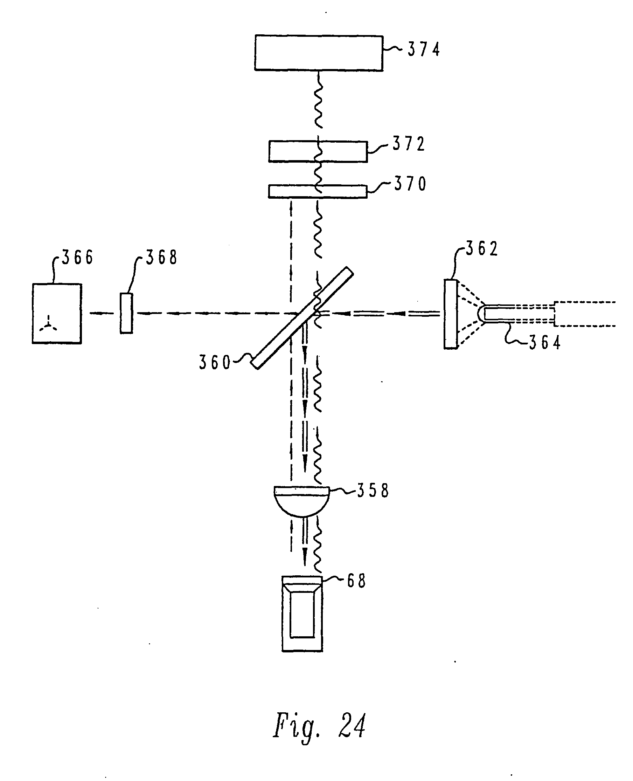

FIGURE 24 is a schematic view of the MEIA system optical assembly 364. An MEIA light source is provided by mercury source lamp 364 which passes light through an excitation filter 362 to a filter reflector 360 before being fed through lens 358 into MEIA cartridge 68. Reflected fluorescent light is fed back through the filter 360 to a photomultiplier tube 374 after passing through a wide band-pass emission filter 370 and narrow band-pass emission filter 372. Part of the light energy from the mercury source lamp 364 passes directly through filter 360 to a bandpass filter 368 before influencing the photo diode 366.

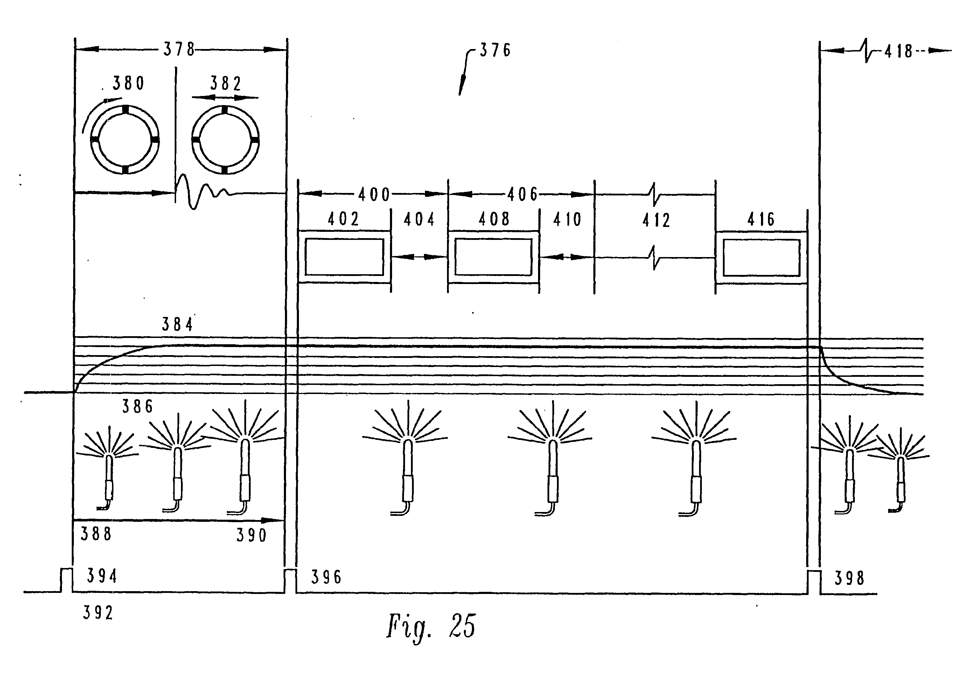

An MEIA read sequence schematic is presented in FIGURE 25 wherein the MEIA read sequence 376 has a preread time 378 inclusive of carousel move time 380 and carousel settle time 382. High voltage settle time is indicated by graph 384 which is coincident with the lamp settlement time 386 showing lamp simmer 388 and lamp full burn 390. MEIA read sequence 376 has activities with scheduling windows 392 inclusive of read prep 394, read parameter 396 and collection results 398. The actual MEIA read sequence 376 is inclusive of sub-read interval 400 having a sub-read 402 and a dwell time 404. Another segment of the MEIA read sequence 376 is indicated by sub-read interval 406 inclusive of sub-read number to 408 and dwell time 410 with additional sub-reads 412 as indicated by number 3 through (N-1) and partial sub-read interval 414 inclusive of sub-read number N-416. The next possible preread time is indicated by 418.

Multiple automated assay analytical systems are feasible through use of the apparatus, software, hardware and process technology of the present invention and include, but are not intended to be limited to, the following menus: ferritin, creatinine kinase MIB (CK-MB), digoxin, phenytoin, phenobarbitol, carbamazepine, vancomycin, valproic acid, quinidine, leutinizing hormone (LH), follicle stimulating hormone (FSH), estradiol, progesterone, IgE, vitamin B2 micro- globulin, glycated hemoglobin (Gly. Hb), cortisol, digitoxin, N-acetylprocainamide (NAPA), procainamide, rubella-lgG, rubella-IgM, toxoplasmosis IgG (Toxo-IgG), toxoplasmosis IgM (Toxo-IgM), testosterone, salicylates, acetaminophen, hepatitis B surface antigen (HBsAg), anti-hepatitis B core antigen IgG IgM (Anti-HBC), human immune deficiency virus 1 and 2 (HIV 1 and 2), human T-cell leukemia virus 1 and 2 (HTLV), hepatitis B envelope antigen (HBeAg), anti-hepatitis B envelope antigen (Anti-HBe), thyroid stimulating hormone (TSH), thyroxine (T4), total triiodothyronine (Total T3), free triiodothyronine (Free T3), carcinoembryoic antigen (CEA), and alpha feta protein (AFP).

A method is also provided for identifying analytical interactions between various steps in a random access analytical system is provided, particularly pipetting sequences whereby the interaction is test sample or reagent carryover or cross contamination. The method of the present invention not only enables the determination of when such interactions are possible, but it also allows for random access processing, that is, allows the software to randomly insert and remove pipetting events from the processing timeline while maintaining control over such carryover or cross-contamination. The method of the present invention allows for processing test sample and reagent with reduced wash volumes since excessive washing only occurs in those instances when carryover or contamination is probable, but not every time such step is performed. Accordingly, the method of the present invention controls carryover or contamination with minimum wash volumes in a random access processor utilizing a simple matrix as described below to relate pipetting steps relative to potential for carryover and contamination and modifies wash volumes between pipetting steps accordingly, and is particularly useful with the automated analytical system described herein which is capable of simultaneously performing two or more assays on a plurality of test samples in a continuous and random access fashion.

In particular, such method is directed to reducing carryover or contamination into simple generic concepts based upon understanding the sources that create the problem. In particular, since each pipette step can result in carryover or contamination, as well as possibly being sensitive to carryover, providing simple categories for the contaminating potential of each pipette step and then identifying which of such categories each assay step is sensitive to, a simple matrix can identify when carryover or contamination is possible. Accordingly, the method of the present invention allows the analytical system to be cleaned to a nominal level, less than the extreme level of the cautious approach previously described. According to the present invention, extra washing can be performed when the software identifies a combination of a potentially contaminating step occurring before a sensitive step, and adds a predetermined super wash that is adequate for controlling the carryover. This approach reduces the amount of washing done in the system because sensitive steps do not necessarily always follow contaminating steps, but because of the nature of random access processing, there is no way to know a *priori* when carryover is or is not possible. The present invention also allows for pipette steps to be removed or inserted into the timeline as necessitated by random access, without danger of creating a contaminating situation. In addition, the present invention allows the software to adjust the required washing without having to manipulate other pipetting steps in the timeline.

The method is designed to minimize wash fluid consumption on the instrument by having the system software track some basic information relating to the pipetting steps that immediately precede and follow any given step on the timeline. Since it involves the interaction of all assays with one another, it is preferred that all assays use the same approach to cleaning the pipette within their protocol. Unlike wash systems and methods previously described, the method according to the present invention (1) reduces wash volumes to help the management of onboard liquid and waste; and (2) reduces washing times to help improve throughput.

In particular, probe wash control in systems previously described was provided by recommendations for post washing after each pipetting block as follows:

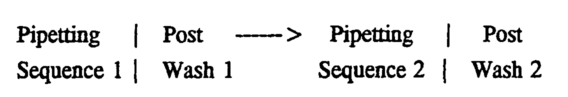

According to the invention, the basic pipette cleaning is provided as before, i.e., with a post wash which should be sufficient to control carryover for most of assay steps that might follow it. However, if the recommended post wash is inadequate for controlling cross-contamination or carryover to the following step, then a prewash is incorporated for that second step as follows:

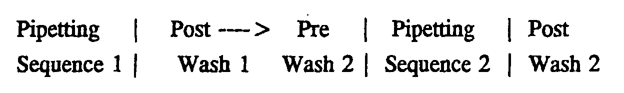

The prewash is variable and has two levels, nominal and super. The nominal prewash is the volume that should be used all the time. When carryover is possible, the super wash would then be used. Typically, the nominal wash volume would be zero. Since the methodology software feature identifies when carryover is possible, the post wash volumes used across the system can be reduced in value from what they were prior to the method, whereby each assay is no longer required to be cleaned well enough to control the worst case carryover situation. Additional wash needed to control carryover will be added through the super wash when the software identifies a carryover potential.

### Parameters, Tables and Terminology

The method preferably utilizes five parameters to describe each pipetting step, two index values and three wash parameters, wherein (i) the two index values are *sus* (susceptibility to contamination) and *con* (probability to contaminate); and (ii) the three wash parameters are *nom* (nominal prewash number), *sup* (super prewash number), and *pw* (post wash number). The wash parameters are not volumes. The wash parameters are numbers that identify washes in a wash library as described below.

| Current Wash Library | | | |
|---|---|---|---|
| Wash Number | Total Volume | Waste | Washcup |
| 0 | 0 ml | - | - |
| 1 | 2 | 1ml | 1ml |
| 2 | 2.5 | 1 | 1.5 |
| 3 | 3 | 1 | 2 |
| 4 | 3.5 | 1.5 | 2 |
| 5 | 4 | 2 | 2 |
| 6 | 4.5 | 2 | 2.5 |
| 7 | 5 | 2 | 3 |
| 8 | 1 | no | yes |
| 9 | 2 | no | yes |
| 10 | 3 | no | yes |
| 11 | 4 | no | yes |
| 12 | 5 | no | yes |

The *sus* and *con* parameters are used to flag the probability for carryover or cross-contamination to occur. They are related to each other through the matrix of the present method.

The matrix of the present method contains only 0's and 1's, corresponding to off and on, respectively; 0 = no probability for carryover; 1 = probability for carryover does exist.

### Method Matrix

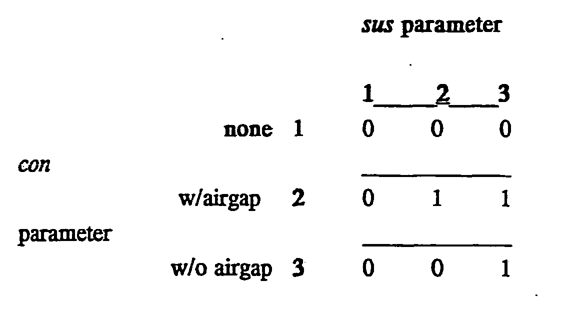

| con | description |
|---|---|
| 1 | not contaminating (no sample) |
| 2 | aspiration of sample or sample mix with airgap |
| 3 | aspiration of sample or sample mix without an airgap |

| *sus* | description |
|---|---|
| 1 | not susceptible to contamination |
| 2 | sensitive to aspiration of sample or sample mix with an airgap |
| 3 | sensitive to aspiration of sample or sample mix without and with an airgap |

For example, a pipette block is susceptible to all sample pipetting *(sus* index = 3). For a preceding pipette step which has a *con* index of 1 (matrix value = 0), no super wash is performed. For a preceding pipette step which has a *con* index of 2 or 3 (matrix value = 1), the super wash is performed.

The matrix of the present method provides information to the software that the probability for carryover or cross-contamination exists, but it does not provide information to the software as to what volumes to use for a wash step, which is instead provided from the *nom, sup* and *pw* parameters. The matrix of the present method may be expanded should other contaminating species in addition to sample be defined.

The *con* parameter and the *pw* numbers describe to the software what state the probe is in prior to the next pipetting step. The rules established for identifying these parameters for pipetting steps are requirements for all assays to follow.

The *con* and *pw* parameters are defined as follows:

| Description | *con* value | *pw* number/vol |
|---|---|---|
| Not contaminating (no sample) | 1 | (2 ml) |
| Asp of sample/sample mix with airgap | 2 | |
| * < = 50 ul aspirated | 1 | (2 ml) |
| * < = 100 ul aspirated | 3 | (3 ml) |
| * < = 150 ul aspirated | 5 | (4 ml) |

| | | |
|---|---|---|
| Aspirating > 150 ul of sample or sample mix with an airgap is discouraged because of the necessity to use excessive washing. Asp of sample/sample mix without an airgap 3, use the same pw values as above. *Indicates the level of sample carryover present when the method of the present invention is not utilized (post wash only) is 10 ppm or less with the above recommendations. In all cases, the minimum allowable *pw* value is 2 ml wash. | | |

The *sus, nom* and *sup* parameters are under the control of the assay protocol. It is to be understood that any criteria established for identifying these parameters are recommendations, and that the assay protocol developer will best know which pipetting sequences are sensitive to carryover, which sequences create the problem and what wash volume is necessary to clean the probe.

Nominal and super washes are used for a susceptible pipette block for control of carryover. Use 0 for Wash Library numbers 8 through 12, where only wash to washcup is needed: *nom* = 0 - no nominal prewash is preformed; *nom =* 8 to 12 - use Wash Library numbers 8 through 12 (1-5 ml wash-washcup); *sup =* 0; no super prewash is performed; *sup* = 8 to 12 ―use Wash Library numbers 8 through 12 (1-5 ml wash-washcup).

Because of scheduling constraints, the super wash volume may not be greater than the minimum post wash (2 ml), plus the nominal wash; if it is necessary to use more super wash volume, the nominal wash should be increased as well. For example, if the nominal wash is 0 ml, super wash may only be 0, 1 or 2 ml. If the required super wash is 4 ml, nominal wash must be at least 2 ml.

The Kitting Center is treated as one pipette block. Carryover experiments have shown that a post wash of at least about 2 ml is sufficient to clean the probe to a carryover level of 1 ppm or less when sample is kitted first followed by wash and pipetting of reagents. Total wash following sample should be about 4 ml total wash before next kitting activity. Contamination of the reagent bottle following sample will come from the outside of the probe. This is reduced to insignificant levels by wash to waste cup, e.g., 200 to 1,000 ul, followed by from between about 1 ml to about 2 ml wash to the wash cup.

In order to ensure consistent, rapid resuspension and continued mixing of reagents with minimal operator involvement, the reagents are mixed automatically each time a new reagent pack is added to the reagent carousel, and periodically during instrument operation. This automated mixing can be accomplished by a back and forth motion of the reagent carousel with asymmetric pauses and is complete within approximately 1-2 minutes. The carousel acceleration, velocity, distance moved, and pause-asymmetry are optimized to yield the most rapid reagent resuspension without foaming or bubble formation for the range of fill volumes used on the instrument.

Automated reagent mixing provides the following benefits. The operator need not manually mix (e.g. by inversion or shaking) reagents which have been stored prior to their placement on the instrument This allows the reagents to be loaded onto the instrument in less time and with less involvement of the operator. There is less tendency for reagents to foam or form bubbles with automatic mixing than with manual mixing such as inversion. Foam and bubble formations are detrimental to instrument function and can negatively impact assay performance. Automated mixing insures that reagents are always mixed sufficiently and that they are mixed consistently. Occasional automatic mixing during instrument operation keeps reagents in a consistent suspension, and makes it unnecessary for the operator to periodically remove reagent packs in order to mix the reagents. In some circumstances, automated mixing can dissipate bubbles present at the start of mixing. A detailed description of kitting and process activities according to the invention are presented in the following for FPIA procedures; system description of process activities for a phenobarbital assay; and MEIA procedures for a CEA assay.

It is to be appreciated that the following description comprises an outline of the various functions and steps involved in preferred methods of the automated analytical system of the invention, which functions and methods as also will be appreciated by those skilled in the art, are conducted under computer control using various types of mathematical algorithms and associated computer software, depending on the particular menu of assays being performed on the instrument.

### DESCRIPTION OF KITTING AND PROCESS AREA ACTIVITIES FOR FPIA

### SYSTEM DESCRIPTION OF KITTING AREA FOR PHENOBARBITAL ASSAY

A. ASSUMPTIONS
   1. Analyzer is in Standby/Ready mode when sample is loaded. System has been previously initialized (All motors are homed, syringe and pumps are purged, all electronics and sensors are checked.)
   2. Waste has been emptied, Diluent, MEIA buffer, MUP, and Quat bulk liquid consumables have been checked for sufficient volume.
   3. All Consumable inventory files have been updated.
B. PREPARATION STEPS
   1. User loads empty Reaction Vessel (RV) into RV carousel.
   2. To load a reagent pack(s), the user must first pause the front end carousels. The system will complete kitting of the current test and transfer the test to the process area.
   3. User opens the reagent carousel cover, loads reagent pack(s) into reagent carousel, closes the reagent carousel cover, then resumes the front-end.
   4. Instrument automatically scans all reagent packs onboard to verify reagent status.
      (a) Each reagent pack is positioned in front of the reagent pack barcode reader by rotation of the reagent carousel.
      (b) Reagent pack barcode reader reads barcode to identify assay type and carousel location.
      (c) If the barcode is unreadable, the system will request a barcode override.
      (d) If the barcode is good or override complete, the system will check the system inventory. The user will be notified if the pack is found to be empty, invalid or outdated. Once the reagent pack is found to be good, it is ready to use.
C. REQUESTING A TEST
   1. User has two options for requesting a test or group of tests on one or more patient samples.
      (a) User may download the test request loadlist from a host computer to create an order list.
      (b) User enters test request or creates an order list on the System directly.
   2. If sample cups (no barcode) are used, the following scenario occurs:
      (a) User refers to order list for segment ID and position number to place sample.
      (b) User loads a sample cup into referenced position in segment.
      (c) User transfers patient sample from blood collection tube into sample cup.
      (d) Segment is placed into sample carousel.
      (e) Indication is made to instrument that samples have been loaded.
      (f) Instrument checks consumable inventories, waste status, cal status, etc.
      (g) Sample carousel rotates segment to segment identification reader.
      (h) Instrument reads segment identification.
   3. If primary tubes (with barcode) are used, the following scenario occurs (two types of carriers are used for primary tubes: one for tubes with heights of 75 mm and a second for tubes with heights of 100 mm.):
      (a) User loads primary tube into next available segment location on sample carousel.
      (b) Indication is made to instrument that samples are available to be run.
      (c) Instrument checks consumable inventories, waste status, cal status, etc.
D. SCHEDULING A TEST
   1. When the sample is presented to the pipettor, the System attempts to schedule the tests ordered on that sample for processing. Each test ordered for the sample will be scheduled separately.
      (b) The System checks for adequate inventory (reagent packs, cartridges, buffer, MUP), system resources, sample time to complete the test.
      (c) The System checks for valid calibration or orders for them on the order list.
      (d) . If all test requirements are met, the test is scheduled for processing.
      (e) If all test requirements are not met, the test request is moved to the exception list. Once the test requirements have been met, the test request is moved back to the order list by the user.
   2. When a test has been scheduled, the System moves it to the processing list and attempts to schedule other tests ordered for that sample.
   3. When all tests for the current sample have been kitted, the System advances to the next sample on the sample carousel.
E. KITTING A TEST
   1. Once a test is scheduled, it is immediately kitted. (No tests are kitted until the scheduler ensures that the test can be transferred onto the process carousel immediately and processed within the timing requirements of the assay.)
   2. RV carousel is rotated clockwise until an RV is detected in pipette axis position.
   3. Reagent pack carousel is rotated until reagent pack for test ordered is at the actuator position. The actuator opens the reagent cartridge caps and the reagent pack carousel is then rotated until a reagent pack for test ordered is in the pipette axis position. After all pipetting steps have been completed, the reagent pack carousel is rotated back to the actuator position where the reagent cartridge caps are closed.
   4. Sample carousel is rotated until sample cup (or primary tube) is in pipette axis position.
   5. Pipette is always at "HOME" position (Pipette R-axis is parked over wash station and Pipette Z-axis is at the Z-clear position) when not in use.
   6. Sample kitting.
      (a) Sample aspirate.
         (i) Syringe aspirates "X" uL of air at a rate of "X" ul/sec.
         (ii) Pipette R-axis is moved over sample cup.
         (iii) Pipette Z-axis is moved down to the Z-above position.
         (iv) LLS is enabled to ensure that no liquid is currently detected.
         (v) Pipette Z-axis is moved down at constant speed until fluid is detected or until Z-Asp limit has been reached (It will be assumed that fluid is detected)
         (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (If insufficient volume is present, the test is aborted and the test request moved to the exception list The exception list provides notice to an operator of tests which cannot be completed).
         (vii) The following occur simultaneously until the total volume of sample required is aspirated:
            (I) Pipette Z-axis motor is moved down at a rate of "X" steps/sec.
            (2) Syringe motor aspirates "X" uL at a rate of "X" ul/sec.
            (3) LLS is checked to ensure probe still in liquid Level Sense (LLS) is disabled. Pipette Z-axis is moved up to Z-clear position.
            (4) Pipette R-axis is moved over the RV sample well.
            (5) Pipette Z-axis is moved down to the dispense position within the RV sample well.
            (6) Syringe dispenses "X" uL of sample at a rate of "X" ul/sec.
            (7) Pipette Z-axis is moved up to Z-clear position.
      (b) Probe Post-Wash
         The probe is washed to ensure that it is free from contamination. It is to be understood that all pipette activities (in both kitting and process areas) are followed with a probe post-wash to minimize carryover from one fluid aspirate to another. In some cases, pipette activities may be preceded with a probe prewash if necessary to guarantee the validity of the next fluid aspirate. For this assay description, it will be assumed that only a post-wash is used.
         (i) The inside of the probe is cleaned first.
            (1) Pipette R-axis is moved over waste area.
            (2) Pipette Z-axis is moved down to appropriate position within the waste area.
            (3) The wash valve is opened for the amount of time specified in the assay protocol.
            (4) Wash valve is closed.
            (5) Pipette Z-axis is moved up to the Z-clear position.
         (ii) The outside of the probe is cleaned next.
            (1) Pipette R-axis is moved over wash cup.
            (2) Pipette Z-axis is moved down to wash position within the wash cup.
            (3) The wash valve is opened for the amount of time specified in the assay protocol.
            (4) Wash valve is closed.
         (iii) Pipette is returned to "HOME" position.
   7. Popper kitting ("Popper" is defined as a substance which eliminates in general interfering substances in assays such as, for example, those discussed and claimed in U.S. Patent 4,492,762 issued January 8, 1985 and hereby incorporated by reference.)
      (a) Popper aspirate.
         (i) Syringe aspirates "X" uL of air at a rate of "X" ul/sec.
         (ii) Pipette R-Axis is moved over the popper reagent bottle in the Reagent Pack.
         (iii) Pipette Z-axis is moved down to the Z-above position.
         (iv) LLS is enabled to ensure no liquid currently detected.
         (v) Pipette Z-axis is moved down at constant speed until fluid is detected or until the Z-aspiration-lower (Z-Asp) limit is reached (it will be assumed that fluid is detected).
         (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
         (vii) The following occur simultaneously until the total volume of popper required is aspirated:
            (1) Pipette Z-axis motor is moved down at a rate of "X" steps/sec.
            (2) Syringe aspirates "X" uL at a rate of "X" ul/sec.
            (3) LLS is checked to ensure probe still in liquid.
            (4) LLS is disabled.
            (5) Pipette Z-axis is moved up to Z-clear position.
            (6) Pipette R-axis is moved over the RV reagent 1 well.
            (7) Pipette Z-axis is moved down to the dispense position within the RV reagent 1 well.
            (8) Syringe dispenses "X" uL of popper at a rate of "X" ul/sec.
            (9) Pipette Z-axis is moved up to Z-clear position.
      (b) Probe post-wash.
         The probe is again washed to ensure that it is free from contamination as described in section 6 (Sample Kitting).
   8. Antiserum kitting
      (a) Antiserum aspirate
         (i) Syringe aspirates "X" uL of air at a rate of "X" ul/sec.
         (ii) Pipette R-Axis is moved over the antiserum reagent bottle in the Reagent Pack.
         (iii) Pipette Z-axis is moved down to the Z-above position.
         (iv) LLS is enabled to ensure no liquid currently detected.
         (v) Pipette Z-axis is moved down at constant speed until fluid is detected or until the Z-Asp limit is reached (it will be assumed that fluid is detected).
         (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
         (vii) The following occur simultaneously until the total volume of antiserum required is aspirated:
            (1) Pipette Z-axis motor is moved down at a rate of "X" steps/sec.
            (2) Syringe aspirates "X" micro liter (uL) at a rate of "X" ul/sec. LLS is checked to ensure probe still in liquid.
            (3) LLS is disabled.
            (4) Pipette Z-axis is moved up to Z-clear position.
            (5) Pipette R-axis is moved over the RV reagent 2 well.
            (6) Pipette Z-axis is moved down to the dispense position within the RV reagent 2 well.
            (7) Syringe dispenses "X" uL of antiserum at a rate of "X" ul/sec.
            (8) Pipette Z-axis is moved up to Z-clear position.
      (b) Probe post-wash.
         The probe is again washed to ensure that it is free from contamination as described in section 6 (Sample Kitting).
   9. Tracer kitting.
      (a) Tracer aspirate.
         (i) Syringe aspirates "X" uL of air at a rate of "X" ul/sec.
         (ii) Pipette R-Axis is moved over the tracer reagent bottle in the Reagent Pack.
         (iii) Pipette Z-axis is moved down to the Z-above position.
         (iv) LLS is enabled to ensure no liquid currently detected.
         (v) Pipette Z-axis is moved down at constant speed until fluid is detected or until the Z-Asp limit is reached (it will be assumed that fluid is detected).
         (vi) Based on the Z-height position at which fluid is detected and the Z-height/volume table, the System calculates the volume of fluid in the well and compares it to the volume specified in the pipetting description. If sufficient volume is present in the well, the aspiration sequence is initiated. (if sufficient volume not is present, the test is aborted and the test request moved to the exception list).
         (vii) The following occur simultaneously until the total volume of tracer required is aspirated:
            (1) Pipette Z-axis motor is moved down at a rate of "X" steps/sec.
            (2) Syringe aspirates "X" uL at a rate of "X" ul/sec.
            (3) LLS is checked to ensure probe still in liquid.
            (4) LLS is disabled.
            (5) Pipette Z-axis is moved up to Z-clear position.
            (6) Pipette R-axis is moved over the RV reagent 3 well.
            (7) Pipette Z-axis is moved down to the dispense position within the RV reagent 2 well.
            (8) Syringe dispenses "X" uL of tracer at a rate of "X" ul/sec.
            (9) Pipette Z-axis is moved up to Z-clear position.
      (b) Probe post-wash.
         The probe is again washed to ensure that it is free from contamination as described in section 6 (Sample Kitting).
F. TRANSFER OF REACTION VESSEL (RV) INTO PROCESS AREA
   1. RV carousel is rotated to transfer station.
   2. Process carousel is rotated so that the empty position is aligned with the transfer station.
   3. Transfer mechanism 0-axis is rotated to sample entry area.
   4. Transfer mechanism R-axis grabs the RV and pulls it into the transfer mechanism.
   5. Transfer mechanism 0-axis is rotated so that RV is aligned with the empty position on the process carousel.
   6. RV is loaded onto process carousel.

### SYSTEM DESCRIPTION OF FPIA PROCESS AREA FOR PHENOBARBITAL

A. Wait for temperature equilibration time and evaporation window to expire.
B. FIRST PIPETTE ACTIVITY (preparation of sample blank comprising diluted sample and popper).
   1. Incubation timer is set according to assay file specifications.
   2. Precision diluent aspirate. The following activities are performed simultaneously:
      (a) Syringe aspirates "X" uL at a rate of "X" ul/sec.
      (b) Wash valve is opened.
      (c) Wait "n" seconds.
      (d) Wash valve is closed.
   3. Sample aspirate.
      (a) Pipette R-axis is moved over the RV sample well.
      (b) LLS is enabled to ensure no liquid currently detected.
      (c) Pipette Z-axis is moved down at constant speed until fluid is detected OR until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (e) The following occur simultaneously until the total volume of sample required is aspirated:
         (i) Pipettor Z-axis motor is moved down at a rate of "X" steps/sec.
         (ii) Syringe aspirates "x" uL of sample at a rate of "X" ul/sec.
         (iii) LLS is checked to ensure probe still in liquid.
         (iv) LLS is disabled.
         (v) Pipette Z-axis is moved up to Z-above position.
   4. Diluent/sample dispensed to the RV predilute well.
      (a) Pipette R-axis is moved over the RV predilute well.
      (b) Pipette Z-axis is moved down to the dispense position within the RV predilute well.
      (c) Syringe dispenses "X" uL of diluent/sample at a rate of "X" ul/sec.
      (d) Pipette Z-axis is moved up to Z-clear position.
   5. Probe post-wash.
      The probe is again washed to ensure that it is free from contamination as described in section 6 (Sample kitting).
   6. Precision diluent aspirate. The following activities are performed simultaneously:
      (a) Syringe aspirates "X" uL at a rate of "X" ul/sec.
      (b) Wash valve is opened.
      (c) Wait "n" seconds.
      (d) Wash valve is closed.
   7. Popper aspirate.
      (a) Pipette R-axis is moved over the RV Reagent (popper) well.
      (b) LLS is enabled to ensure no liquid currently detected.
      (c) Pipette Z-axis is moved down at constant speed until fluid is detected or until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (e) The following occur simultaneously until the total volume of popper required is aspirated:
         (i) Pipette Z-axis motor is moved down at a rate of "X" steps/sec.
         (ii) Syringe aspirates "X" uL at a rate of "x" ul/sec.
         (iii) LLS is checked to ensure probe still in liquid.
         (iv) LLS is disabled.
         (v) Pipette Z-axis is moved up to the Z-above position.
   8. Diluted sample aspirate.
      (a) Pipette R-axis is moved over the RV predilute well.
      (b) LLS is enabled to ensure no liquid currently detected.
      (c) Pipette Z-axis is moved down at constant speed until fluid is detected or until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (e) The following occur simultaneously until the total volume of diluted sample required is aspirated:
         (i) Pipette Z-axis motor is moved down at a rate of "X" steps/sec.
         (ii) Syringe aspirates "X" uL at a rate of "x" ul/sec.
         (iii) LLS is checked to ensure probe still in liquid.
         (iv) LLS is disabled.
         (v) Pipette Z-axis is moved up to the Z-above position.
   11. Diluted sample/popper diluent dispensed to RV cuvette.
      (a) Pipette R-axis is moved over to the RV cuvette position.
      (b) Pipette Z-axis is moved down to the dispense position in the RV cuvette.
      (c) Syringe dispenses "X" uL of diluted sample/popper/diluent at a rate of "X" uL/sec.
      (d) Pipette Z-axis is moved up to the Z-above position.
   12. Probe post-wash.
      The probe is again washed to ensure that it is free from contamination as described in section 6 (sample kitting) to complete first pipette activity.
C. BLANK READ PREPARATION
   When incubation timer expired, the following activities are started:
   1. The FPIA reader is prepared to take a read; lamp intensity is brought from simmer state to burn state.
   2. Photomultiplier tube (PMT) gain is set.
D. BLANK READ (BACKGROUND)
   1. Incubation timer is set according to assay file specifications.
   2. Process carousel is rotated so that the RV is at the read station.
   3. Horizontal intensity is read for "X.XX" seconds.
   4. The crystal is flipped for the vertical read.
   5. Wait "n" seconds until the crystal settles.
   6. Vertical intensity is read for "X.XX" seconds.
   7. The raw reads are converted to normalized reads (light intensity hitting detector/lamp intensity) by the optics microprocessor.
   8. Background reads are stored.
   9. System calculates BLANK I to complete blank read.
   10. Next activity started when incubation timer expires.
E. SECOND PIPETTE ACTIVITY (for reaction between diluted sample, popper, tracer and antiserum).
   1. Incubation timer is set according to assay file specifications.
   2. Precision diluent aspirate.
      (a) The following activities are performed simultaneously:
         (i) Syringe aspirates "X" uL at a rate of "X" ul/sec.
         (ii) Wash valve is opened.
         (iii) Wait "n" seconds.
         (iv) Wash valve is closed.
   3. Antiserum aspirate.
      (i) Pipette R-axis is moved over the RV Reagent 2 (antiserum) well.
      (ii) LS is enabled to ensure no liquid currently detected.
      (iii) Pipette Z-axis is moved down at constant speed until fluid is detected OR until the Z-Asp limit is reached (it will be assumed that fluid is detected).
      (iv) Based on the Z-height position at which fluid is detected and the Z-height/volume table, the System calculates the volume of fluid in the well and compares it to the volume specified in the pipetting description. If sufficient volume is present, the aspiration sequence is initiated. (If sufficient volume is not present, the test is aborted and the test request moved to the exception list.)
      (v) The following occur simultaneously until the total volume of antiserum required is aspirated:
         (1) Pipette Z-axis motor is moved down at a rate of "X" steps/sec.
         (2) Syringe aspirates "X" uL at a rate of "X" ul/sec.
         (3) LLS is checked to ensure probe still in liquid.
         (4) LLS is disabled.
         (5) Pipette Z-axis is moved up to the Z-above position.
   4. Tracer aspirate.
      (a) Syringe aspirates "X" uL of air at a rate of "X" ul/sec.
      (b) Pipette R-axis is moved over the RV Reagent 3 (tracer) well.
      (c) LLS is enabled to ensure no liquid currently detected.
      (d) Pipette Z-axis is moved down at constant speed until fluid is detected OR until the Z-Asp limit is reached (it will be assumed that fluid is detected).
      (e) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (f) The following occur simultaneously until the total volume of tracer required is aspirated:
         (i) Pipette Z-axis motor is moved down at a rate of "X" steps/sec.
         (ii) Syringe aspirates "X" uL at a rate of "X" ul/sec.
         (iii) LLS is checked to ensure probe still in liquid.
         (v) LLS is disabled.
         (vi) Pipette Z-axis is moved up to the Z-above position.
   5. Diluted sample aspirate.
      (a) Pipette R-axis is moved over the RV predilute well.
      (b) LLS is enabled to ensure no liquid currently detected.
      (c) Pipette Z-axis is moved down at constant speed until fluid is detected OR until the Z-Asp limit is reached (it will be assumed that fluid is detected).
      (d) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list)
      (e) The following occur simultaneously until the total volume of diluted sample required is aspirated:
         (1) Pipette Z-axis motor is moved down at a rate of "X" steps/sec.
         (2) Syringe aspirates "X" uL at a rate of "X" ul/sec.
         (3) LLS is checked to ensure probe still in liquid.
         (4) LLS is disabled.
         (5) Pipette Z-axis is moved up to the Z-above position.
   6. Diluted sample/tracer/aspirate/antiserum/diluent dispensed to RV cuvette.
      (a) Pipette R-axis is moved over to the RV cuvette position.
      (b) Pipette Z-axis is moved down to the dispense position in the RV cuvette.
      (c) Syringe dispenses "X" uL of diluted sample/tracer/air/antiserum/diluent at a rate of "X" ul/sec.
      (d) Pipette Z-axis is moved up to the Z-above position.
   7. Probe post-wash.
      The probe is again washed to ensure that it is free from contamination as described in section 6 (Sample kitting) to complete the second pipette activity.
   8. Next activity started when incubation timer expires.
E. FINAL READ PREPARATION
   1. The FPIA reader is prepared to take a read; lamp intensity is brought from simmer state to burn state.
   2. PMT gain is set.
F. FINAL READ
   1. Process carousel is rotated so that the RV is at the read station.
   2. Horizontal intensity is read for "X.XX" seconds.
   3. The crystal is flipped for the vertical read.
   4. The System delays "n" seconds until the crystal settles.
   5. Vertical intensity is read for "X.XX" seconds.
   6. The raw reads are converted to normalized reads (light intensity hitting detector/lamp intensity) by the optics microprocessor.
   7. Reads are stored.
   8. System calculates NET intensity (I) and milipolarization (mP).
   9. mP value is fitted to calibration curve to yield a concentration result.
G. RV UNLOAD (this activity occurs when resources are not in use. The following are performed simultaneously:
   1. Process carousel is rotated so that the empty position is at the transfer station. Transfer mechanism 0-axis is moved to process carousel.
   2. RV is grabbed with the transfer mechanism R-axis and pulled into the transfer mechanism.
   3. Transfer mechanism 0-axis is rotated so that RV is aligned with the waste container.
   4. RV is pushed into the waste container.

### DESCRIPTION OF KITTING AND PROCESS AREA ACTIVITIES FOR MEIA

### SYSTEM DESCRIPTION OF KITTING AREA FOR CEA ASSAY

A. ASSUMPTIONS
   1. Analyzer is in Standby/Ready mode when sample is loaded. System has been previously initialized (All motors are homed, syringe and pumps are purged, all electronics and sensors are checked).
   2. Waste has been emptied, dilution, MEIA buffer, MUP, and Quat bulk liquid consumables have been checked for sufficient volume.
   3. Cartridges have been placed into hopper and are available for loading onto auxiliary carousel when needed (for MEIA assays only).
   4. All Consumable inventory files have been updated.
B. PREPARATION STEPS
   1. User loads empty RVs into RV carousel.
   2 To load a reagent pack(s), the user must first pause the front end carousels. The system will complete kitting of the current test and transfer the test to the process area.
   3. User opens the reagent carousel, loads reagent pack(s) into reagent carousel, closes the reagent carousel cover, then resumes the front-end.
   4. Instrument automatically scans all reagent packs onboard to verify reagent status.
   5. Each reagent pack is positioned in front of the reagent pack barcode reader by rotation of the reagent carousel.
   6. Reagent pack barcode reader reads barcode to identify assay type and carousel location. If the barcode is unreadable, the system will request a barcode override.
   7. If the barcode is good or override complete, the system will check the system inventory. The user will be notified if the pack is found to be empty, invalid or outdated. Once the reagent pack is found to be good, it is ready to use.
C. REQUESTING A TEST
   1. User has two options for requesting a test or group of tests on one or more patient samples.
      (a) User may download the test request loadlist from a host computer to create an order list.
      (b) User enters test request or creates an order list on the System directly.
   2. If sample cups (no barcode) are used, the following scenario occurs:
      (a) User refers to order list for segment ID and position number to place sample.
      (b) User loads a sample cup into referenced position in segment.
      (c) User transfers patient sample from blood collection tube into sample cup.
      (d) Segment is placed into sample carousel.
      (e) Indication is made to instrument that samples have been loaded.
      (f) Instrument checks consumable inventories, waste status, assay calibration, etc.
      (g) Sample carousel rotates segment to segment identification reader.
      (h) Instrument reads segment identification.
   3. If primary tubes (with barcode) are used, the following scenario occurs:
      (a) User loads primary tube into next available segment location on sample carousel (two types of carriers are used for primary tubes: one for tubes with heights of 75 mm and a second for tubes with heights of 100 mm.).
      (b) Indication is made to instrument that samples are available to be run.
      (c) Sample carousel rotates segment to segment identification reader.
D. SCHEDULING A TEST
   1. When the sample is presented to the pipettor, the System attempts to schedule the tests ordered on that sample for processing. Each test ordered for the sample will be scheduled separately.
      (a) The System checks for adequate inventory (reagent packs, cartridges, buffer, MUP), system resources, sample time to complete the test.
      (b) The System checks for valid calibration or orders for them on the order list.
      (c) If all test requirements are met, the test is scheduled for processing.
      (d) If all test requirements are not met, the test request is moved to the exception list. Once the test requirements have been met, the test request is moved back to the order list by the user.
   2. When a test has been scheduled, the system moves it to the processing list and attempts to schedule other tests ordered for that sample.
   3. When all tests for the current sample have been kitted, the System advances to the next sample on the sample carousel.
E. KITTING A TEST
   1. Once a test is scheduled, it is immediately kitted (no tests are kitted until the scheduler ensures that the test can be transferred onto the process carousel immediately and processed within the timing requirements of the assay).
   2. RV carousel is rotated clockwise until an RV is detected in pipette axis position.
   3. Reagent pack carousel is rotated until reagent pack for test ordered is at the actuator position. The actuator opens the reagent cartridge caps and the reagent pack carousel is then rotated until reagent pack for test ordered is in the pipette axis position. After all pipetting steps have been completed, the reagent pack carousel is rotated back to the actuator position where the reagent cartridge caps are closed.
   4. Sample carousel is rotated until sample cup (or primary tube) is in pipette axis position.
   5. Pipette is always at HOME position (Pipette R-axis is parked over wash station and Pipette Z-axis is at the Z-clear position) when not in use.
   6. Sample kitting.
      (a) Sample aspirate.
         (i) Syringe aspirates "X" uL of air at a rate of "X" ul/sec.
         (ii) Pipette R-axis is moved over sample cup.
         (iii) Pipette Z-axis is moved down to the Z-above position.
         (iv) Pipette Z-axis is moved down to the Z-LLS position.
         (v) LLS is enabled to ensure that no liquid is currently detected.
         (vi) Pipette Z-axis is moved down at constant speed until fluid is detected or until Z-Asp limit has been reached (it will be assumed that fluid is detected).
         (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
         (viii) The following occur simultaneously until the total volume of sample required is aspirated:
            (1) Pipette Z-axis motor is moved down at a rate of "X" steps/sec.
            (2) Syringe aspirates "X" uL at a rate of "X" ul/sec.
            (3) LLS is checked to ensure probe still in liquid.
            (4) LLS is disabled.
            (5) Pipette Z-axis is moved up to Z-clear position.
            (6) Pipette R-axis is moved over the RV sample well.
            (7) Pipette Z-axis is moved down to the dispense position within the RV sample well.
            (8) Syringe dispenses "X" uL of sample at a rate of "X" ul/sec.
            (9) Pipette Z-axis is moved up to Z-clear position.
      (b) Probe post-wash.
         The probe is washed to ensure that it is free from contamination. It is to be understood that pipette activities in both kitting and process areas are generally followed with a probe post-wash to minimize carryover from one fluid aspirate to another. In some cases, pipette activities may be preceded with a probe prewash if necessary to guarantee the validity of the next fluid aspirate. For this assay description, it will be assumed that only a post-wash is used.
         (i) The inside of the probe is cleaned first.
            (1) Pipette R-axis is moved over waste area.
            (2) Pipette Z-axis is moved down to appropriate position within the waste area.
            (3) The wash valve is opened for the amount of time specified in the assay protocol.
            (4) Wash valve is closed.
         (ii) Pipette Z-axis is moved up to the Z-clear position.
         (iii) The outside of the probe is cleaned next.
            (1) Pipette R-axis is moved over wash cup.
            (2) Pipette Z-axis is moved down to wash position within the wash cup.
            (3) The wash valve is opened for the amount of time specified in the assay protocol.
            (4) Wash valve is closed.
            (5) Pipette is returned to "HOME" position.
   7. Microparticle kitting.
      (a) Microparticle aspirate (microparticles are pipetted directly into the RV incubation well to save on volume, as this is the most costly MEIA reagent).
         (i) Syringe aspirates "X" uL of air at a rate of "X" ul/sec.
         (ii) Pipette R-Axis is moved over the microparticle reagent bottle in the Reagent Pack.
         (iii) Pipette Z-axis is moved down to the Z-above position.
         (iv) Pipette Z-axis is moved down to the Z-LLS position.
         (v) LLS is enabled to ensure no-liquid currently detected.
         (vi) Pipette Z-axis is moved down at constant speed until fluid is detected or until the Z-Asp limit is reached (it will be assumed that fluid is detected)
         (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
         (viii) The following occur simultaneously until the total volume of microparticles required is aspirated:
            (1) Pipette Z-axis motor is moved down at a rate of "X" steps/sec.
            (2) Syringe aspirates "X" uL at a rate of "X" ul/sec.
            (3) LLS is checked to ensure probe still in liquid.
         (ix) LLS is disabled.
         (x) Pipette Z-axis is moved up to Z-clear position.
         (xi) Pipette R-axis is moved over the RV incubation well.
         (xii) Pipette Z-axis is moved down to the dispense position within the RV incubation well.
         (xiii) Syringe dispenses "X" uL of microparticles at a rate of "X" ul/sec. Pipette Z-axis is moved up to Z-clear position.
      (b) Probe post-wash.
         The probe is again washed to ensure that it is free from contamination as described in section 6 (Sample kitting).
   8. Conjugate kitting.
      (a) Conjugate aspirate (conjugate, special wash fluid, and/or specimen diluent are pipetted into either RV reagent wells or RV predilution well, depending on volume requirements).
         (i) Syringe aspirates "X": uL of air at a rate of "X" ul/sec.
         (ii) Pipette R-Axis is moved over the conjugate reagent bottle in the Reagent Pack.
         (iii) Pipette Z-axis is moved down to the Z-above position.
         (iv) Pipette Z-axis is moved down to the Z-LLS position.
         (v) LLS is enabled to ensure no liquid currently detected.
         (vi) Pipette Z-axis is moved down at constant speed until fluid is detected or until the Z-Asp limit is reached (it will be assumed that fluid is detected.
         (vii) Based on the Z-height position at which fluid is detected and the Z-height/volume table, the System calculates the volume of fluid in the well and compares it to the volume specified in the pipetting description. If sufficient volume is present in the well, the aspiration sequence is initiated (if sufficient volume is not present, the test is aborted and the test request moved to the exception list).
         (viii) The following occur simultaneously until the total volume of conjugate required is aspirated:
            (1) Pipette Z-axis motor is moved down at a rate of "x" steps/sec.
            (2 ) Syringe aspirates "X" uL at a rate of "X" ul/sec.
            (3) LLS is checked to ensure probe still in liquid.
         (ix) LLS is disabled.
         (x) Pipette Z-axis is moved up to Z-clear position.
         (xi) Pipette R-axis is moved over the RV reagent well.
         (xii) Pipette Z-axis is moved down to the dispense position within the RV r reagent well.
         (xiii) Syringe dispenses "X" uL of conjugate at a rate of "X" ul/sec.
         (xiv) Pipette Z-axis is moved up to Z-clear position.
      (b) Probe post-wash.
         The probe is again washed to ensure that it is free from contamination as described in section 6 (Sample kitting).
   9. MEIA Buffer Kitting.
      (a) RV Carousel is rotated until RV buffer well is under the MEIA buffer dispenser at buffer kitting station.
      (b) "X" uL of MEIA buffer is dispensed into the buffer well at a rate of "X" ul/sec
F. TRANSFERRING RV INTO PROCESS AREA
   1. RV carousel is rotated to transfer station.
   2. Process carousel is rotated so that the empty position is aligned with the transfer station.
   3. Transfer mechanism 0-axis is rotated to sample entry area.
   4. Transfer mechanism R-axis grabs the RV and pulls it into the transfer mechanism.
   5. Transfer mechanism 0-axis is rotated so that RV is aligned with the empty position on the process carousel.
   6. RV is loaded onto process carousel.

### SYSTEM DESCRIPTION OF MEIA PROCESS AREA FOR CEA

A. System waits for temperature equilibration time and evaporation window to expire.
B. FIRST PIPETTE ACTIVITY (microparticle/sample reaction)
   1. Incubation timer is set according to assay file specifications.
   2. MEIA buffer aspirate.
      (a) The process carousel is moved so that the RV is at the pipetting station.
      (b) Syringe aspirates "X" uL of air at a rate of "X" ul/sec.
      (c) Pipette R-axis is moved over the RV buffer well.
      (d) Pipette Z-axis is moved down to the Z-above position over the RV buffer well.
      (e) Pipette Z-axis is moved down to the Z-LLS position.
      (f) LLS is enabled to ensure no liquid currently detected.
      (g) Pipette Z-axis is moved down at constant speed until fluid is detected or until the Z-Asp limit is reached (it will be assumed that fluid is detected).
      (h) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (i) The following occur simultaneously until the total volume of MEIA buffer required is aspirated:
         (1) Pipette Z-axis motor is moved down at a rate of "X" steps/sec.
         (2) Syringe aspirates "X" uL at a rate of "X" ul/sec.
      (j) LLS is checked to ensure probe still in liquid.
      (k) LLS is disabled.
      (l) Pipette Z-axis is moved up to Z-above position.
   3. Sample aspirate
      (a) Pipette R-axis is moved over the RV sample well.
      (b) Pipette Z-axis is moved down to the Z-LLS position.
      (c) LLS is enabled to ensure no liquid currently detected.
      (d) Pipette Z-axis is moved down at constant speed until fluid is detected or until the Z-Asp limit is reached (it will be assumed that fluid is detected).
      (e) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
      (f) The following occur simultaneously until the total volume of sample required is aspirated:
         (1) Pipettor Z-axis motor is moved down at a rate of "X" steps/sec.
         (2) Syringe aspirates "X" uL at a rate of "X" ul/sec.
      (g) LLS is checked to ensure probe still in liquid.
      (h) LLS is disabled.
      (i) Pipette Z-axis is moved up to Z-above position.
   4. MEIA buffer and sample are added to microparticles in incubation well.
      (a) Pipette Z-axis is moved down to the dispense position within the RV incubation well.
      (b) Syringe dispenses "X" uL of MEIA buffer and sample at a rate of "X" ul/sec.
      (c) Pipette Z-axis is moved up to Z-clear position.
   5. Probe post-wash.
      The probe is again washed to ensure that it is free from contamination as described in section 6 (Sample kitting).
C. CARTRIDGE LOAD (This activity occurs when resources are not in use)
   1. Move the auxiliary carousel so that reserved position is under feeder.
   2. Cycle trap-door mechanism to load flashlight into carousel.
   3. Cycle shuttle mechanism to place another MEIA cartridge on trap door (for next tab load).
   4. Check incubation timer. When expires start next pipetting.
D. SECOND PIPETTE ACTIVITY (transfer of reaction mixture to matrix)
   1. Incubation timer is set according to assay file specifications.
   2. Buffer aspirate.
      (a) The process carousel is moved so that the RV is at the pipetting station.
      (b) Syringe aspirates "X" uL of air at a rate of "X" ul/sec.
      (c) Pipette R-axis is moved over the RV buffer well.
      (d) Pipette Z-axis is moved down to the Z-above position.
      (e) Pipette Z-axis is moved down to the Z-LLS position.
      (f) LLS is enabled to ensure no liquid currently detected.
      (g) Pipette Z-axis is moved down at constant speed until fluid is detected or until the Z-Asp limit is reached (it will be assumed that fluid is detected).
      (h) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
      (i) The following occur simultaneously until the total volume of buffer required is aspirated:
         (1) Pipette Z-axis motor is moved down at a rate of "X" steps/sec.
         (2) Syringe aspirates "X" uL at a rate of "X" ul/sec.
      (j) LLS is checked to ensure probe still in liquid.
      (k) LLS is disabled.
      (l) Pipette Z-axis is moved up to the Z-above position.
   3. Reaction mixture aspirate.
      (a) Pipette R-axis is moved over the RV incubation well.
      (b) Pipette Z-axis is moved down to the Z-LLS position.
      (c) LLS is enabled to ensure no liquid currently detected.
      (d) Pipette Z-axis is moved down at constant speed until fluid is detected or until the Z-Asp limit is reached (it will be assumed that fluid is detected).
      (e) Based on the Z-height position at which fluid is detected and the Z-height/volume table, the system calculates the volume of fluid in the well and compares it to the volume specified in the pipetting description. If sufficient volume is present, the aspiration sequence is initiated (if sufficient volume is not present, the test is aborted and the test request moved to the exception list).
      (f) The following occur simultaneously until the total volume of reaction mixture required is aspirated:
         (1) Pipette Z-axis motor is moved down at a rate of "X" steps/sec.
         (2) Syringe aspirates "X" uL at a rate of "X" ul/sec.
      (g) LLS is checked to ensure probe still in liquid.
      (h) LLS is disabled.
      (i) Pipette Z-axis is moved up to the Z-clear position.
   4. Reaction mixture dispense onto matrix.
      (a) The following are performed simultaneously and concurrently with the reaction mixture aspirate (above):
         (i) The auxiliary carousel is moved so that the cartridge is at the pipetting station.
         (ii) Pipette R-axis is moved over the MEIA cartridge (matrix) surface.
         (iii) Pipette Z-axis is moved down to the matrix dispense position.
         (iv) Syringe dispenses "X" uL of reaction mixture at a rate of "X" ul/sec.
         (v) System delays "X" seconds until reaction mixture has been absorbed by matrix.
      5. Buffer wash of matrix.
         (a) Syringe dispenses "X" uL of buffer at a rate of "X" ul/sec.
         (b) Pipette Z-axis is moved up to the Z-clear position.
      6. Probe post-wash.
         The probe is again washed to ensure that it is free from contamination as described in section 6 (Sample kitting).
   7. When incubation timer expires, next pipette activity begins.
E. THIRD PIPETTE ACTIVITY (conjugate addition)
   1. Incubation timer is set according to assay file specifications.
   2. Conjugate aspirate.
      (a) The process carousel is moved so that the RV is at the pipetting station.
      (b) Syringe aspirates "X" uL of air at a rate of "X" ul/sec.
      (c) Pipette R-axis is moved over the RV reagent 1 (conjugate) well.
      (d) Pipette Z-axis is moved down to the Z-above position.
      (e) LLS is enabled to ensure no liquid currently detected.
      (f) Pipette Z-axis is moved down at constant speed until fluid is detected or until the Z-Asp limit is reached (it will be assumed that fluid is detected).
      (g) Based on the Z-height position at which fluid is detected and the Z-height/volume table, the System calculates the volume of fluid in the well and compares it to the volume specified in the pipetting description. If sufficient volume is present, the aspiration sequence is initiated (if sufficient volume is not present, the test is aborted and the test request moved to the exception list).
      (h) The following occur simultaneously until the total volume of conjugate required is aspirated:
         (i) Pipette Z-axis motor is moved down at a rate of "X" steps/sec.
         (ii) Syringe aspirates "X" uL at a rate of "X" ul/sec.
      (i) LLS is checked to ensure probe still in liquid.
      (j) LLS is disabled.
      (k) Pipette Z-axis is moved up to the Z-clear position.
   3. Conjugate dispense (performed simultaneously).
      (a) The auxiliary carousel is moved so that the cartridge is at the pipetting station.
      (b) Pipette R-axis is moved over the cartridge (matrix) surface.
      (c) Pipette Z-axis is moved down to the matrix dispense position.
      (d) Syringe dispenses "X" uL of conjugate at a rate of "X" ul/sec.
      (e) Pipette Z-axis is moved up to the Z-clear position.
      (f) Wait "X" seconds until reaction mixture has been absorbed by matrix.
   4. Probe post-wash.
      The probe is again washed to ensure that it is free from contamination as described in section 6 (Sample kitting).
F. RV UNLOAD (This activity occurs when resources are not in use)
   1. The following are performed simultaneously:
      (a) Process carousel is rotated so that the empty position is at the transfer station.
      (b) Transfer mechanism 0-axis is moved to process carousel.
   2. RV is grabbed with the transfer mechanism R-axis and pulled into the transfer mechanism.
   3. Transfer mechanism 0-axis is rotated so that RV is aligned with the waste container.
   4. RV is pushed into the waste container.
   5. Check incubation timer. When expires start next activity.
G. MEIA READ PREPARATION
   1. Lamp intensity is brought from simmer state to burn state.
   2. PMT gain is set.
H. MATRIX WASH
   1. Auxiliary carousel is rotated so that the cartridge is at the matrix wash station.
   2. The following steps are repeated until all the buffer specified in the assay file for cartridge wash has been dispensed.
      (a) "X" uL of heated MEIA buffer are dispensed in 5OuL cycles at a rate of "X" ul/sec onto the matrix.
      (b) Wait "n" seconds.
I. MUP DISPENSE
   1. Auxiliary carousel is rotated so that the cartridge is at the MUP station.
   2. 50uL of heated MUP are dispensed at a rate of "X" uL/sec onto the matrix.
   3. Wait "n" seconds.
J. MEIA READ
   1. Auxiliary carousel is rotated so that the cartridge is at the read station.
   2. The following steps are repeated until the number of micro-reads specified in the assay file have been taken (usually 8)
      (a) Read for "X.XX" seconds.
      (b) Wait "X.XX" seconds.
   3. The reader is returned to its idle state.
      (a) Lamp intensity is turned to simmer state.
      (b) PMT gain is set.
   4. The raw reads are converted to normalized reads (light intensity hitting detector/lamp intensity) by the optics microprocessor.
   5. A rate is calculated by the System from the normalized reads vs time.
   6. For quantitative assays, the rate is fitted to a calibration curve to yield a concentration result
   7. For qualitative assays, the sample rate is compared to an index or cutoff rate to determine if the sample is positive or negative (or reactive or nonreactive).
K. CARTRIDGE UNLOAD (This activity occurs when resources are not in use)
   1. Auxiliary carousel is rotated so that cartridge is at the ejector station.
   2. Ejector is cycled to place cartridge into waste container.

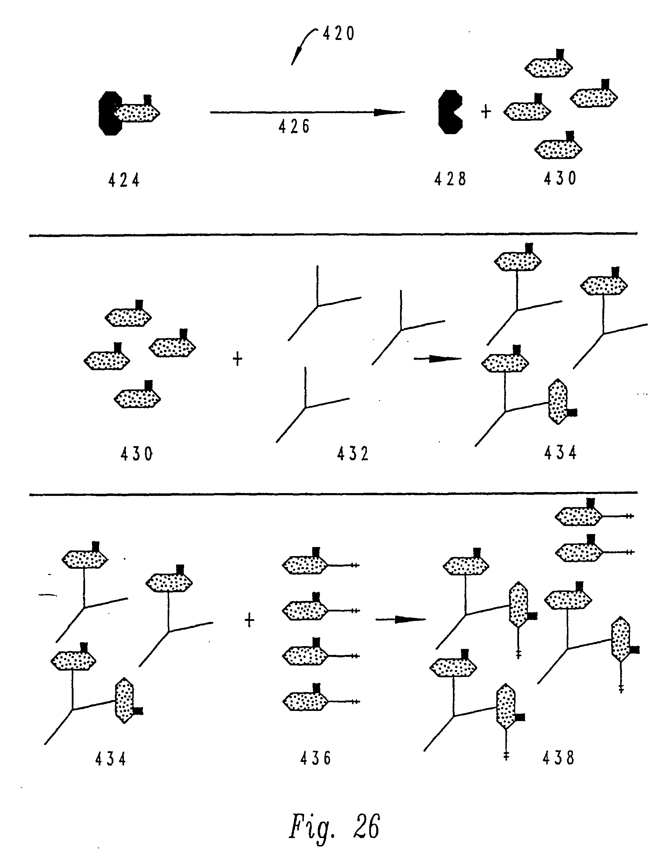

Schematic reaction sequences are presented in FIGURES-26, 27 and 28 which are typical of assays that can be handled by the automated immunoassay analytical system of the invention. In FIGURE 26, a T4 assay, FPIA sequence 420, is presented wherein Step 1, T4 bound by thyroxine binding protein (TBP) 424, is reacted with T4 displacing agent 426 to yield TBP 428 plus unbound T4 (430). In step 2, the T4 (430) is added to T4 antibody 432 which yields a reaction product 434 (T4 antibody-T4 complex). In Step 3, the T4 antibody-T4 complex 434 is treated with T4 tracer (fluorescent) 436 which yields a fluorescent polarization measurable reaction product 438.

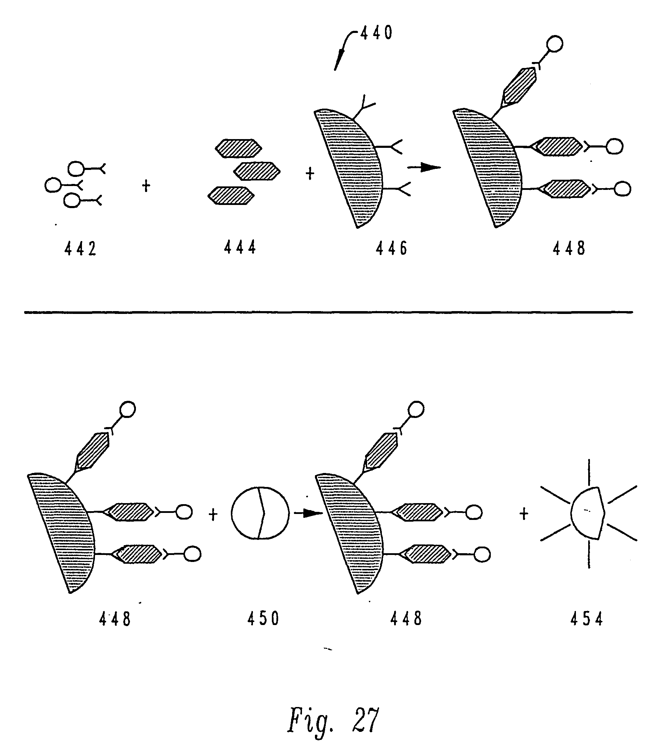

In FIGURE 27, a schematic reaction sequence 440 for a 1-step sandwich MEIA determination (ferritin) is presented. In Steps 1 and 2 an anti-ferritin alkaline phosphatase conjugate is mixed with ferritin sample 444 and anti-ferritin microparticles 446 to yield a ferritin antibody-antigen-antibody complex 448. In step 3, the antibody-antigen-antibody complex 448 is reacted with 4-methylumbelliferyl phosphate (MUP) 450 which yields methylumbelliferone (MU) which is fluorescent. The rate of MU production is measured.

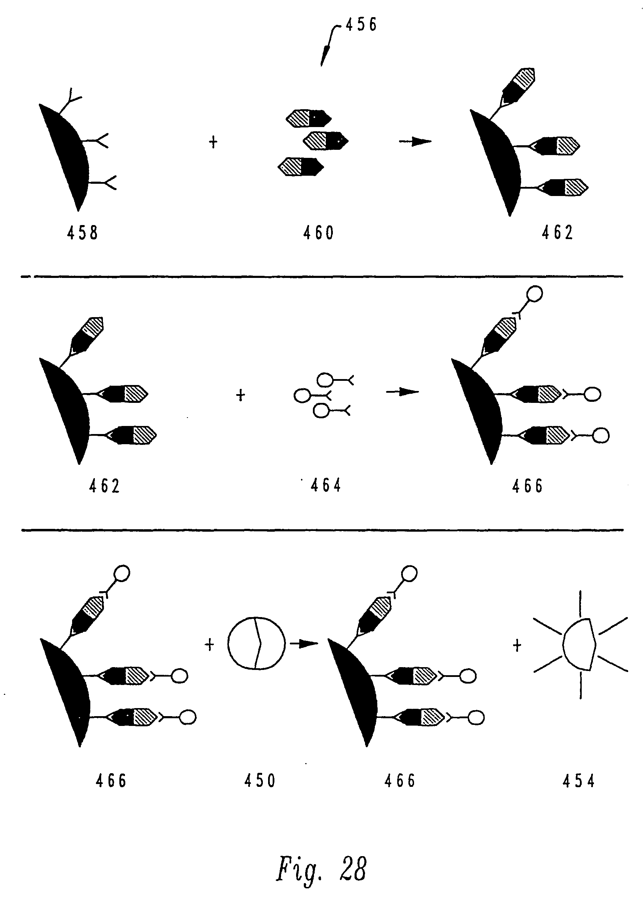

In FIGURE 28, the schematic reaction sequence 456 for a 2-step sandwich MEIA is provided for HTSH assay. Anti-HTSH specific microparticles 458 are added to the HTSH sample 460 which provides a reaction product HTSH antibody-antigen complex 462. In Steps 2 through 4, the complex 462 is combined with an anti-hTSH alkaline phosphatase 464 yielding hTSH antibody-antigen-antibody complex 466. In step 5, the complex 466 is reacted with MUP 450 to yield MU which is fluorescent. The rate of MU production is measured.

In accordance with the embodiments of the present invention, the automated immunoassay analytical system provides apparatus, software, hardware and process technology for performing a multitude of assays continuously and with random access being available to the operator. The utilization of carousel pipettor technology for kitting and pipetting operations at either the main carousel or the process carousel, depending on the scheduled test, provides scheduling flexibilities heretofore unachievable. The inventive system allows for a commonality of kitting and pipetting for either immuno precipitation or competitive immunoassay technologies utilizing a common main carousel, transfer station, first kitting and pipetting probe and process carousel as well as a second pipetting probe before separating into respective apparatus and process requirements. Also shared is the commonality of cabinetry disposal and supply materials as well as a common computer network for scheduling, testing, kitting and pipetting.

### EXAMPLE 1

### Beta-hCG Assay Protocol

The beta-hCG protocol (TABLE 1) is an assay sensitive to carryover which can be performed on the automated continuous and random access analytical system described herein. It is an example of precision pipetting of sample to the incubation well followed by a wash. The reagents are sequentially aspirated and dispensing to the sample well. This sequential pipetting saves time as no washing occurs between reagent applications. The wash requirements in the Kitting Center for this assay include the method parameters shown (NOTE 1). Since the wash recommendations in the Kitting Center result in carryover of 1 ppm or less to the next kitting activity, so that even beta-hCG is not susceptible or contaminating (these indices are each = 1), no prewashes are needed; the post wash is number 3. This wash occurs at the end of the block shown in (NOTE 3). The contamination of the reagent pack bottle following sample is reduced to insignificant levels by wash to waste cup of 200 ul, followed by a 2 ml wash to the wash cup as shown in (NOTE 2).

The parameters of the present method for the processing area are identified in (NOTE 4). The first pipette block transfers only reagents, so that it is susceptible (calls for 1 ml super wash), but not contaminating, and requires minimal post wash of about 2 ml. The second pipette block transfers about 93 ul of sample mixture to the matrix. This step is susceptible to carryover (*sus* index = 2) and is contaminating (no airgap, *con* index = 3) and requires about a 4 ml post wash to keep carryover to about 10 ppm or less. HAVABM assay is an example of separate pipetting of sample followed by reagents (TABLE 2). After each aspiration and dispensation of sample and reagent, a WASH2 WASTE_CUP of 500 ul and WASH2 WASH_CUP of 1,000 ul keeps each reagent from being contaminated. The post wash of 2 ml is called (NOTE 5) and occurs after kitting is complete (NOTE 6). This minimal post wash results in a total wash volume after sample of about 6.5 ml, more than enough to prevent a sample carryover.

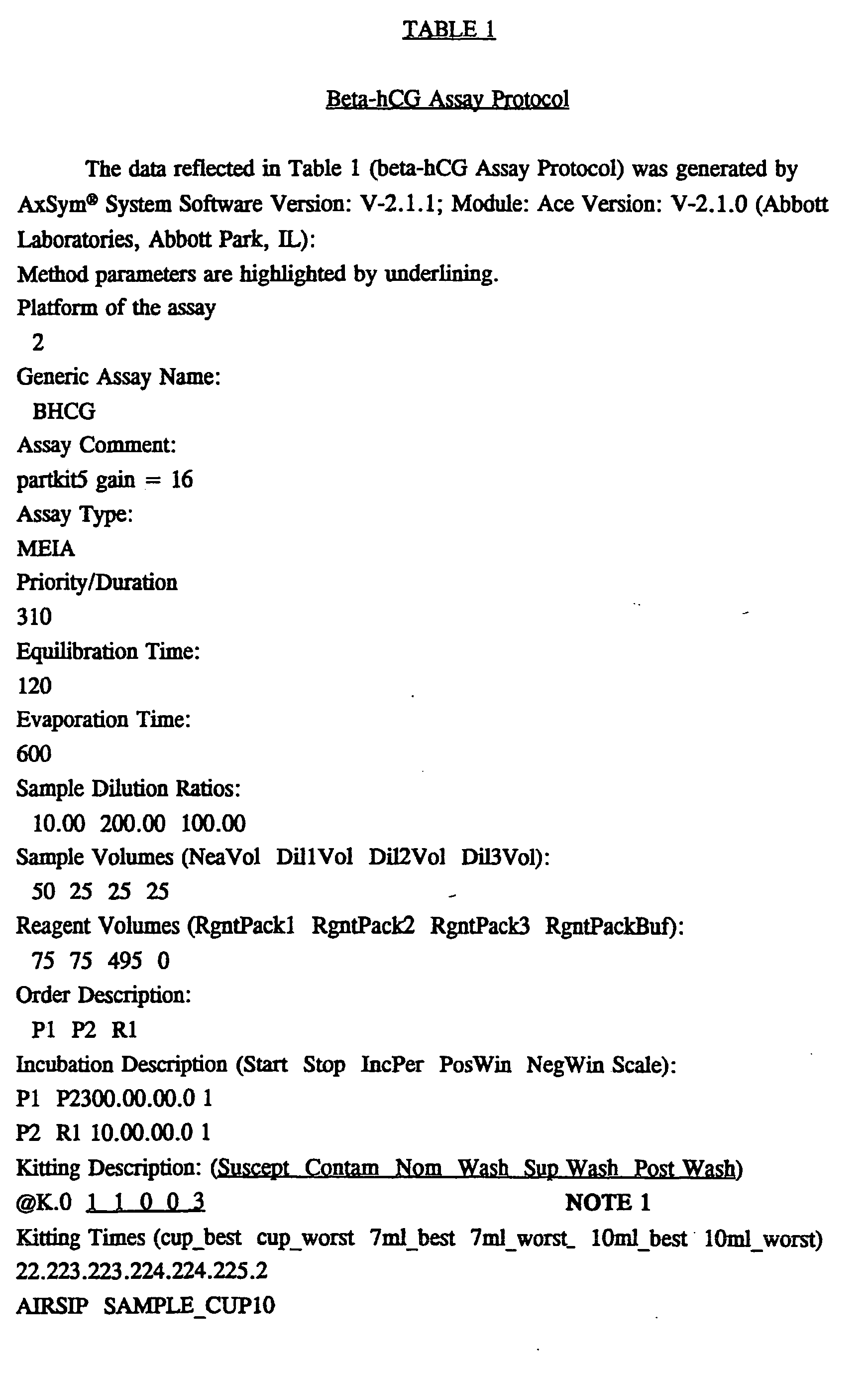

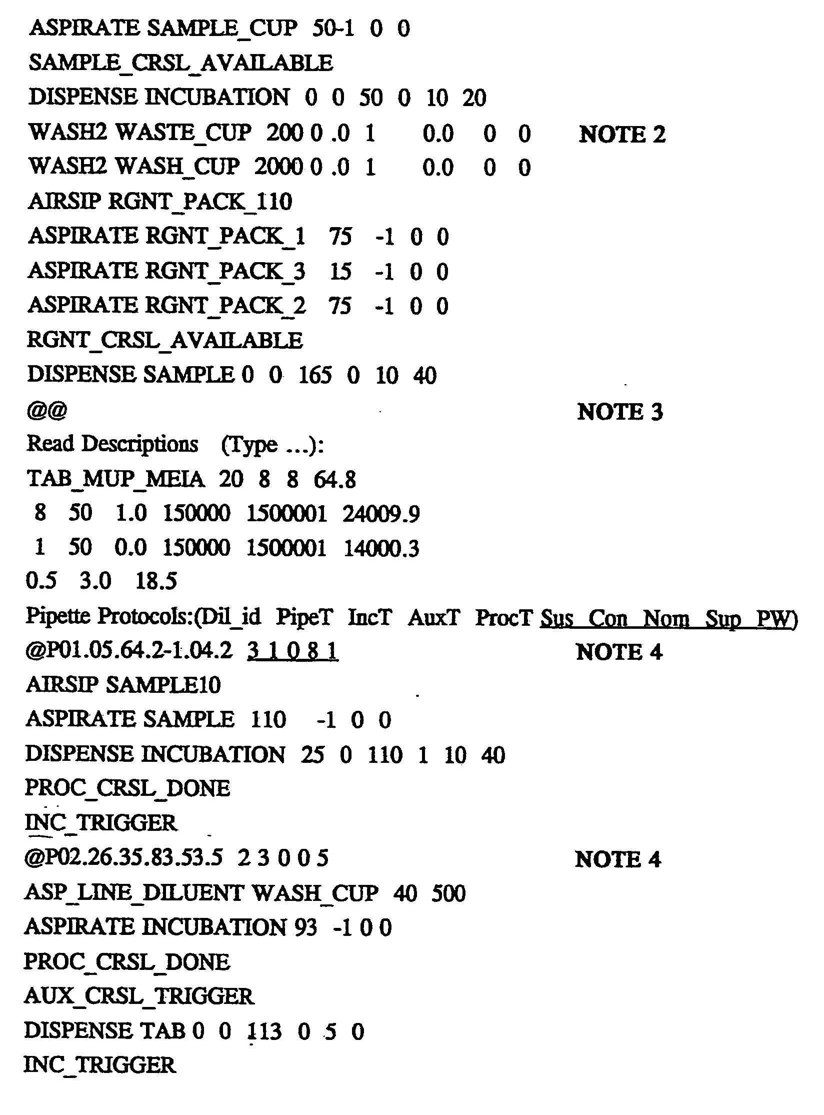

### EXAMPLE 2

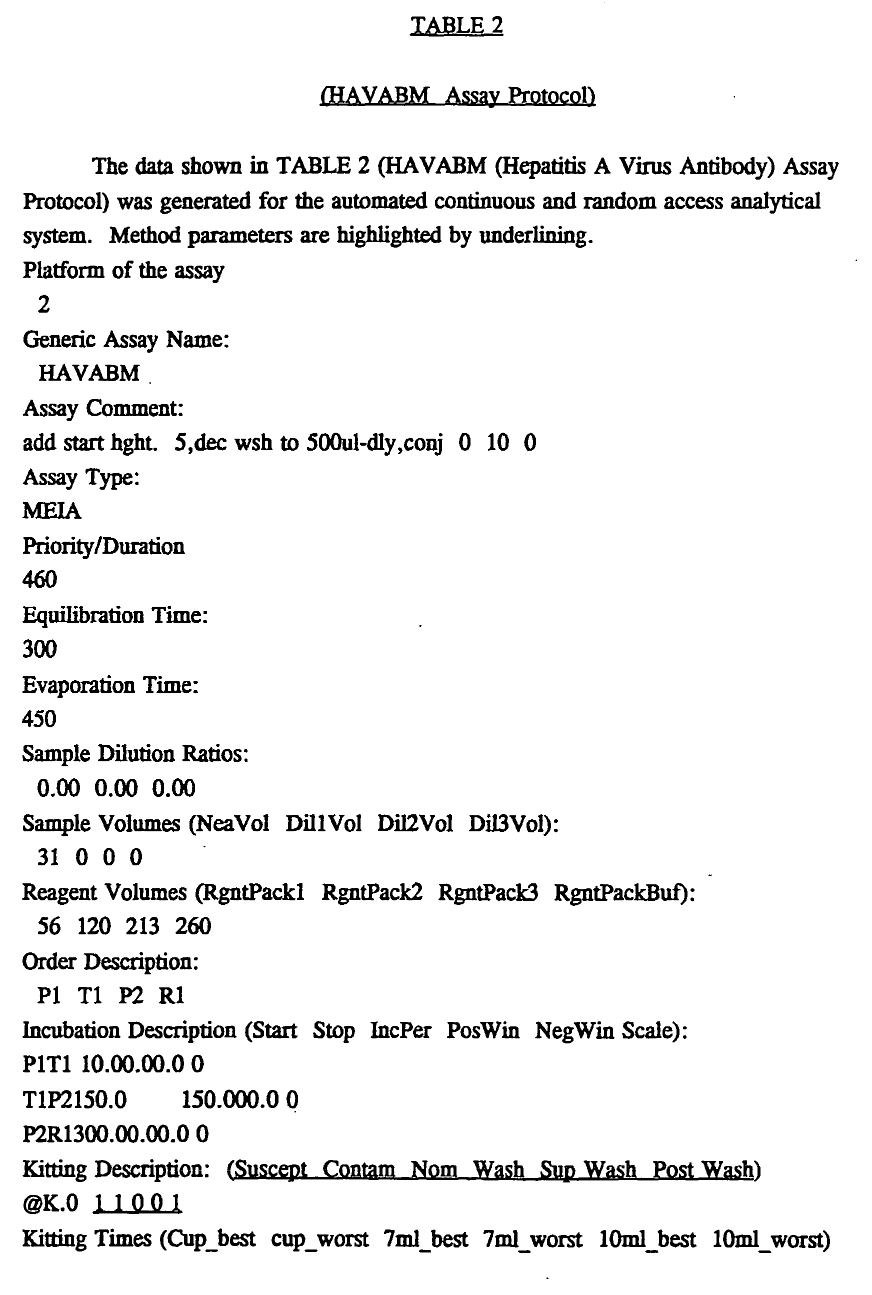

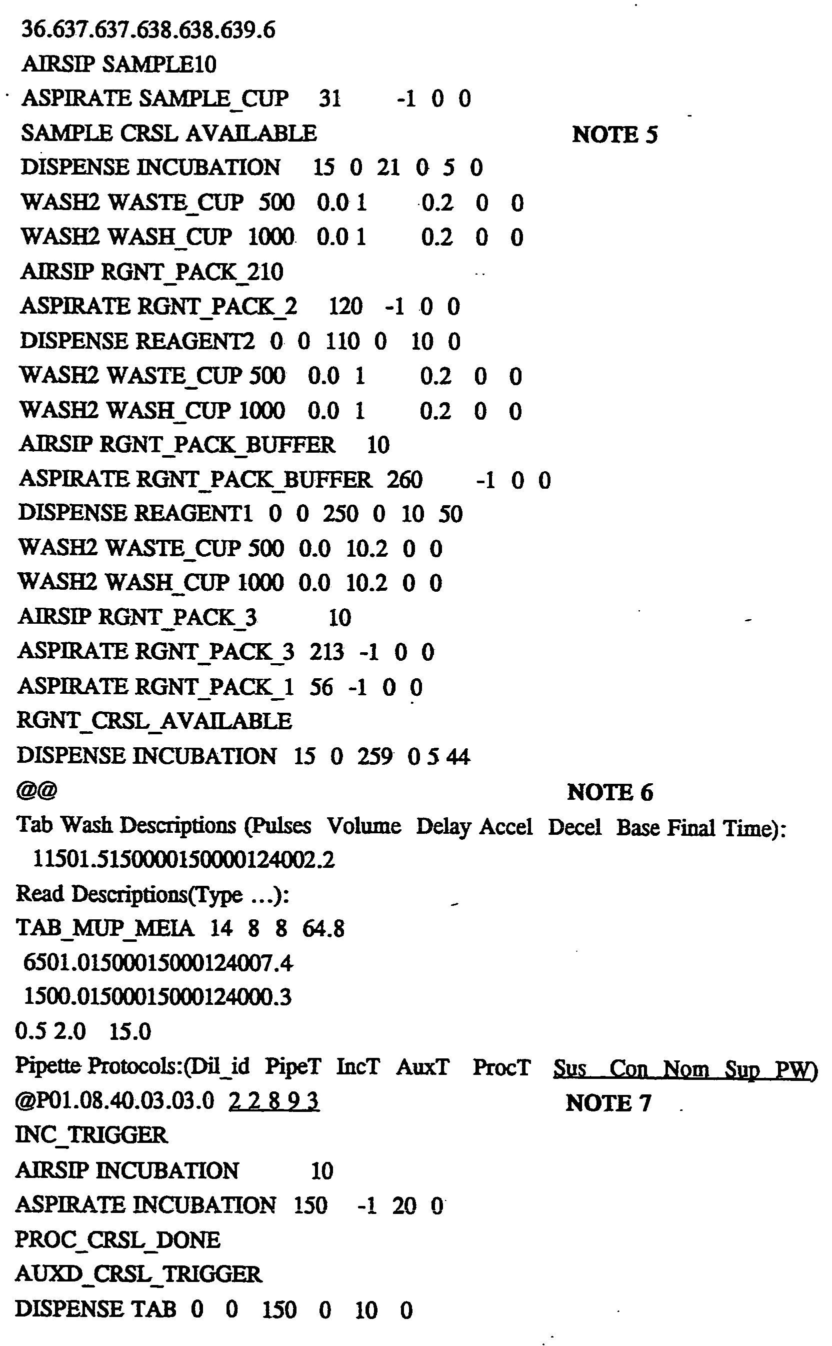

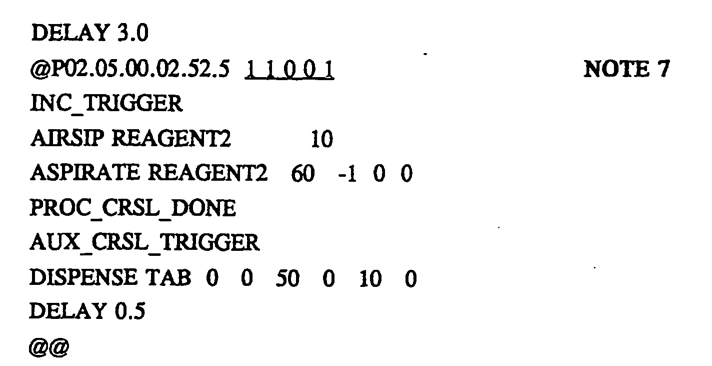

According to a first embodiment of the invention, there is provided a method of operating an automated, continuous and random access analytical system capable of simultaneously effecting multiple assays of a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first liquid sample with one or more of said reagents at different times in said reaction vessel to form a first reaction mixture, (e) mixing aliquots of the same or different one or more samples with one or more of said reagents at different times in different reaction vessels to form multiple, independently scheduled reaction mixures, (f) incubating said multiple reaction mixtures simultaneously and independently, (g) performing more than one scheduled assay on said reaction mixtures in any order in which more than scheduled assays are presented, and (h) analyzing said incubated reaction mixtures independently and individually by at least two assay procedures. Preferably in such method, at least two different assays are scheduled to be performed on the system for a plurality of liquid samples, and said method provides scheduling of said assays in advance of performance thereof, each assay test definition containing several timing parameters with each activity of the assay test containing time values which scheduling uses to determine which resources of the system and activity required by each of said assays and the time period that said resources need. In this embodiment, the system may be capable of allowing special priority handling through stat procedure scheduling of specific samples, wherein said stat procedure scheduling interrupts prior scheduling, allowing the system to finish preparing assays on a current sample and then prepare to perform an assay on the sample through a modification of the scheduling, and preferably calibration procedures scheduling is scheduled as a stat procedure. Also in this embodiment, scheduling for performing assays may maximize the number of assays the system is capable of processing per unit time by allowing sufficient time gaps between assay protocol steps to enable other assay protocol steps to be performed within such time gaps. Also in such method, the scheduling process preferably includes scheduling of each activity to be performed in an assay before the assay is kitted, and scheduling performance of each assay activity prior to its originally scheduled execution time, thus minimizing resource idle time. In such way, assay throughput may be increased in the system. In this embodiment, operating the automated continuous and random access analytical system may include kitting a unit dose disposable by separately transferring assay samples and reagents to a reaction vessel without initiation of an assay reaction sequence.

In said first embodiment, the assay performed on said reaction mixture in said reaction vessel may be a homogeneous assay or a heterogeneous assay. Preferably, at least two assays are immunoassays, for example immunoassays comprised of MEIA and FPIA assays. The analyzing step may include optically monitoring said reaction mixtures. The reaction mixture may be monitored by turbidimetric, colorimetric, fluorometric or luminescent means. Partial initiation of an assay reaction sequence may be achieved simultaneously with the creating of the unit dose disposable. The system may achieve simultaneous creation of unit dose disposables, transferring of a unit dose disposable reaction vessel and mixing of a reaction mixture while incubating multiple reaction mixtures and performing at least one scheduled assay and analysis simultaneously.

According to a second embodiment of the invention, there is provided a method of operating an automated, continuous and random access analytical system capable of simultaneously effecting multiple assays of a plurality liquid samples, comprising (a) introducing sample cups, reagent packs, and reaction vessels for performing said assays onto concentric carousels of a front end carousel, the reaction vessels being introduced to an outer carousel, (b) identifying the reagent packs and sample cups, (c) scheduling the assays, (d) aligning the sample cups and reagent packs with a reaction vessel at a kitting station by rotating the respective carousels, (e) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack (f) transferring the kitted reaction vessel to a process carousel which is maintained under controlled environment conditions (g) pipetting the sample and various reagents into a reaction well of the reaction vessel, the amounts of reagent, sequencing of transfer and time spacing there between being predetermined by assay scheduling, (h) incubating the pipetted sample and reagent mix, (i) identifying and transferring the incubated mixture in the reaction well to one of at least two assay analytical stations, (j) performing an analysis by reading the prepared reaction mixture and calibrating the reading, and (k) recording the resulting assay reading analysis. Preferably, the front-end carousel and the concentric carousels of the front end carousel as well as the process carousel are rotatably disposed for bidirectional rotational motion about a vertical axis, particularly when the front end carousel capable of bidirectional motion provides a bidirectional shaking motion for stirring or agitating reagent pack reagents after a period of inactivity of the front end carousel. In said second embodiment, preferably both kitting and partial initiation of an assay reaction sequence is achieved simultaneously creating a unit dose disposable within the reaction vessel. The assay being performed on said reaction mixture in said reaction vessel may be a heterogeneous assay or a homogenous assay. Preferably, at least two assays are immunoassays, for example immunoassays comprised of a fluorescent polarization immunoassay and a microparticle immunoassay. In the fluorescence polarization immunoassay, a FPIA reading sequence may include lamp simmer and full burn modes. In the microparticle immunoassay, settling of the microparticles may be substantially eliminated by providing a sufficient sucrose concentration to microparticle diluent ratio to achieve neutral density. Furthermore, in the microparticle immunoassay, the kitted sample and reagents may be pipetted directly from the reaction vessel on the process carousel to a microparticle immunoassay matrix for optically monitoring said reaction mixtures. In said second embodiment of the invention, the reagent packs may be provided with closure elements for avoiding reagent evaporation, particularly when covering is provided to the reagent packs when not in use to avoid evaporation of the reagents. Pipetting functions on the front end carousel and pipetting functions on the process carousel may be achieved by aspirating-dispensing, driven by an airless syringe pump.

In a further embodiment of the invention, there is provided a method of operating an automated, continuous and random access analytical system capable of simultaneously effecting multiple assays to determine the presence or amount of a plurality of analyte of interest in a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first sample with one or more of said reagents at different times in said reaction vessel to form a first reaction mixture, (e) mixing aliquots of the same or different ones of said samples with one or more of said reagents at different times in different reaction vessels to form multiple, independently scheduled reaction mixtures, (f) incubating said multiple reaction mixtures simultaneously and independently, (g) performing more than one scheduled assay on said reaction mixtures in any order in which said scheduled assays are presented, and (h) analyzing said incubated reaction mixtures independently and individually by at least two assay procedures to determine the presence or amount of one or more analyte of interests in said samples.

The present invention also provides an automated, continuous and random access analytical system apparatus capable of simultaneously effecting multiple assays of a plurality of liquid samples, comprising (a) a front end carousel assembly inclusive of a sample cup carousel, a reagent pack carousel and a reaction vessel carousel mounted concentrically and serviced by a transfer pipetting means suitable for kitting a reaction vessel, (b) a transfer station providing means for transferring a kitted reaction vessel to a process carousel, the process carousel maintained within a controlled environment, (c) a process carousel transfer pipetting means suitable for mixing reagents with the sample in a reaction well of the reaction vessel, (d) means for transferring the resulting reaction mixture to one of at least two assay reader means, (e) means for transferring a reaction vessels from the assay reader to a transfer station, and (f) means associated with said transfer station for removing the disposable reaction vessel from the system. One assay reader means may be comprised of a cartridge wheel carousel containing multiple disposable cartridges, the apparatus providing means for supplying said cartridges to the cartridge wheel carousel and disposing of the cartridges from the cartridge wheel carousel. The assay reader means may include means for optically monitoring said assay reaction. Preferably, the assay reader means provides calibration and reader means as well as recording means for the resulting assay data. The transfer station transfer means may be comprised of a carousel rotatable about an axis, an arm with a pick for mating with a reaction vessel transfer projection means and means for pulling the reaction vessel from the front end carousel, rotating and placing the reaction vessel onto the process carousel through rotation and rack and pinion movement of the pick arm. The sample handling means and reagent handling means may include means for identifying said liquid samples and liquid reagents from coded information associated with the sample cups and the reagent packs. The apparatus may further include means to store the output readings of the assay reader. The apparatus may further include means to calculate the concentration of an analyte from the output readings of said assay reader. Advantageously, the reaction vessel may contain a reaction cuvette having a physical characteristic of low birefringence through an optical read region.

A still further embodiment of the invention is an automated, continuous and random access analytical system capable of simultaneously effecting multiple assays of a plurality of liquid samples, comprising (a) a front end carousel assembly inclusive of sample cup carousel, reagent pack carousel and reaction vessel carousel, the reaction vessel carousel being concentrically mounted exterior of the reagent pack carousel and the reagent pack carousel being concentrically mounted exterior of the sample cup carousel, (b) means for rotating the respective carousels to align with a kitting pippettor means for kitting reaction vessels, (c) means for transferring kitted reaction vessels from the reaction vessel carousel to a transfer station, the transfer station providing means for transferring the reaction vessel to a process carousel, the process carousel having environmental means for maintaining temperature control and timing of reaction incubation, (d) a transfer pippettor means for servicing the process carousel and a cartridge wheel carousel offset from the process carousel, the cartridge wheel carousel having means for receiving pipetted reaction mixtures from the process carousel and; means for supplying cartridges to the process carousel, (e) the process carousel having integrated therewith a microparticle enzyme immunoassay reader and processing station, (f) a fluorescent polarization immunoassay reader and processing station integrated with the process carousel, (g) means for removing the reaction vessel from the process carousel by operation of the transfer station and means for removing the cartridges from the cartridge wheel carousel, and (h) means for analyzing the reaction mixture either by fluorescent polarization immunoassay or microparticle immunoassay. The assay-reader means may include means for optically monitoring said assay reaction. The assay reader means may provide calibration and reader means as well as recording means for the resulting assay data. The transfer station transfer means may be comprised of a carousel rotatable about an axis, an arm with a pick for mating with a reaction vessel transfer projection means and means for pulling the reaction vessel from the front end carousel, rotating and placing the reaction vessel onto the process carousel through rotation and rack and pinion movement of the pick arm. The sample handling means and reagent handling means may include means for identifying said liquid samples and liquid reagents from coded information associated with the sample cups and the reagent packs. The system may include means to store the output readings of the assay reader. The system may include a means to calculate the concentration of an analyte from the output readings of said assay reader. Advantageously, the sample cup may be free standing on its base when separated from the sample cup carousel.

The present invention also provides a heater assembly for precision temperature and delivery control of liquids, said assembly comprising a body having internal resistance heating means and temperature control means, said body having a cavity suitable for accommodating liquid tubing means, said liquid tubing means in communication with liquid inlet and outlet means, wherein said cavity is suitable for housing a tubing means for maintaining liquids at a predetermined temperature, and wherein said liquid outlet means is suitable for forced or gravity release of the liquids and is mounted directly above analytical means for receiving liquids. The predetermined temperature is preferably from between about ± 1°C and about ± 0.5°C of a required temperature of said liquid. The temperature control means may comprise a thermistor and thermostat for controlling the electrical energy provided to the electrical resistance heating means. The liquid outlet means may be positioned below the lower planar surface of the liquid heater block to thereby provide minimum air gap release to analytical means having openings on a parallel plane, the air gap being preferably from between about 1/2 inch and about 3/8 inch. In the heater assembly, said body is preferably metal. In the liquid heater block assembly, the resistance heating means may comprise multiple loop electrical resistive heating element.

The present invention also provides an automated, continuous and random access analytical system capable of simultaneously effecting multiple assays of a plurality of liquid samples, comprising (a) a front end carousel assembly inclusive of sample cup carousel, reagent pack carousel and reaction vessel carousel, the reaction vessel carousel being concentrically mounted exterior of the reagent pack carousel and the reagent pack carousel being concentrically mounted exterior of the sample cup carousel, (b) means for rotating the respective carousels to align with a kitting pipettor means for kitting reaction vessels, (c) means for transferring kitted reaction vessels from a reaction vessel carousel to a transfer station, the transfer station providing means for transferring the reaction vessel to a process carousel, the process carousel having environmental means for maintaining temperature control and timing of reaction incubation, (d) a transfer pipettor means for servicing the process carousel and a cartridge wheel carousel offset from the process carousel, the cartridge wheel carousel having means for receiving pipetted reaction mixtures from the process carousel and analytical means for receiving liquids from a heater assembly, said heater assembly comprising a metal body having internal resistance heating means and temperature control means, said body having a cavity suitable for accommodating liquid tubing means, said liquid tubing means in communication with liquid inlet and outlet means, wherein said cavity is suitable for housing a tubing means for maintaining liquids at a predetermined temperature, and wherein said liquid outlet means is suitable for forced or gravity release of the liquids and is mounted directly above the analytical means for receiving the liquids, (e) means for supplying cartridges to the process carousel, (f) a microparticle enzyme immunoassay reader and processing station integrated with the process carousel, (g) a fluorescent polarization immunoassay reader and process station integrated with the process carousel, (h) means for removing the reaction vessel from the process carousel by operation of the transfer station and means for removing the cartridges from the cartridge wheel carousel, and (i) means for analyzing the reaction mixture either by fluorescent polarization immunoassay or microparticle immunoassay. The predetermined temperature is from between about ± 1 °C and about ± 0.5°C.

According to a further method of the invention of operating an automated analytical system, the method comprises the steps of (a) identifying interactions between steps in a random access system, (b) allowing for random access processing, (c) randomly inserting and removing events from a prescheduled timeline, and (d) continuing operations of scheduling of events while performing analytical steps. In one embodiment of such method, the events are pipetting steps and the interactions are cross-contamination of liquid test samples or reagents within an analytical system for the analysis of test samples. In the latter embodiment, preferably the random inserting and removing events from a prescheduled timeline comprises the steps of inserting and removing pipetting steps from a processing timeline while maintaining control over cross-contamination, particularly when each pipetting step comprises two index values and three wash parameters, the two index values comprising (a) susceptibility to contamination, and (b) probability to contaminate, and the wash parameters comprise (a) nominal prewash number, (b) super prewash number, and (c) post wash number, whereby the wash parameters are numbers that identify washes in a wash library and the susceptibility of contamination and probability of contaminant parameters are used to indicate the probability of cross-contamination occurrence.

The present invention provides a further method of operating an analytical instrument capable of performing multiple assays on one or more test samples, said method comprising the steps of (a) scheduling operations of the instrument on each test sample, (b) scheduling a cleaning operation during operation of the instrument, (c) conducting one or more assay on the test samples by (1) performing the scheduled instrument operations, and (2) performing a cleaning operation during operation of the scheduled instrument scheduled prior to said cleaning operation, and (d) continuing operation of scheduling of events while performing the assays.

Additionally, a method of operating an automated, continuous and random access analytical system capable of simultaneously effecting multiple assays of a plurality of test samples is provided, said method comprising the steps of (a) scheduling various assays of a plurality of test samples, (b) creating one or more unit dose disposables by separately transferring a first of said test samples and reagents to a reaction vessel without initiation of an assay reaction sequence, (c) transferring one or more said unit dose disposables to a processing work station, (d) mixing an aliquot of said first test sample with one or more of said reagents at different times in said reaction vessel to form a first reaction mixture, (e) mixing aliquots of the same or different one or more samples with one or more of said reagents at different times and different reaction vessels to form multiple, independently scheduled reactions mixtures, (f) identifying interactions between pipetting steps, (g) randomly inserting and removing pipetting steps from a processing timeline while maintaining control on cross-contamination, (h) continuing operations of scheduling of events while performing multiple assays of a plurality of test samples, (i) incubating said multiple reaction mixtures simultaneously and independently, (i) performing more than one scheduled assay on one reaction mixture in any order in which more than scheduled assays are presented, and (k) analyzing said incubated reaction mixtures independently and individually by at least two assay procedures. Preferably, the events are pipetting steps and the interactions are cross-contamination within an analytical system for the analysis of test samples. Preferably, the random inserting and removing events from a prescheduled timeline comprises the steps of inserting and removing pipetting steps from a processing timeline while maintaining control over cross-contamination, particularly when each pipetting step comprises two index values and three wash parameters, the two index values comprising (a) susceptibility to contamination, and (b) probability to contaminate, and the wash parameters comprise (a) a nominal prewash number, (b) super prewash number, and (c) post wash number, whereby the wash parameters are numbers that identify washes in a wash library and the susceptibility of contamination and probability of contaminant parameters are used to indicate the probability of cross-contamination occurrence. Preferably, at least two different assays are scheduled to be performed on the system for a plurality of test samples, said method providing scheduling of said assays in advance of performance thereof, each assay test definition containing several timing parameters with each activity of the assay test containing time values which scheduling uses to determine which resources of the system and activity required by each of said assays and the time period that said resources need. In one embodiment of the method, the scheduling process includes scheduling of each activity to be performed in an assay before the assay is kitted, and scheduling performance of each assay activity prior to its original scheduled execution time, thus minimizing resource and idle time. Preferably in such embodiment, operating the automated, continuous and random access analytical system includes kitting a unit dose disposable by separately transferring assay samples and reagents to a reaction vessel without initiation of an assay reaction sequence. Preferably in such embodiment, the system is capable of allowing special priority handling through stat procedure scheduling of specific samples, and said stat procedure scheduling interrupts prior scheduling, allowing the system to finish preparing assays on a current sample and then prepare to perform an assay on the sample through a modification of the scheduling. Preferably in such embodiment, scheduling for performing assays maximizes the number of assays the system is capable of processing per unit time by allowing sufficient time gaps between assay protocol steps to enable other assay protocol steps to be performed within such time gaps.

The present invention additionally provides a method of operating an automated, continuous and random access analytical system capable of simultaneously effecting multiple assays of a plurality of test samples, comprising (a) introducing sample cups, reagent packs and reaction vessels for performing said assays onto concentric carousels of a front end carousel, the reaction vessels being introduced to an outer carousel, (b) identifying the reagent packs and sample cups, (c) scheduling the assay, (d) aligning the sample cups and reagent packs with a reaction vessel at a kitting station by rotating the respective carousel, (e) kitting a unit dose disposable in a reaction vessel having multiple independent open chambers in accordance with the scheduled assay by transfer of the sample from sample cup to a reaction vessel chamber and transfer of specific reagents to separate reaction vessel chambers from the reagent pack, (f) transferring the kitted reaction vessel to a process carousel which is maintained under controlled environment conditions, (g) pipetting the sample and various reagents into a reaction well of the reaction vessel, the amounts of reagent, sequencing of transfer and time spacing therebetween being predetermined by assay scheduling, (h) identifying interactions between pipetting steps, (i) randomly inserting and removing pipetting steps from the prescheduled timeline, (i) incubating the pipetted sample and reagent mix, (k) identifying and transferring the incubated mixture in the reaction well of one of at least two assay analytical stations, (1) performing an analysis by reading the prepared reaction mixture and calibrating the reading, and (m) recording the resulting assay reading analyses. In one embodiment of such method, the events are pipetting steps and the interactions are cross-contamination within an analytical system for the analysis of test samples. Preferably, the random inserting and removing events from a prescheduled timeline comprises the steps of inserting and removing pipetting steps from a processing timeline while maintaining control over cross-contamination. Each pipetting step may comprise two index values and three wash parameters, the two index values comprising (a) susceptibility to contamination, and (b) probability to contaminate, and the wash parameters comprise (a) a nominal prewash number, (b) super prewash number, and (c) post wash number, whereby the wash parameters are numbers that identify washes in a wash library and the susceptibility of contamination and probability of contaminant parameters are used to indicate the probability of cross-contamination occurrence.

The invention additionally provides a test sample container assembly for loading a plurality of test samples onto a test sample carousel of an automated analytical system, said assembly comprising: a housing with two parallel curved sides, said sides having the same radius of curvature as the test sample carousel; a top shelf with at least two openings for receiving test sample containers and a bottom portion parallel to and spaced apart from the top shelf, wherein said bottom portion and said curved surfaces define a cavity therebetween wherein the at least two openings provide a predefined vertical alignment; and said assembly presents said mounted cups with cup openings in a plane parallel with the plane of the assembly shelf. Preferably, the assembly has handling means elevated above the plane of the shelf. The assembly may have opening and cup receiving sleeve receiving means which define two rows for receiving test sample containers. The assembly may have mounting pins mounted on the bottom for positioning and mounting said test sample container segment assemblies in aligned position on the test sample carousel. The assembly bottom may have pin receiving openings for positioning and mounting onto the test sample carousel, said carousel having pins exposed for receiving the test sample container segment assembly. The assembly cavity may have individual spring means for receiving and holding the test sample containers once inserted into the assembly. The assembly may have cup holding arms spaced between the shelf openings and the sleeves mounted on the bottom. Said cavity may comprise test sample container receiving sleeves mounted on the assembly bottom, said receiving sleeves in alignment with the shelf openings for holding inserted test sample containers.

The invention also provides a test sample tube segment assembly for loading a plurality of test samples contained in a plurality of test tubes onto a test sample carousel of an automated analytical system, said assembly comprising: a housing with two parallel curved sides, said sides having the same radius of curvature as the test sample carousel; a top shelf with at least two openings for receiving test sample adaptor tubes; the assembly having a bottom parallel to and spaced apart from the top shelf, wherein said bottom portion and said curved sides define an assembly cavity therebetween, whereinsaid cavity having test sample tube adapter tube receiving sleeves mounted on the assembly bottom, said receiving sleeves in axial alignment with the shelf openings for holding the inserted test tubes in a predefined vertical alignment; and wherein said assembly presents the open ends of the test tubes in a parallel plane with the plane of the shelf. Preferably, the assembly has handling means elevated above the plane of the shelf. The assembly may have opening and tube receiving sleeve receiving means which define two rows for receiving test sample tube adapter tubes. The assembly may have mounting pins mounted on the bottom for positioning and holding said test sample tube segment assemblies in aligned position on the test sample carousel. The assembly bottom may have pin receiving openings for positioning and mounting the assembly onto the test sample carousel, said carousel having pins exposed for receiving the test sample tube segment assembly. The assembly cavity may have individual spring means for receiving and holding the test sample tube adapter tubes once inserted into the assembly. The assembly may have test sample tube adapter tubes holding arms spaced between the shelf openings and the sleeves mounted on the bottom. One example of the test sample tube is a Vacutainer® tube.

According to a further aspect of the invention, an automated, continuous and random access analytical system capable of simultaneously effecting multiple assays of a plurality of liquid samples is provided, comprising (a) a front end carousel assembly inclusive of test sample container carousel, reagent pack carousel and reaction vessel carousel, the reaction vessel carousel being concentrically mounted exterior of the reagent pack carousel and the reagent pack carousel being concentrically mounted exterior of the test sample container carousel, (b) a test sample container segment assembly means for loading multiple test sample containers on the test sample carousel, said assembly comprising (i) a housing with two parallel curved sides, said sides having the same radius of curvature as the test sample carousel, (ii) a top shelf with at least two openings for receiving test sample containers, (iii) a bottom parallel to and spaced apart from the top shelf defining with the curved surfaces therebetween a cavity, and (iv) wherein said bottom portion and said curved surfaces define a cavity therebetween wherein the at least two openings provide a predefined vertical alignment container with test sample container openings in a plane parallel with the plane of the assembly shelf, and (c) means for rotating the respective carousels to align with a kitting pipettor means for kitting reaction vessels. Said cavity may comprise test sample container receiving sleeves mounted on the assembly bottom, said receiving sleeves in alignment with the shelf openings for holding inserted test sample containers. Said system may comprise test sample handling means and reagent handling means, said test sample and reagent handling means comprising means for identifying test samples and liquid reagents from coded information associated with the sample containers and reagent packs.

A further embodiment of the invention is a test sample container for use in an automated analytical system, said container comprising modified tubular dimensions within a tubular configuration, said test sample container having upper portion outer skirts for mounting said test sample container onto a test sample container assembly. Preferably, the container upper portion and skirt have a greater outside diameter than the container lower portion and skirt.

A still further embodiment of the invention is a test sample container adapter tube comprising a tube with an expanded upper portion outside diameter and open end cavity of sufficient length to receive a cylindrical test sample container. Preferably, said tube has a liquid level element comprising a conductive material capable of creating a capacitative path for capacitative lead sensing means.

The present invention also provides a liquid level sensing device for sensing the level of a liquid in a container, said device comprising (a) means for producing an electrical sinusoidal signal, (b) means for evaluating the amount of signal as it propagates from a probe to a sense antenna, (c) circuit means for creating an electrical signal wherein said electrical signal changes when a probe contacts liquid, (d) signal processing means for enhancing the electrical signal and for degrading and suppressing signals not associated with the probe contacting said liquid, (e) decision circuit means for causing a basic digital output indicating either the presence or absence of liquid, and (f) means for utilizing the digital signal for controlling motion, and wherein said sensing device detects signal change and rate of signal change. Preferably, the signal processing means for enhancing the signal comprises a synchronous receiver which provides narrow band acceptance of the electrical signals received by the antenna, particularly when the synchronous receiver comprises a synchronous amplitude detector which multiplies the incoming signal by reference to extract amplitude information from the signal. Such synchronous amplitude detector is preferably followed by a low pass filter to extract the information desired, the low pass filter comprising a linear phase filter. Preferably, the transmitted signal is generated from a reference signal whereby both the transmitted and received signals are of the same frequency. Preferably, the decision circuit means is a differential rate sensing circuitry requiring the receive signal to increase a minimum amount within a specific time reference to indicate liquid level detection, particularly when the decision circuitry comprises an autozero loop means, followed by a fixed threshold.

According to another aspect of the invention, an automated, continuous and random access analytical system apparatus capable of simultaneously effecting multiple assays of a plurality of liquid samples is provided, comprising (a) a front end carousel assembly inclusive of a sample cup carousel, a reagent pack carousel and a reaction vessel carousel mounted concentrically and serviced by a transfer pipetting means suitable for kitting a reaction vessel, (b) means for producing an electrical sinusoidal signal, means for evaluating the amount of signal as it propagates from a probe to a sense antenna, circuit means for creating an electrical signal wherein said electrical signal changes when a probe contacts liquid, signal processing means for enhancing the electrical signal and for degrading and suppressing signals not associated with the probe contacting said liquid, decision circuit means for causing a basic digital output indicating either the presence or absence of liquid, and means for utilizing the digital signal for controlling motion, and wherein said sensing device detects signal change and rate of signal change, (c) transfer station providing means for transferring a kitted reaction vessel to a process carousel, the process carousel maintained within a controlled environment, (d) a process carousel transfer pipetting means suitable for mixing reagents with the sample in a reaction well of the reaction vessel, (e) means for transferring the resulting reaction mixture to one of at least two assay reader means, (f) means for transferring a reaction vessels from the assay reader to a transfer station, and (g) means associated with said transfer station for removing the disposable reaction vessel from the system.

The present invention furthermore provides a method of sensing liquid level of a liquid in a container, said method comprising the steps of (a) generating an electrical sinusoidal signal, (b) evaluating the amount of signal as the signal propagates from a probe to a sense antenna, (c) positioning the sense antenna at a predetermined distance from the probe, (d) inserting a liquid container between said probe and said sensing antenna, (e) filtering and processing the electrical signal by enhancing the signal sought while degrading and suppressing electrical signals not associated with the probe when contacting liquid, (f) detecting signal change and rate of signal change, (g) electronically evaluating the process signal to determine when liquid has been contacted to thereby cause a basic digital output indicating either the presence or the absence of liquid, and (h) utilizing the digital signal for controlling motion of the probe in relationship to the liquid container. In one embodiment of such method, the signal enhancing occurs by providing narrow band acceptance of the electrical signals received by the antenna. In the latter embodiment, enhancing of the signal is through multiplying the incoming signal by reference to extract amplitude information from the signal, particularly when filtering and suppressing electrical signals not associated with the probe is achieved by low pass filtering to extract the information desired. In another embodiment of such method, the filtering and processing of the electrical signal is achieved by transmitting signal generating said signal from a reference signal whereby both the transmitted and received signals are of the same frequency. The method preferably employs differential rate sensing circuitry requiring the receive signal to increase a minimal amount within a specific time reference indicating liquid level detection.

According to a further aspect of the invention, a method of operating an automated, continuous and random access analytical system capable of simultaneously effecting multiple assays of a plurality liquid samples is provided, comprising (a) introducing containers for liquids for performing said assays onto concentric carousels of a front end carousel, said containers comprising sample cups, reagent packs, and reaction vessels, the reaction vessels being introduced to an outer carousel, (b) sensing liquid levels in said containers by producing an electrical sinusoidal signal, means for evaluating the amount of signal as it propagates from a probe to a sense antenna, circuit means for creating an electrical signal wherein said electrical signal changes when a probe contacts liquid, signal processing means for enhancing the electrical signal and for degrading and suppressing signals not associated with the probe contacting said liquid, decision circuit means for causing a basic digital output indicating either the presence or absence of liquid, and means for utilizing the digital signal for controlling motion, and wherein said sensing device detects signal change and rate of signal change, (c) identifying the reagent packs and sample cups, (d) scheduling the assays, (e) aligning the sample cups and reagent packs with a reaction vessel at a kitting station by rotating the respective carousels, (f) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack, (g) transferring the kitted reaction vessel to a process carousel which is maintained under controlled environment conditions, (h) pipetting the sample and various reagents into a reaction chamber of the reaction vessel, the amounts of reagent, sequencing of transfer and time spacing there between being predetermined by assay scheduling, (i) incubating the pipetted sample and reagent mix, (j) identifying and transferring the incubated mixture in the reaction chamber to one of at least two assay analytical stations, (k) performing an analysis by reading the prepared reaction mixture and calibrating the reading, and (l) recording the resulting assay reading analysis.

The present invention also provides a liquid level sensing device for sensing the level of liquid in a container, said device comprising (a) means for producing an electrical sinusoidal signal, (b) detection means comprising bridge circuit means connected to a probe and liquid sample wherein said circuit means creates an electrical signal which changes when the probe touches liquid and wherein said detection means detects capacitance change when the probe contacts liquid, (c) a phase lock loop for detecting background noise, said phase lock loop comprising a narrow center frequency, long filter band width, and narrow filter band width, and (d) means for measuring said capacitance in the form of an electrical phase shift of the sinusoidal signal present on the probe. Preferably, the high center frequency utilizes frequencies higher than the frequencies produced by system noise dominated by noise. Preferably, the liquid level sensing device comprises a variable phase control means, wherein said variable phase control means comprises a phase shifter, a multiplier and a digital DC control tracking counter means for measuring said capacitance in the form of an electrical phase shift of the sinusoidal signal present on the probe, and the liquid level sensing device may further comprise: means for converting a DC voltage signal to a digital signal by an analog to digital converter; and means for utilizing the digital signal for controlling motion relationship between the liquid container and the probe.

According to a further embodiment of the invention, a method of sensing liquid level in a container is provided comprising the steps of (a) generating an electrical sinusoidal signal, (b) detecting capacitance change when the probe contacts the liquid in the container through a bridge circuit between the probe and the liquid in the container, (c) forming an electrical phase shift of the sinusoidal signal present on the probe, and (d) providing a phase lock loop for tracking background, wherein said phase lock loop provides a center frequency along a narrow filter band width, said center frequency peaking at frequencies greater than background noise frequencies induced by system noise. Measuring of capacitance change may be in the form of an electrical phase shift of the sinusoidal signal present on the probe, particularly when such method includes converting a DC voltage signal to a digital signal and utilizing the digital signal for controlling motion relationship between the liquid container and the probe.

According to another aspect of the invention, a method of operating an automated, continuous and random access analytical system capable of simultaneously effecting multiple'assays of a plurality liquid samples by sensing liquid level in a container is provided comprising the steps of (a) introducing containers for liquids for performing said assays onto concentric carousels of a front end carousel, said containers comprising sample cups, reagent packs, and reaction vessels, the reaction vessels being introduced to an outer carousel, (b) sensing liquid level of liquids in such containers by (1) generating an electrical sinusoidal signal, (2) detecting capacitance change when the probe contacts the liquid in the container through a bridge circuit between the probe and the liquid in the container, (3) providing a phase lock loop for tracking background, wherein said phase lock loop provides a center frequency along a narrow filter band width, said center frequency peaking at frequencies greater than background noise frequencies induced by noise, (c) identifying the reagent packs and sample cups, (d) scheduling the assays, (e) means for producing an electrical sinusoidal signal, detection means having a bridge circuit means connected to a probe and liquid sample, said circuit means which creates an electrical signal which changes when the probe touches liquid, said detection means detecting capacitance change when the probe contacts liquid, a phase lock loop for background having a narrow center of frequency, long and narrow filter band width and means for measuring said capacitance in the form of an electrical phase shift of the sinusoidal signal present on the probe, (f) aligning the sample cups and reagent packs with a reaction vessel at a kitting station by rotating the respective carousels, (g) kitting a unit dose disposable in a reaction vessel having multiple independent open chambers in accordance with the scheduled assay by transfer of the sample from the sample cup to a reaction vessel chamber and transfer of specific reagents to separate reaction vessel chambers from the reagent pack, (h) transferring the kitted reaction vessel to a process carousel which is maintained under controlled environment conditions, (i) pipetting the sample and various reagents into a reaction chamber of the reaction vessel, the amounts of reagent, sequencing of transfer and time spacing there between being predetermined by assay scheduling, (j) incubating the pipette sample and reagent mix, (k) identifying and transferring the incubated mixture in the reaction chamber to one of at least two assay analytical stations, (l) performing an analysis by reading the prepared reaction mixture and calibrating the reading, and (m) recording the resulting assay reading analysis.

According to a still further aspect of the invention, an automated, continuous and random access analytical system apparatus capable of simultaneously effecting multiple assays of a plurality of liquid samples is provided comprising (a) a front end carousel assembly inclusive of a sample cup carousel, a reagent pack carousel and a reaction vessel carousel mounted concentrically and serviced by a transfer pipetting means suitable for kitting a reaction vessel, (b) a liquid level sensing device comprising (1) means for producing an electrical sinusoidal signal, (2) detection means comprising a bridge circuit means connected to a probe and liquid sample, wherein said circuit means creates an electrical signal which changes when the probe contacts liquid, and wherein said detection means detects capacitance change when the probe contacts liquid, (3) a phase lock loop for detecting background noise having a narrow center frequency, long filter band width and narrow filter band width, and (4) means for measuring said capacitance in the form of an electrical phase shift of the sinusoidal signal present on the probe;(c) transfer station providing means for transferring a kitted reaction vessel to a process carousel, the process carousel maintained within a controlled environment, (d) a process carousel transfer pipetting means suitable for mixing reagents with the sample in a reaction well of the reaction vessel, (e) means for transferring the resulting reaction mixture to one of at least two assay reader means, (f) means for transferring a reaction vessels from the assay reader to a transfer station, and (g) means associated with said transfer station for removing the disposable reaction vessel from the system.

Another embodiment of the invention is a method of operating an automated, continuous and random access analytical system capable of simultaneously effecting multiple assays of a plurality of liquid samples, comprising (a) scheduling various assays of a plurality of liquid samples, (b) creating one or more unit dose disposables by separately transferring a first of said liquid samples and reagents to a reaction vessel without initiation of an assay reaction sequence, (c) transferring one or more of said unit dose disposables to a processing workstation, (d) mixing an aliquot of said first liquid sample with one or more of said reagents at different times in said reaction vessel to form a first reaction mixture, (e) mixing aliquots of the same or different one or more samples with one or more of said reagents at different times in different reaction vessels to form multiple, independently scheduled reaction mixtures, (f) incubating said multiple reaction mixtures simultaneously and independently, (g) performing at least one scheduled assay on said reaction mixtures in any order in which more than scheduled assays are presented, and (h) analyzing said incubated reaction mixtures independently and individually by at least two assay procedures. Said at least one scheduled assay may be a homogeneous assay or a heterogeneous assay. Said at least one scheduled assay may be an immunoassay. Said analyzing step may include optically monitoring said reaction mixtures. The reaction mixtures may be monitored by turbidimetric, colorimetric, fluorometric, chemiluminscent or luminescent means.

The present invention also provides an automated, continuous and random access analytical system capable of simultaneously effecting multiple assays of a plurality of liquid test samples, said system comprising (a) a front end carousel assembly inclusive of sample cup carousel, reagent pack carousel and reaction vessel carousel, the reaction vessel carousel being concentrically mounted exterior of the reagent pack carousel and the reagent pack carousel being concentrically mounted exterior of the sample cup carousel, (b) means for rotating the respective carousels to align with a kitting pipettor means for kitting reaction vessels, (c) means for transferring kitted reaction vessels from the reaction vessel carousel to a transfer station, the transfer station providing means for transferring the reaction vessel to a process carousel, the process carousel having environmental means for maintaining temperature control and timing of reaction incubation, (d) a transfer pipettor means for servicing the process carousel and a cartridge wheel carousel offset from the process carousel, the cartridge wheel carousel having means for receiving pipetted reaction mixtures from the process carousel and; means for supplying cartridges to the process carousel, (e) at least one reader and processing station integrated with the process carousel, (f) means for performing at least one assay, and (g) means for analyzing the reaction mixture. Said means for performing at least one assay may comprise a heterogeneous assay reader and processing station integrated with the cartridge wheel carousel. Said means for performing at least one assay may comprise a homogeneous assay reader and processing station integrated with the process carousel. Preferably, said means for analyzing the reaction mixture is capable of analyzing fluorescent polarization immunoassay reaction mixtures, chemiluminescent reaction mixtures, or microparticle immunoassay reaction mixtures.

In one embodiment, said means for performing at least one assay comprises a heterogeneous assay reader and processing station integrated with the process carousel. In such embodiment, the assay may be a chemiluminescent heterogeneous assay. In such case, said means for performing a chemiluminescent assay preferably comprises an incubation chamber, wherein all or some reactant components are incubated; a container, separate from said incubation container; a solid, porous element in said container, said solid, porous element having an interactive property with a chemiluminescent moiety in a chemiluminescent assay wherein the chemiluminescent moiety is immobilized by said solid, porous element thereby preventing the migration thereof while permitting passage of other reaction components of said chemiluminescent assay; a porous absorptive material in said container which is chemically interactive with respect to a chemiluminescent activating reaction; means, adjacent an aperture, for evenly distributing to said porous element a chemiluminescent activating solution for said chemiluminescent activating reaction, wherein said means comprises a plurality of ports disposed toward an inclined interior surface of said aperture; means for providing a light-tight seal around said container comprising a shroud for positioning a detection head around said container to create said light-tight seal; and means for photodetection adjacent said aperture. In the latter preferred case, said solid, porous element may comprise a fibrous matrix. Also in the latter preferred case, said interactive property may be a hydrophobic interaction between said chemiluminescent moiety and said solid, porous element, preferably when said solid, porous element is treated with a hydrophobic reagent. Alternatively, in the latter preferred case, said interactive property may be an ionic interaction between said chemiluminescent moiety and said solid, porous element.

The system may comprise means for performing at least one assay including means for conducting chemiluminescent magnetic particle assays, a heterogeneous assay reader and a processing station integrated with the process carousel. Alternatively, the system may comprise means for performing at least one assay including means for conducting chemiluminescent membrane particle assays, a heterogeneous assay reader and processing station integrated with the cartridge wheel carousel.

The present invention also may relate to a method of operating an automated, continuous and random access analytical system capable of simultaneously effecting multiple assays to determine the presence or amount of a plurality of analyte of interest in a plurality of liquid test samples, said method comprising (a) scheduling various assays of a plurality of liquid test samples, (b) creating one or more unit dose disposables by separately transferring a first of said liquid test samples and reagents to a reaction vessel without initiation of an assay reaction sequence, (c) transferring one or more of said unit dose disposables to a processing workstation, (d) mixing an aliquot of said first test sample with one or more of said reagents at different times in said reaction vessel to form a first reaction mixture, (e) mixing aliquots of the same or different ones of said samples with one ormore of said reagents at different times in different reaction vessels to form multiple, independently scheduled reaction mixtures, (f) incubating said multiple reaction mixtures simultaneously andindependently, (g) means for conducting a chemiluminescent assay, (h) performing more than one scheduled assay on said reaction mixtures in any order in which said scheduled assays are presented, and (i) analyzing said incubated reaction mixtures independently and individually by at least two assay procedures to determine the presence or amount of one or more analyte of interests in said test samples. The chemiluminescent assay may be a chemiluminescent magnetic particle assay or a chemiluminescent membrane particle assay.

In a preferred embodiment, said method comprises the steps of: forming a reaction mixture comprising an analyte from a test sample with a chemiluminescent moiety in an incubation chamber, said chemiluminescent moiety capable of binding to said analyte as a function of the amount of analyte present in said test sample; transferring said reaction mixture from said incubation chamber to a container having a solid, porous element in said container, said porous elements having an interactive property with said chemiluminescent moiety wherein said analyte bound to said chemiluminescent moiety is immobilized by said interactive property between said chemiluminescent moiety and said porous element to thereby prevent said chemiluminescent moiety from migrating from said porous element; distributing a chemiluminescent activating solution onto said porous element by means of a plurality of ports disposed toward an inclined interior surface of said container, said activating solution capable of reacting with said chemiluminescent moiety immobilized to said porous element to provide a chemiluminescent signal therefrom; light sealing around said container; and measuring said chemiluminescent signal from said porous element. In such preferred embodiment, preferably said distribution step comprises the step of activating a chemiluminescent reaction by applying an alkaline oxidizing solution to said porous element. The features which follow may furthermore characterize such preferred embodiment. Said solid, porous element may comprise a fibrous matrix. Said interactive property may be a hydrophobic interaction. Alternatively, said interactive property may be an ionic interaction between said chemiluminescent moiety and said solid, porous element. Said solid, porous element may be treated with a hydrophobic reagent. As an alternative, said solid, porous element may be treated with a cationic reagent and said chemiluminescent moiety is treated with an anionic reagent.

It will be seen that multiple assays can be performed with a minimum of operator input or handling on the system and the system can be utilized for other processes and assays which have not been directly discussed but will be readily apparent to one practiced in the art in view of the above invention disclosure and the claims. It will also be appreciated that although particular embodiments of the present invention have been disclosed, various changes and adaptations to the apparatus and methods can be made without departing from the teachings of the specification and scope of the invention as set out in the following claims.

## Claims

1. A heater assembly for precision temperature and delivery control of liquids, said assembly comprising a body having internal resistance heating means and temperature control means, said body having a cavity suitable for accommodating liquid tubing means, said liquid tubing means in communication with liquid inlet and outlet means, wherein said cavity is suitable for housing a tubing means for maintaining liquids at a predetermined temperature, and wherein said liquid outlet means is suitable for forced or gravity release of the liquids and is mounted directly above analytical means for receiving liquids.

2. The heater assembly according to Claim 1, wherein said predetermined temperature is from between about ± 1°C and about ± 0.5°C of a required temperature of said liquid.

3. The heater assembly according to Claim 1, wherein the temperature control means comprises a thermistor and thermostat for controlling the electrical energy provided to the electrical resistance heating means.

4. The heater assembly according to Claim 1, wherein the liquid outlet means is positioned below the lower planar surface of the liquid heater block to thereby provide minimum air gap release to analytical means having openings on a parallel plane.

5. The heater assembly according to Claim 4, wherein the air gap is from between about 1/2 inch and about 3/8 inch.

6. The heater assembly according to Claim 1, wherein said body is metal.

7. The liquid heater block assembly according to Claim 1, wherein the resistance heating means comprises multiple loop electrical resistive heating element.

8. An automated, continuous and random access analytical system capable of simultaneously effecting multiple assays of a plurality of liquid samples, comprising:
(a) a front end carousel assembly inclusive of sample cup carousel, reagent pack carousel and reaction vessel carousel, the reaction vessel carousel being concentrically mounted exterior of the reagent pack carousel and the reagent pack carousel being concentrically mounted exterior of the sample cup carousel;
(b) means for rotating the respective carousels to align with a kitting pipettor means for kitting reaction vessels;
(c) means for transferring kitted reaction vessels from a reaction vessel carousel to a transfer station, the transfer station providing means for transferring the reaction vessel to a process carousel, the process carousel having environmental means for maintaining temperature control and timing of reaction incubation;
(d) a transfer pipettor means for servicing the process carousel and a cartridge wheel carousel offset from the process carousel, the cartridge wheel carousel having means for receiving pipetted reaction mixtures from the process carousel and analytical means for receiving liquids from a heater assembly, said heater assembly comprising a metal body having internal resistance heating means and temperature control means, said body having a cavity suitable for accommodating liquid tubing means, said liquid tubing means in communication with liquid inlet and outlet means, wherein said cavity is suitable for housing a tubing means for maintaining liquids at a predetermined temperature, and wherein said liquid outlet means is suitable for forced or gravity release of the liquids arid is mounted directly above the analytical means for receiving the liquids;
(e) means for supplying cartridges to the process carousel;
(f) a microparticle enzyme immunoassay reader and processing station integrated with the process carousel;
(g) a fluorescent polarization immunoassay reader and process station integrated with the process carousel;
(h) means for removing the reaction vessel from the process carousel by operation of the transfer station and means for removing the cartridges from the cartridge wheel carousel; and
(i) means for analyzing the reaction mixture either by fluorescent polarization immunoassay or microparticle immunoassay.

9. The system according to Claim 8, wherein the predetermined temperature is from between about ±1°C and about ± 0.5°C.
